# EUROPEAN PATENT SPECIFICATION

(11) **EP 4 341 258 B1**
(45) Date of publication and mention of the grant of the patent: **03.12.2025**
(21) Application number: 22728525.1
(22) Date of filing: 10.05.2022
(51) Int. Cl.: C07D 413/04, C07D 498/10, A01N 43/72, A01N 43/90

(54) **NEW SUBSTITUTED PYRIDINES AS FUNGICIDES**
NEUE SUBSTITUIERTE PYRIDINE ALS FUNGIZIDE
NOUVELLES PYRIDINES SUBTITUÉES EN TANT QUE FONGICIDES

(30) Priority: 18.05.2021 EP 21174267
(43) Date of publication of application: 27.03.2024
(73) Proprietor: BASF SE, 67056 Ludwigshafen am Rhein (DE)
(72) Inventor: GRAMMENOS, Wassilios, 67056 Ludwigshafen (DE); MERGET, Benjamin Juergen, 67056 Ludwigshafen (DE); MUELLER, Bernd, 67056 Ludwigshafen (DE); SEET, Michael, 67056 Ludwigshafen (DE); SEEBERGER, Philipp Georg Werner, 67056 Ludwigshafen (DE); LE VEZOUET, Ronan, 67056 Ludwigshafen (DE); LOHMANN, Jan Klaas, 67056 Ludwigshafen (DE); PETKOVA, Desislava Slavcheva, 67056 Ludwigshafen (DE); MINAKAR, Amin, 67056 Ludwigshafen (DE); ZIEGLER, Dorothee Sophia, 67056 Ludwigshafen (DE); STOESSER, Tim Alexander, 67056 Ludwigshafen (DE); RIEDIGER, Nadine, 67117 Limburgerhof (DE); KOCH, Andreas, 67117 Limburgerhof (DE)
(74) Representative: BASF IP Association
(86) International application number: PCT/EP2022/062622
(87) International publication number: WO 2022/243111

(56) References cited:
- EP-A1- 2 314 583
- EP-A1- 2 762 002

## Description

The present invention relates to new pyridine compounds and the N-oxides and the salts thereof as fungicides as well to their use. The invention also relates to the composition comprising at least one compound I, to the method for combating phytopathogenic fungi and to the seed coated with at least one compound of the formula I.

WO201018686, WO201347441 disclose some pyridine compounds. However, in many cases, in particular at low application rates, the fungicidal activity of known compounds is unsatisfactory. Based on this, it was an objective of the present invention to provide compounds having improved activity and/or a broader activity spectrum against phytopathogenic fungi. Another object of the present invention is to provide fungicides with improved toxicological properties or with improved environmental fate properties.

These and further objects are achieved by the pyridine compounds of formula I, as defined below, and by their agriculturally suitable.

Accordingly, the present invention relates to the compounds of formula I 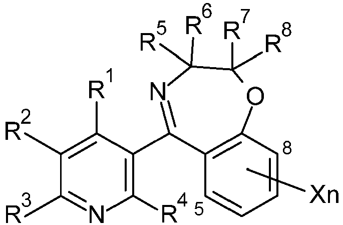 wherein
- R¹: is H;
- R²: is in each case independently selected from halogen, CN, C₁-C₆-alkyl, C₁-C₆-halogenalkyl, C₂-C₆-alkenyl, C₂-C₆-halogenalkenyl, C₂-C₆-alkynyl, O-C₁-C₆-alkyl, C₃-C₆-cycloalkyl;
- R³: is in each case independently selected from C₁-C₆-alkyl, C₁-C₆-halogenalkyl, C₂-C₆-alkenyl, C₂-C₆-halogenalkenyl, C₂-C₆-alkynyl, O-C₁-C₆-alkyl, C₃-C₆-cycloalkyl;
- R⁴: is H;
- R⁵: are in each case independently selected from H, F, CN, C₁-C₆-alkyl, C₁-C₆-halogenalkyl, C₂-C₆-alkenyl, phenyl, benzyl,
wherein the moieties are unsubstituted or substituted by one to three groups R^{5a}, which independently of one another are selected from:
halogen, CN, C₁-C₆-alkyl, C₁-C₆-halogenalkyl, O-C₁-C₆-alkyl;
- R⁶: are in each case independently selected from H, F, CN, C₁-C₆-alkyl, C₁-C₆-halogenalkyl, C₂-C₆-alkenyl, phenyl, benzyl,
wherein the moieties of R⁶ are unsubstituted or substituted by one to three groups R^{6a}, which independently of one another are selected from:
halogen, CN, C₁-C₆-alkyl, C₁-C₆-halogenalkyl, O-C₁-C₆-alkyl;
   or
R⁶ and R⁶ form together with the C atoms to which they are bound =O;
   or
R⁶ and R⁶ form together with the C atoms to which they are bound a C₃-C₆-cycloalkyl or a a 3- to 6-membered saturated heterocycle which contains 1, 2 or 3 heteroatoms from the group consisting of O and S; wherein the cycloalkyl or heterocycle can be unsubsituted or substitued by halogene, C₁-C₆-alkyl, C₁-C₆-halogenalkyl;
- R⁷: are in each case independently selected from H, F, CN, C₁-C₆-alkyl, C₁-C₆-halogenalkyl, C₂-C₆-alkenyl, phenyl, benzyl,
wherein the moieties are unsubstituted or substituted by one to three groups R^{7a}, which independently of one another are selected from:
halogen, CN, C₁-C₆-alkyl, C₁-C₆-halogenalkyl, O-C₁-C₆-alkyl;
- R⁸: are in each case independently selected from H, F, CN, C₁-C₆-alkyl, C₁-C₆-halogenalkyl, C₂-C₆-alkenyl, phenyl, benzyl,
wherein the moieties are unsubstituted or substituted by one to three groups R^{8a}, which independently of one another are selected from:
halogen, CN, C₁-C₆-alkyl, C₁-C₆-halogenalkyl, O-C₁-C₆-alkyl;
   or
R⁷ and R⁸ form together with the C atoms to which they are bound a C₃-C₆-cycloalkyl or a a 3- to 6-membered saturated heterocycle which contains 1, 2 or 3 heteroatoms from the group consisting of O and S;
- X: is in each case independently selected from halogen, CN, C₁-C₆-alkyl, C₁-C₆-halogenalkyl, O-C₁-C₆-alkyl, O-C₁-C₆-halogenalkyl, C₃-C₆-cycloalkyl, C₂-C₆-alkenyl, C₂-C₆-alkynyl;
- n: is 0, 1, 2 or 3,
with the proviso that
R⁵, R⁶, R⁷, R⁸ can not be all H;
and the N-oxides and the agriculturally acceptable salts thereof as fungicides.

The N-oxides may be prepared from the inventive compounds according to conventional oxidation methods, e. g. by treating compounds I with an organic peracid such as metachloroperbenzoic acid (cf. WO 03/64572 or J. Med. Chem. 38(11), 1892-903, 1995); or with inorganic oxidizing agents such as hydrogen peroxide (cf. J. Heterocyc. Chem. 18(7), 1305-8, 1981) or oxone (cf. J. Am. Chem. Soc. 123(25), 5962-5973, 2001). The oxidation may lead to pure mono-N-oxides or to a mixture of different N-oxides, which can be separated by conventional methods such as chromatography.

Agriculturally acceptable salts of the compounds of the formula I encompass especially the salts of those cations or the acid addition salts of those acids whose cations and anions, respectively, have no adverse effect on the fungicidal action of the compounds I. Suitable cations are thus in particular the ions of the alkali metals, preferably sodium and potassium, of the alkaline earth metals, preferably calcium, magnesium and barium, of the transition metals, preferably manganese, copper, zinc and iron, and also the ammonium ion which, if desired, may be substituted with one to four C₁-C₄-alkyl substituents and/or one phenyl or benzyl substituent, preferably diisopropylammonium, tetramethylammonium, tetrabutylammonium, trimethylbenzylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of acceptable acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting a compound I with an acid of the corresponding anion, preferably of hydrochloric acid, hydrobromic acid, sulfuric acid, phosphoric acid or nitric acid.

Compounds of the formula I can exist as one or more stereoisomers. The various stereoisomers include enantiomers, diastereomers, atropisomers arising from restricted rotation about a single bond of asymmetric groups and geometric isomers. They also form part of the subject matter of the present invention. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers. The compounds of the invention may be present as a mixture of stereoisomers, e.g. a racemate, individual stereoisomers, or as an optically active form.

Compounds of the formula I can be present in different crystal modifications whose biological activity may differ. They also form part of the subject matter of the present invention.

In respect of the variables, the embodiments of the intermediates obtained during preparation of compounds I correspond to the embodiments of the compounds of formula I. The term "compounds I" refers to compounds of the formula I.

In the following, the intermediate compounds are further described. A skilled person will readily understand that the preferences for the substituents, also in particular the ones given in the tables below for the respective substituents, given herein in connection with compounds I apply for the intermediates accordingly. Thereby, the substituents in each case have independently of each other or more preferably in combination the meanings as defined herein.

If the synthesis yields mixtures of isomers, a separation is generally not necessarily required since in some cases the individual isomers can be interconverted during work-up for use or during application (e. g. under the action of light, acids or bases). Such conversions may also take place after use, e. g. in the treatment of plants in the treated plant, or in the harmful fungus to be controlled.

In the definitions of the variables given above, collective terms are used which are generally representative for the substituents in question. The term "Cₙ-Cₘ" indicates the number of carbon atoms possible in each case in the substituent or substituent moiety in question.

The term "halogen" refers to fluorine, chlorine, bromine and iodine.

The term "C₁-C₆-alkyl" refers to a straight-chained or branched saturated hydrocarbon group having 1 to 6 carbon atoms, e.g. methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Likewise, the term "C₂-C₄-alkyl" refers to a straight-chained or branched alkyl group having 2 to 4 carbon atoms, such as ethyl, propyl (n-propyl), 1-methylethyl (iso-propoyl), butyl, 1-methylpropyl (sec.-butyl), 2-methylpropyl (iso-butyl), 1,1-dimethylethyl (tert.-butyl).

The term "C₁-C₆-halogenalkyl" refers to an alkyl group having 1 or 6 carbon atoms as defined above, wherein some or all of the hydrogen atoms in these groups may be replaced by halogen atoms as mentioned above. Examples are "C₁-C₂-halogenalkyl" groups such as chloromethyl, bromomethyl, dichloromethyl, trichloromethyl, fluoromethyl, difluoromethyl, trifluoromethyl, chlor-ofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl or pentafluoroethyl.

The term "C₁-C₆-alkoxy" refers to a straight-chain or branched alkyl group having 1 to 6 carbon atoms which is bonded via an oxygen, at any position in the alkyl group. Examples are "C₁-C₄-alkoxy" groups, such as methoxy, ethoxy, n-propoxy, 1-methylethoxy, butoxy, 1-methyl-propoxy, 2-methylpropoxy or 1,1-dimethylethoxy.

The term "C₁-C₆-halogenalkoxy" refers to a C₁-C₆-alkoxy radical as defined above, wherein some or all of the hydrogen atoms in these groups may be replaced by halogen atoms as mentioned above. Examples are "C₁-C₄-halogenalkoxy" groups, such as OCH₂F, OCHF₂, OCF₃, OCH₂Cl, OCHCl₂, OCCI₃, chlorofluoromethoxy, dichlorofluoromethoxy, chlorodifluoromethoxy, 2-fluoroethoxy, 2-chlorothoxy, 2-bromoethoxy, 2-iodoethoxy, 2,2-difluoroethoxy, 2,2,2-trifluoro-ethoxy, 2-chloro-2-fluoroethoxy, 2-chloro-2,2-difluoroethoxy, 2,2-dichloro-2-fluoroethoxy, 2,2,2-trichloroethoxy, OC₂F₅, 2-fluoropropoxy, 3-fluoropropoxy, 2,2-difluoropropoxy, 2,3-difluoro-propoxy, 2 chloropropoxy, 3-chloropropoxy, 2,3-dichloropropoxy, 2-bromopropoxy, 3 bromopropoxy, 3,3,3-trifluoropropoxy, 3,3,3-trichloropropoxy, OCH₂-C₂F₅, OCF₂-C₂F₅, 1-fluoromethyl-2-fluoroethoxy, 1-chloromethyl-2-chloroethoxy, 1-bromomethyl-2-bromoethoxy, 4-fluorobutoxy, 4-chlorobutoxy, 4-bromobutoxy or nonafluorobutoxy.

The term "C₂-C₆-alkenyl" refers to a straight-chain or branched unsaturated hydrocarbon radical having 2 to 6 carbon atoms and a double bond in any position. Examples are "C₂-C₄-alkenyl" groups, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₆-halogenalkenyl" refers to an alkyl group having 2 or 6 carbon atoms as defined above, wherein some or all of the hydrogen atoms in these groups may be replaced by halogen atoms as mentioned above.

The term "C₂-C₆-alkenyloxy" refers to a straight-chain or branched alkenyl group having 2 to 6 carbon atoms which is bonded via an oxygen, at any position in the alkenyl group. Examples are "C₂-C₄-alkenyloxy" groups.

The term "C₂-C₆-alkynyl" refers to a straight-chain or branched unsaturated hydrocarbon radical having 2 to 6 carbon atoms and containing at least one triple bond. Examples are "C₂-C₄-alkynyl" groups, such as ethynyl, prop-1-ynyl, prop-2-ynyl (propargyl), but-1-ynyl, but-2-ynyl, but-3-ynyl, 1-methyl-prop-2-ynyl.

The term "C₂-C₆-halogenalkynyl" refers to an alkyl group having 2 or 6 carbon atoms as defined above, wherein some or all of the hydrogen atoms in these groups may be replaced by halogen atoms as mentioned above.

The term "C₂-C₆-alkynyloxy" refers to a straight-chain or branched alkynyl group having 2 to 6 carbon atoms which is bonded via an oxygen, at any position in the alkynyl group. Examples are "C₂-C₄-alkynyloxy" groups.

The term "C₃-C₆-cycloalkyl" refers to monocyclic saturated hydrocarbon radicals having 3 to 6 carbon ring members, such as cyclopropyl, cyclobutyl, cyclopentyl, cyclohexyl. Accordingly, a saturated three-, four-, five-, six-, seven-, eight-, nine or ten-membered carbocyclyl or carbocycle is a "C₃-C₁₀-cycloalkyl".

The term "C₃-C₆-cycloalkenyl" refers to a monocyclic partially unsaturated 3-, 4- 5- or 6-membered carbocycle having 3 to 6 carbon ring members and at least one double bond, such as cyclopentenyl, cyclopentadienyl, cyclohexadienyl. Accordingly, a partially unsaturated three-, four-, five-, six-, seven-, eight-, nine or ten-membered carbocyclyl or carbocycle is a "C₃-C₁₀-cycloalkenyl".

The term "C₃-C₈-cycloalkyl-C₁-C₄-alkyl" refers to alkyl having 1 to 4 carbon atoms (as defined above), whereAccording to one hydrogen atom of the alkyl radical is replaced by a cycloalkyl radical having 3 to 8 carbon atoms (as defined above).

The term "saturated or partially unsaturated three-, four-, five-, six-, seven-, eight-, nine or ten-membered heterocyclyl or heterocycle, wherein the heterocyclyl or heterocycle contains 1, 2, 3 or 4 heteroatoms selected from N, O and S" is to be understood as meaning both saturated and partially unsaturated heterocycles, wherein the ring member atoms of the heterocycle include besides carbon atoms 1, 2, 3 or 4 heteroatoms independently selected from the group of O, N and S. For example:
a 3- or 4-membered saturated heterocycle which contains 1 or 2 heteroatoms from the group consisting of O, N and S as ring members such as oxirane, aziridine, thiirane, oxetane, azetidine, thiethane, [1,2]dioxetane, [1,2]dithietane, [1,2]diazetidine; and
a 5- or 6-membered saturated or partially unsaturated heterocycle which contains 1, 2 or 3 heteroatoms from the group consisting of O, N and S as ring members such as 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyra-zol-1-yl, 2,3-dihydropyrazol-2-yl, 2,3-dihydropyrazol-3-yl, 2,3-dihydropyrazol-4-yl, 2,3-dihydropy-razol-5-yl, 3,4-dihydropyrazol-1-yl, 3,4-dihydropyrazol-3-yl, 3,4-dihydropyrazol-4-yl, 3,4-dihydro-pyrazol-5-yl, 4,5-dihydropyrazol-1-yl, 4,5-dihydropyrazol-3-yl, 4,5-dihydropyrazol-4-yl, 4,5-dihy-dropyrazol-5-yl, 2,3-dihydrooxazol-2-yl, 2,3-dihydrooxazol-3-yl, 2,3-dihydrooxazol-4-yl, 2,3-dihy-drooxazol-5-yl, 3,4-dihydrooxazol-2-yl, 3,4-dihydrooxazol-3-yl, 3,4-dihydrooxazol-4-yl, 3,4-dihy-drooxazol-5-yl, 3,4-dihydrooxazol-2-yl, 3,4-dihydrooxazol-3-yl, 3,4-dihydrooxazol-4-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl and also the corresponding -ylidene radicals; and
a 7-membered saturated or partially unsaturated heterocycle such as tetra- and hexahydroazepinyl, such as 2,3,4,5-tetrahydro[1H]azepin-1-,-2-,-3-,-4-,-5-,-6- or-7-yl, 3,4,5,6-tetrahy-dro[2H]azepin-2-,-3-,-4-,-5-,-6- or-7-yl, 2,3,4,7-tetrahydro[1H]azepin-1-,-2-,-3-,-4-,-5-,-6- or-7-yl, 2,3,6,7-tetrahydro[1H]azepin-1-,-2-,-3-,-4-,-5-,-6- or-7-yl, hexahydroazepin-1-,-2-,-3- or-4-yl, tetra- and hexahydrooxepinyl such as 2,3,4,5-tetrahydro[1H]oxepin-2-,-3-,-4-,-5-,-6- or-7-yl, 2,3,4,7-tetrahydro[1H]oxepin-2-,-3-,-4-,-5-,-6- or-7-yl, 2,3,6,7-tetrahydro[1H]oxepin-2-, -3-,-4-,-5-, -6- or-7-yl, hexahydroazepin-1-,-2-,-3- or-4-yl, tetra- and hexahydro-1,3-diazepinyl, tetra- and hexahydro-1,4-diazepinyl, tetra- and hexahydro-1,3-oxazepinyl, tetra- and hexahydro-1,4-oxa-zepinyl, tetra- and hexahydro-1,3-dioxepinyl, tetra- and hexahydro-1,4-dioxepinyl and the corresponding -ylidene radicals.

The term "substituted" refers to substitued with 1, 2, 3 or up to the maximum possible number of substituents.

The term "5-or 6-membered heteroaryl" or "5-or 6-membered heteroaromatic" refers to aromatic ring systems incuding besides carbon atoms, 1, 2, 3 or 4 heteroatoms independently selected from the group consisting of N, O and S, for example,
a 5-membered heteroaryl such as pyrrol-1-yl, pyrrol-2-yl, pyrrol-3-yl, thien-2-yl, thien-3-yl, furan-2-yl, furan-3-yl, pyrazol-1-yl, pyrazol-3-yl, pyrazol-4-yl, pyrazol-5-yl, imidazol-1-yl, imidazol-2-yl, imidazol-4-yl, imidazol-5-yl, oxazol-2-yl, oxazol-4-yl, oxazol-5-yl, isoxazol-3-yl, isoxazol-4-yl, isoxazol-5-yl, thiazol-2-yl, thiazol-4-yl, thiazol-5-yl, isothiazol-3-yl, isothiazol-4-yl, isothiazol-5-yl, 1,2,4-triazolyl-1-yl, 1,2,4-triazol-3-yl 1,2,4-triazol-5-yl, 1,2,4-oxadiazol-3-yl, 1,2,4-oxadiazol-5-yl and 1,2,4-thiadiazol-3-yl, 1,2,4-thiadiazol-5-yl; or
a 6-membered heteroaryl, such as pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl and 1,3,5-triazin-2-yl and 1,2,4-triazin-3-yl.

In the following, particular embodiments of the inventive compounds are described. Therein, specific meanings of the respective substituents are further detailled, wherein the meanings are in each case on their own but also in any combination with one another, particular embodiments of the present invention.

Furthermore, in respect of the variables, generally, the embodiments of the compounds I also apply to the intermediates.

According to one embodiment of the compound of formula I, R¹ is H.

According to one embodiment of the compound of formula I, R² is selected from halogen, CN, C₁-C₆-alkyl, C₁-C₆-halogenalkyl, C₂-C₆-alkenyl, C₂-C₆-halogenalkenyl, C₂-C₆-alkynyl, O-C₁-C₆-alkyl, C₃-C₆-cycloalkyl.

According to still another embodiment of formula I, R² is halogen, in particular F, Cl, Br or I, more specifically F, Cl or Br, in particular F or CI.

According to still another embodiment of formula I, R² is F.

According to still another embodiment of formula I, R² is CI.

According to still another embodiment of formula I, R² is Br.

According to still another embodiment of formula I, R² is CN.

According to still another embodiment of formula I, R² is C₁-C₆-alkyl, in particular C₁-C₄-alkyl, such as CH₃ or C₂H₅, in particular CH₃ or CH₂CH₃.

According to still another embodiment of formula I, R² is C₁-C₆-halogenalkyl, in particular C₁-C₄-halogenalkyl, such as CF₃.

According to still a further embodiment of formula I, R² is C₂-C₆-alkenyl, in particular C₂-C₄-alkenyl, such as CH=CH₂, C(CH₃)=CH₂, CH₂CH=CH₂.

According to a further specific embodiment of formula I, R² is O-C₁-C₆-alkyl, in particular C₁-C₄-alkyl, more specifically C₁-C₂-alkoxy. R² is such as OCH₃ or OCH₂CH₃.

According to still another embodiment of formula I, R² is C₃-C₆-cycloalkyl, in particular cyclopropyl or cyclobutyl.

Particularly preferred embodiments of R² according to the invention are in Table P2 below, wherein each line of lines P2-1 to P2-19 corresponds to one particular embodiment of the invention, wherein P2-1 to P2-19 are also in any combination with one another a preferred embodiment of the present invention. The connection point to the carbon atom, to which R² is bound is marked with "#" in the drawings.

**Table P2:**

| **No.** | **R²** |
|---|---|
| P2-1 | CH₃ |
| P2-2 | CH₂F |
| P2-3 | CHF₂ |
| P2-4 | CF₃ |
| P2-5 | C₂H₅ |
| P2-6 | CH(CH₃)₂ |
| P2-7 | CH₂CH₂CH₃ |
| P2-8 | CH=CH₂ |
| P2-9 | CH₂CH=CH₂ |
| P2-10 | CΞCH |
| P2-11 | CH₂CΞCH |
| P2-12 | OCH₃ |
| P2-13 | OC₂H₅ |
| P2-14 | CN |
| P2-15 | F |
| P2-16 | Cl |
| P2-17 | Br |
| P2-18 | 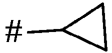 |
| P2-19 | 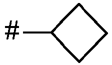 |

According to one embodiment of formula I, R³ is selected from the group consisting of C₁-C₆-alkyl, C₁-C₆-halogenalkyl, in particular CH₃, C₂H₅, CF₃, CH₂F, CHF₂, more specifically CH₃, CH₂F, CF₂H, CF₃, cyclopropyl, cyclobutyl most preferred CH₃, CF₃, CF₂H.

According to still another embodiment of formula I, R³ is C₁-C₆-alkyl, in particular C₁-C₄-alkyl, such as CH₃ or C₂H₅, in particular CH₃ or CH₂CH₃.

According to still another embodiment of formula I, R³ is C₁-C₆-halogenalkyl, in particular C₁-C₄-halogenalkyl, such as CF₃, FCH₂, F₂CH, CF₃CH₂.

According to still a further embodiment of formula I, R³ is C₂-C₆-alkenyl, in particular C₂-C₄-alkenyl, such as CH=CH₂, C(CH₃)=CH₂, CH₂CH=CH₂.

According to a further specific embodiment of formula I, R³ is O-C₁-C₆-alkyl, in particular C₁-C₄-alkyl, more specifically C₁-C₂-alkoxy. R³ is such as OCH₃ or OCH₂CH₃.

According to still another embodiment of formula I, R³ is C₃-C₆-cycloalkyl, in particular cyclopropyl, cyclobutyl.

Particularly preferred embodiments of R³ according to the invention are in Table P3 below, wherein each line of lines P3-1 to P3-15 corresponds to one particular embodiment of the invention, wherein P3-1 to P3-15 are also in any combination with one another a preferred embodiment of the present invention. The connection point to the carbon atom, to which R³ is bound is marked with "#" in the drawings.

**Table P3:**

| **No.** | **R³** |
|---|---|
| P3-1 | CH₃ |
| P3-2 | CH₂F |
| P3-3 | CHF₂ |
| P3-4 | CF₃ |
| P3-5 | C₂H₅ |
| P3-6 | CH(CH₃)₂ |
| P3-7 | CH₂CH₂CH₃ |
| P3-8 | CH=CH₂ |
| P3-9 | CH₂CH=CH₂ |
| P3-10 | CΞCH |
| P3-11 | CH₂CΞCH |
| P3-12 | OCH₃ |
| P3-13 | OC₂H₅ |
| P3-14 | 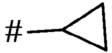 |
| P3-15 | 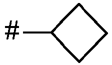 |

According to one embodiment of the compound of formula I, R⁴ is H.

According to one embodiment of the compound of formula I,
R⁵ is in each case independently selected from H, F, CN, C₁-C₆-alkyl, C₁-C₆-halogenalkyl, C₂-C₆-alkenyl, phenyl, benzyl,
wherein the are unsubstituted or substituted by one to three groups R^{5a}, which independently of one another are selected from:
   halogen, CN, C₁-C₆-alkyl, C₁-C₆-halogenalkyl, O-C₁-C₆-alkyl.

According to one embodiment of the compound of formula I, R⁵ is in each case independently selected from C₁-C₆-alkyl (embodiment 5.1), C₁-C₆-halogenalkyl (embodiment 5.2), H (embodiment 5.3), phenyl, CH₂-phenyl (embodiment 5.4), wherein phenyl and CH₂-phenyl is unsubstituted or substituted by one or two halogen.

According to one further embodiment of the compound of formula I, R⁵ is CH₃ or CF₃.

According to one further embodiment of the compound of formula I, R⁵ is CH₃.

According to one further embodiment of the compound of formula I, R⁵ is H.

According to one further embodiment of the compound of formula I, R⁵ is H, CH₂CH₃, CH(CH₃)₂, CH(CH₃)CH₂CH₃, C(CH₃)₃, CH₂-CH(CH₃)₂, CH₂-C(CH₃)₃.

According to one further embodiment of the compound of formula I, R⁵ is phenyl, 2-F-phenyl, 4-F-phenyl, 2,4-F₂-phenyl, 2-Cl-phenyl, 4-Cl-phenyl, CH₂-phenyl, CH₂-2-F-phenyl, CH₂-4-F-phenyl.

According to one embodiment of the compound of formula I, R⁶ is in each case independently selected from are in each case independently selected from H, F, CN, C₁-C₆-alkyl, C₁-C₆-halo-genalkyl, C₂-C₆-alkenyl, phenyl, benzyl,
wherein the moieties are unsubstituted or substituted by one to three groups R^{6a}, which independently of one another are selected from:
halogen, CN, C₁-C₆-alkyl, C₁-C₆-halogenalkyl, O-C₁-C₆-alkyl.

According to one embodiment of the compound of formula I, R⁶ is in each case independently selected from C₁-C₆-alkyl (embodiment 6.1), H (embodiment 6.2).

According to one further embodiment of the compound of formula I, R⁶ is H, CH₃, CH₂CH₃, CH(CH₃)₂, CH(CH₃)CH₂CH₃, C(CH₃)₃, CH₂-CH(CH₃)₂, CH₂-C(CH₃)₃, CH₂-CH(CH₃)-C(CH₃)₃, CH₂-CH₂-C(CH₃)₃.

According to one further embodiment of the compound of formula I, R⁵ and R⁶ form together with the C atoms to which they are bound =O or a C₃-C₆-cycloalkyl or a a 3- to 6-membered saturated heterocycle which contains 1, 2 or 3 heteroatoms from the group consisting of O and S; wherein the cycloalkyl or heterocycle can be unsubsituted or substitued by halogene, C₁-C₆-alkyl, C₁-C₆-halogenalkyl;

According to one further embodiment of the compound of formula I, R⁵ and R⁶ form =O (embodiment 6.3).

According to one further embodiment of the compound of formula I, R⁵ and R⁶ form C₃-C₆-cycloalkyl (embodiment 6.4).

According to one further embodiment of the compound of formula I, R⁵ and R⁶ form 3- to 6-membered saturated heterocycle which contains 1, 2 or 3 heteroatoms from the group consisting of O and S.

According to one further embodiment of the compound of formula I, R⁵ and R⁶ form 3- to 6-membered saturated heterocycle which contains one O (embodiment 6.5).

Prefferred embodiments of R⁵, R⁶ according to the invention are in Table P5 below, wherein each line of lines P5-1 to P5-19 corresponds to one particular embodiment of the invention, wherein P5-1 to P5-19 are also in any combination with one another a preferred embodiment of the present invention. The connection point to the carbon atom, to which R⁵ and R⁶ is bound is marked with "#" in the drawings.

**Table P5,6:**

| **No.** | **R⁵** | **R⁶** |
|---|---|---|
| P5-1 | 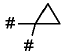 | |
| P5-2 | 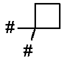 | |
| P5-3 | 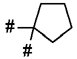 | |
| P5-4 | 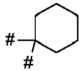 | |
| P5-5 | 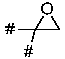 | |
| P5-6 | 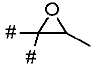 | |
| P5-7 | 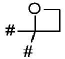 | |
| P5-8 | 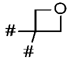 | |
| P5-9 | 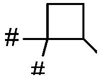 | |
| P5-10 | 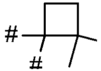 | |
| P5-11 | 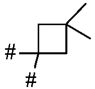 | |
| P5-12 | 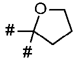 | |
| P5-13 | 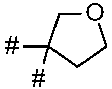 | |
| P5-14 | 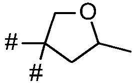 | |
| P5-15 | 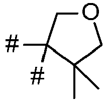 | |
| P5-16 | 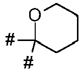 | |
| P5-17 | 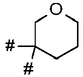 | |
| P5-18 | 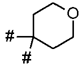 | |
| P5-19 | =O | |

According to one embodiment of the compound of formula I,
R⁷ is in each case independently selected from H, F, CN, C₁-C₆-alkyl, C₁-C₆-halogenalkyl, C₂-C₆-alkenyl, C₁-C₆-alkyl-O-C₁-C₆-alkyl, phenyl, benzyl,
wherein the moieties are unsubstituted or substituted by one to three groups R^{7a}, which independently of one another are selected from:
   halogen, CN, C₁-C₆-alkyl, C₁-C₆-halogenalkyl, O-C₁-C₆-alkyl.

According to one embodiment of the compound of formula I, R⁷ is in each case independently selected from C₁-C₆-alkyl (embodiment 7.1), C₁-C₆-halogenalkyl (embodiment 7.2), C₁-C₆-alkyl-O-C₁-C₆-alkyl (embodiment 7.3), phenyl, CH₂-phenyl (embodiment 7.4), wherein phenyl and CH₂-phenyl is unsubstituted or substituted by one or two halogen.

According to one further embodiment of the compound of formula I, R⁷ is CH₃ or CF₃.

According to one further embodiment of the compound of formula I, R⁷ is CH₃.

According to one further embodiment of the compound of formula I, R⁷ is H.

According to one further embodiment of the compound of formula I, R⁷ is CH₂CH₃, CH(CH₃)₂, CH(CH₃)CH₂CH₃, C(CH₃)₃, CH₂-CH(CH₃)₂, CH₂-C(CH₃)₃, CH₂-O-CH₃.

According to one further embodiment of the compound of formula I, R⁷ is phenyl, 2-F-phenyl, 4-F-phenyl, 2,4-F₂-phenyl, 2-Cl-phenyl, 4-Cl-phenyl, CH₂-phenyl, CH₂-2-F-phenyl, CH₂-4-F-phenyl.

According to one embodiment of the compound of formula I, R⁸ is in each case independently selected from are in each case independently selected from H, F, CN, C₁-C₆-alkyl, C₁-C₆-halo-genalkyl, C₂-C₆-alkenyl, C₁-C₆-alkyl-O-C₁-C₆-alkyl, phenyl, benzyl, C₁-C₆-alkyl-O-phenyl,
wherein the moieties are unsubstituted or substituted by one to three groups R^{6a}, which independently of one another are selected from:
halogen, CN, C₁-C₆-alkyl, C₁-C₆-halogenalkyl, O-C₁-C₆-alkyl.

According to one embodiment of the compound of formula I, R⁸ is in each case independently selected from C₁-C₆-alkyl (embodiment 8.1), C₁-C₆-alkyl-O-phenyl (embodiment 8.2), C₁-C₆-alkyl-O-C₁-C₆-alkyl (embodiment 8.3).

According to one further embodiment of the compound of formula I, R⁸ is CH₂CH₃, CH(CH₃)₂, CH(CH₃)CH₂CH₃, C(CH₃)₃, CH₂-CH(CH₃)₂, CH₂-C(CH₃)₃, CH₂-CH(CH₃)-C(CH₃)₃, CH₂-CH₂-C(CH₃)₃, CH₂-O-CH₃, CH₂-O-(CH₃)₃, CH₂-O-phenyl.

According to one further embodiment of the compound of formula I, R⁷ and R⁸ form together with the C atoms to which they are bound a C₃-C₆-cycloalkyl or a a 3- to 6-membered saturated heterocycle which contains 1, 2 or 3 heteroatoms from the group consisting of O and S.

According to one further embodiment of the compound of formula I, R⁷ and R⁸ form C₃-C₆-cycloalkyl (embodiment 8.4).

According to one further embodiment of the compound of formula I, R⁷ and R⁸ form 3- to 6-membered saturated heterocycle which contains 1, 2 or 3 heteroatoms from the group consisting of O and S.

According to one further embodiment of the compound of formula I, R⁷ and R⁸ form 3- to 6-membered saturated heterocycle which contains one O (embodiment 8.5).

Prefferred embodiments of R⁷ and R⁸ according to the invention are in Table P5 below, wherein each line of lines P5-1 to P5-18 corresponds to one particular embodiment of the invention, wherein P5-1 to P5-18 are also in any combination with one another a preferred embodiment of the present invention. The connection point to the carbon atom, to which R⁵ and R⁶ is bound is marked with "#" in the drawings.

**Table P7,8:**

| **No.** | **R⁷** | **R⁸** |
|---|---|---|
| P7-1 | 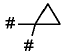 | |
| P7-2 | 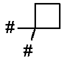 | |
| P7-3 | 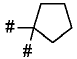 | |
| P7-4 | 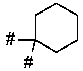 | |
| P7-5 | 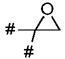 | |
| P7-6 | 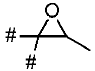 | |
| P7-7 | 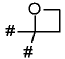 | |
| P7-8 | 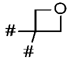 | |
| P7-9 | 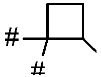 | |
| P7-10 | 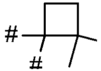 | |
| P7-11 | 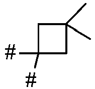 | |
| P7-12 | 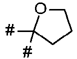 | |
| P7-13 | 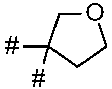 | |
| P7-14 | 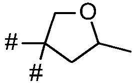 | |
| P7-15 | 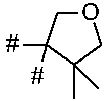 | |
| P7-16 | 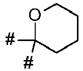 | |
| P7-17 | 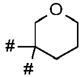 | |
| P7-18 | 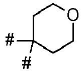 | |

According to one embodiment of the compound of formula I, X is in each case independently selected from halogen (embodiment X.1), CN, C₁-C₆-alkyl (embodiment X.2), C₁-C₆-halogen-alkyl (embodiment X.3), O-C₁-C₆-alkyl (embodiment X.4), O-C₁-C₆-halogenalkyl (embodiment X.5).

According to one embodiment of the compound of formula I, X is in each case independently selected from halogen, O-C₁-C₆-alkyl.

According to one embodiment of the compound of formula I, X is in each case independently selected from F or CI.

According to one embodiment Xn is as defined below: 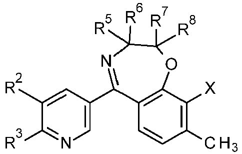 and X is selected from F, Cl, I, CH₃, cyclopropyl, CH=CH₂, CΞCH, OCH₃, OCHF₂, CF₃, CHF₂, CH₂CH₃, CN.

According to one embodiment Xn is as defined below: 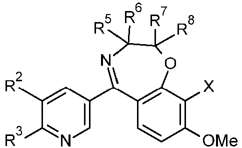 and X is selected from F, Cl, I, CH₃, cyclopropyl, CH=CH₂, CΞCH, OCH₃, OCHF₂, CF₃, CHF₂, CH₂CH₃, CN.

According to one embodiment Xn is as defined below: 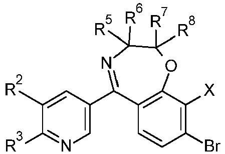 and X is selected from F, Cl, I, CH₃, cyclopropyl, CH=CH₂, CΞCH, OCH₃, OCHF₂, CF₃, CHF₂, CH₂CH₃, CN.

According to one embodiment Xn is as defined below: 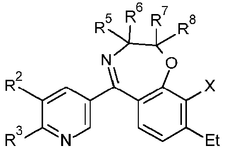 and X is selected from F, Cl, I, CH₃, cyclopropyl, CH=CH₂, CΞCH, OCH₃, OCHF₂, CF₃, CHF₂, CH₂CH₃, CN.

According to one embodiment Xn is as defined below: 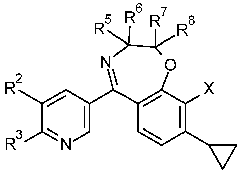 and X is selected from F, Cl, I, CH₃, cyclopropyl, CH=CH₂, CΞCH, OCH₃, OCHF₂, CF₃, CHF₂, CH₂CH₃, CN.

According to one embodiment Xn is as defined below: 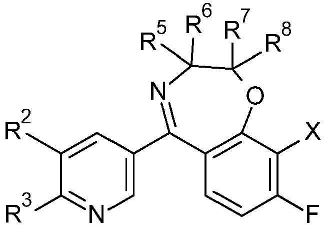 and X is selected from F, Cl, I, CH₃, cyclopropyl, CH=CH₂, CΞCH, OCH₃, OCHF₂, CF₃, CHF₂, CH₂CH₃, CN.

According to one embodiment Xn is as defined below: 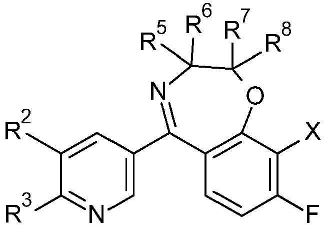 and X is F.

According to one embodiment Xn is as defined below: 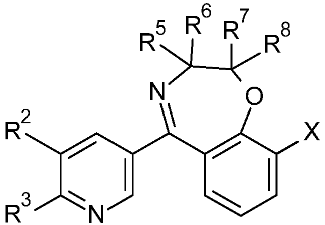 and X is selected from F, Cl, I, CH₃, cyclopropyl, CH=CH₂, CΞCH, OCH₃, OCHF₂, CF₃, CHF₂, CH₂CH₃, CN.

According to one embodiment Xn is as defined below: 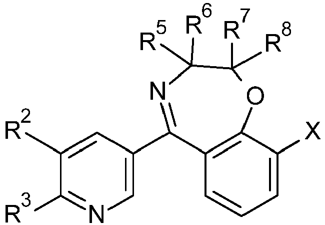 and X is F.

According to one embodiment Xn is as defined below: 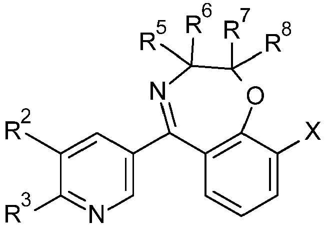 and X is H.

According to one embodiment Xn is as defined below: 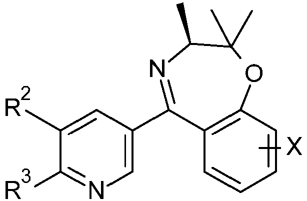 and X is selected from F, Cl, I, CH₃, cyclopropyl, CH=CH₂, CΞCH, OCH₃, OCHF₂, CF₃, CHF₂, CH₂CH₃, CN.

According to one embodiment of the compound of formula I, n is 0.

According to one embodiment of the compound of formula I, n is 1.

According to one embodiment of the compound of formula I, n is 2.

The compounds of the formula I comprise one or more chiral center and are generally obtained in the form of a racemate. The R- and S-enantiomers can be separated and isolated in pure form with methods known by the skilled person, e.g. by using chiral HPLC.

Therefore, according to the present invention, the compound of the formula I can be used in form of
- a racemic mixture of the of the (R)-enantiomer and the (S)-enantiomer;
- a mixture with any other proportions of the (R)-enantiomer and the (S)-enantiomer;
- pure (R)-enantiomer or
- pure (S)-enantiomer.

According a particular embodiment of the present invention, the compound of the formula I is present as racemic composition of the (R)-enantiomer and (S)-enantiomer, but the (R)-enantiomer and the (S)-enantiomer may also be present in any other proportion, for example the pure enantiomer (R) or the pure enantiomer (S) of the compound of the formula I.

According to one specific embodiment, the compound of the formula I is provided and used as (R)-enantiomer with an enantiomeric excess (e.e.) of at least 40%, for example, at least 50%, 60%, 70% or 80%, preferably at least 90%, more preferably at least 95%, yet more preferably at least 98% and most preferably at least 99%.

According to a further specific embodiment, the compound of the formula I is provided and used as (S)-enantiomer with an enantiomeric excess (e.e.) of at least 40%, for example, at least 50%, 60%, 70% or 80%, preferably at least 90%, more preferably at least 95%, yet more preferably at least 98% and most preferably at least 99%.

In further aspects the present invention relates to the embodiments E.1 to E.275 listed in Table E, which represent preferred combinations of embodiments that are defined above for each of the variables R², R³ and X (represented by embodiments X.1 to X.6), n in compounds of formula I as defined below.

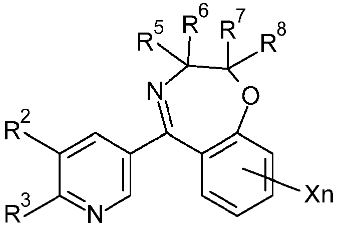

**Table E:**

| **Embodiment** | **X** | **n** | **R²** | **R³** |
|---|---|---|---|---|
| E.1 | (X.1) | 0, 1, or 2 | C₁-C₆-alkyl | C₁-C₆-alkyl |
| E.2 | (X.2) | 0, 1, or 2 | C₁-C₆-alkyl | C₁-C₆-alkyl |
| E.3 | (X.3) | 0, 1, or 2 | C₁-C₆-alkyl | C₁-C₆-alkyl |
| E.4 | (X.4) | 0, 1, or 2 | C₁-C₆-alkyl | C₁-C₆-alkyl |
| E.5 | (X.5) | 0, 1, or 2 | C₁-C₆-alkyl | C₁-C₆-alkyl |
| E.6 | (X.1) | 0, 1, or 2 | halogen | C₁-C₆-alkyl |
| E.7 | (X.2) | 0, 1, or 2 | halogen | C₁-C₆-alkyl |
| E.8 | (X.3) | 0, 1, or 2 | halogen | C₁-C₆-alkyl |
| E.9 | (X.4) | 0, 1, or 2 | halogen | C₁-C₆-alkyl |
| E.10 | (X.5) | 0, 1, or 2 | halogen | C₁-C₆-alkyl |
| E.11 | (X.1) | 0, 1, or 2 | C₁-C₆-halogenalkyl | C₁-C₆-alkyl |
| E.12 | (X.2) | 0, 1, or 2 | C₁-C₆-halogenalkyl | C₁-C₆-alkyl |
| E.13 | (X.3) | 0, 1, or 2 | C₁-C₆-halogenalkyl | C₁-C₆-alkyl |
| E.14 | (X.4) | 0, 1, or 2 | C₁-C₆-halogenalkyl | C₁-C₆-alkyl |
| E.15 | (X.5) | 0, 1, or 2 | C₁-C₆-halogenalkyl | C₁-C₆-alkyl |
| E.16 | (X.1) | 0, 1, or 2 | C₂-C₆-alkenyl | C₁-C₆-alkyl |
| E.17 | (X.2) | 0, 1, or 2 | C₂-C₆-alkenyl | C₁-C₆-alkyl |
| E.18 | (X.3) | 0, 1, or 2 | C₂-C₆-alkenyl | C₁-C₆-alkyl |
| E.19 | (X.4) | 0, 1, or 2 | C₂-C₆-alkenyl | C₁-C₆-alkyl |
| E.20 | (X.5) | 0, 1, or 2 | C₂-C₆-alkenyl | C₁-C₆-alkyl |
| E.21 | (X.1) | 0, 1, or 2 | C₂-C₆-halogenalkenyl | C₁-C₆-alkyl |
| E.22 | (X.2) | 0, 1, or 2 | C₂-C₆-halogenalkenyl | C₁-C₆-alkyl |
| E.23 | (X.3) | 0, 1, or 2 | C₂-C₆-halogenalkenyl | C₁-C₆-alkyl |
| E.24 | (X.4) | 0, 1, or 2 | C₂-C₆-halogenalkenyl | C₁-C₆-alkyl |
| E.25 | (X.5) | 0, 1, or 2 | C₂-C₆-halogenalkenyl | C₁-C₆-alkyl |
| E.26 | (X.1) | 0, 1, or 2 | C₂-C₆-alkynyl | C₁-C₆-alkyl |
| E.27 | (X.2) | 0, 1, or 2 | C₂-C₆-alkynyl | C₁-C₆-alkyl |
| E.28 | (X.3) | 0, 1, or 2 | C₂-C₆-alkynyl | C₁-C₆-alkyl |
| E.29 | (X.4) | 0, 1, or 2 | C₂-C₆-alkynyl | C₁-C₆-alkyl |
| E.30 | (X.5) | 0, 1, or 2 | C₂-C₆-alkynyl | C₁-C₆-alkyl |
| E.31 | (X.1) | 0, 1, or 2 | C₂-C₆-halogenalkynyl | C₁-C₆-alkyl |
| E.32 | (X.2) | 0, 1, or 2 | C₂-C₆-halogenalkynyl | C₁-C₆-alkyl |
| E.33 | (X.3) | 0, 1, or 2 | C₂-C₆-halogenalkynyl | C₁-C₆-alkyl |
| E.34 | (X.4) | 0, 1, or 2 | C₂-C₆-halogenalkynyl | C₁-C₆-alkyl |
| E.35 | (X.5) | 0, 1, or 2 | C₂-C₆-halogenalkynyl | C₁-C₆-alkyl |
| E.36 | (X.1) | 0, 1, or 2 | O-C₁-C₆-alkyl | C₁-C₆-alkyl |
| E.37 | (X.2) | 0, 1, or 2 | O-C₁-C₆-alkyl | C₁-C₆-alkyl |
| E.38 | (X.3) | 0, 1, or 2 | O-C₁-C₆-alkyl | C₁-C₆-alkyl |
| E.39 | (X.4) | 0, 1, or 2 | O-C₁-C₆-alkyl | C₁-C₆-alkyl |
| E.40 | (X.5) | 0, 1, or 2 | O-C₁-C₆-alkyl | C₁-C₆-alkyl |
| E.41 | (X.1) | 0, 1, or 2 | O-C₂-C₆-alkenyl | C₁-C₆-alkyl |
| E.42 | (X.2) | 0, 1, or 2 | O-C₂-C₆-alkenyl | C₁-C₆-alkyl |
| E.43 | (X.3) | 0, 1, or 2 | O-C₂-C₆-alkenyl | C₁-C₆-alkyl |
| E.44 | (X.4) | 0, 1, or 2 | O-C₂-C₆-alkenyl | C₁-C₆-alkyl |
| E.45 | (X.5) | 0, 1, or 2 | O-C₂-C₆-alkenyl | C₁-C₆-alkyl |
| E.46 | (X.1) | 0, 1, or 2 | O-C₂-C₆-alkynyl | C₁-C₆-alkyl |
| E.47 | (X.2) | 0, 1, or 2 | O-C₂-C₆-alkynyl | C₁-C₆-alkyl |
| E.48 | (X.3) | 0, 1, or 2 | O-C₂-C₆-alkynyl | C₁-C₆-alkyl |
| E.49 | (X.4) | 0, 1, or 2 | O-C₂-C₆-alkynyl | C₁-C₆-alkyl |
| E.50 | (X.5) | 0, 1, or 2 | O-C₂-C₆-alkynyl | C₁-C₆-alkyl |
| E.51 | (X.1) | 0, 1, or 2 | C₃-C₆-cycloalkyl | C₁-C₆-alkyl |
| E.52 | (X.2) | 0, 1, or 2 | C₃-C₆-cycloalkyl | C₁-C₆-alkyl |
| E.53 | (X.3) | 0, 1, or 2 | C₃-C₆-cycloalkyl | C₁-C₆-alkyl |
| E.54 | (X.4) | 0, 1, or 2 | C₃-C₆-cycloalkyl | C₁-C₆-alkyl |
| E.55 | (X.5) | 0, 1, or 2 | C₃-C₆-cycloalkyl | C₁-C₆-alkyl |
| E.56 | (X.1) | 0, 1, or 2 | C₁-C₆-alkyl | C₁-C₆-halogenalkyl |
| E.57 | (X.2) | 0, 1, or 2 | C₁-C₆-alkyl | C₁-C₆-halogenalkyl |
| E.58 | (X.3) | 0, 1, or 2 | C₁-C₆-alkyl | C₁-C₆-halogenalkyl |
| E.59 | (X.4) | 0, 1, or 2 | C₁-C₆-alkyl | C₁-C₆-halogenalkyl |
| E.60 | (X.5) | 0, 1, or 2 | C₁-C₆-alkyl | C₁-C₆-halogenalkyl |
| E.61 | (X.1) | 0, 1, or 2 | halogen | C₁-C₆-halogenalkyl |
| E.62 | (X.2) | 0, 1, or 2 | halogen | C₁-C₆-halogenalkyl |
| E.63 | (X.3) | 0, 1, or 2 | halogen | C₁-C₆-halogenalkyl |
| E.64 | (X.4) | 0, 1, or 2 | halogen | C₁-C₆-halogenalkyl |
| E.65 | (X.5) | 0, 1, or 2 | halogen | C₁-C₆-halogenalkyl |
| E.66 | (X.1) | 0, 1, or 2 | C₁-C₆-halogenalkyl | C₁-C₆-halogenalkyl |
| E.67 | (X.2) | 0, 1, or 2 | C₁-C₆-halogenalkyl | C₁-C₆-halogenalkyl |
| E.68 | (X.3) | 0, 1, or 2 | C₁-C₆-halogenalkyl | C₁-C₆-halogenalkyl |
| E.69 | (X.4) | 0, 1, or 2 | C₁-C₆-halogenalkyl | C₁-C₆-halogenalkyl |
| E.70 | (X.5) | 0, 1, or 2 | C₁-C₆-halogenalkyl | C₁-C₆-halogenalkyl |
| E.71 | (X.1) | 0, 1, or 2 | C₂-C₆-alkenyl | C₁-C₆-halogenalkyl |
| E.72 | (X.2) | 0, 1, or 2 | C₂-C₆-alkenyl | C₁-C₆-halogenalkyl |
| E.73 | (X.3) | 0, 1, or 2 | C₂-C₆-alkenyl | C₁-C₆-halogenalkyl |
| E.74 | (X.4) | 0, 1, or 2 | C₂-C₆-alkenyl | C₁-C₆-halogenalkyl |
| E.75 | (X.5) | 0, 1, or 2 | C₂-C₆-alkenyl | C₁-C₆-halogenalkyl |
| E.76 | (X.1) | 0, 1, or 2 | C₂-C₆-halogenalkenyl | C₁-C₆-halogenalkyl |
| E.77 | (X.2) | 0, 1, or 2 | C₂-C₆-halogenalkenyl | C₁-C₆-halogenalkyl |
| E.78 | (X.3) | 0, 1, or 2 | C₂-C₆-halogenalkenyl | C₁-C₆-halogenalkyl |
| E.79 | (X.4) | 0, 1, or 2 | C₂-C₆-halogenalkenyl | C₁-C₆-halogenalkyl |
| E.80 | (X.5) | 0, 1, or 2 | C₂-C₆-halogenalkenyl | C₁-C₆-halogenalkyl |
| E.81 | (X.1) | 0, 1, or 2 | C₂-C₆-alkynyl | C₁-C₆-halogenalkyl |
| E.82 | (X.2) | 0, 1, or 2 | C₂-C₆-alkynyl | C₁-C₆-halogenalkyl |
| E.83 | (X.3) | 0, 1, or 2 | C₂-C₆-alkynyl | C₁-C₆-halogenalkyl |
| E.84 | (X.4) | 0, 1, or 2 | C₂-C₆-alkynyl | C₁-C₆-halogenalkyl |
| E.85 | (X.5) | 0, 1, or 2 | C₂-C₆-alkynyl | C₁-C₆-halogenalkyl |
| E.86 | (X.1) | 0, 1, or 2 | C₂-C₆-halogenalkynyl | C₁-C₆-halogenalkyl |
| E.87 | (X.2) | 0, 1, or 2 | C₂-C₆-halogenalkynyl | C₁-C₆-halogenalkyl |
| E.88 | (X.3) | 0, 1, or 2 | C₂-C₆-halogenalkynyl | C₁-C₆-halogenalkyl |
| E.89 | (X.4) | 0, 1, or 2 | C₂-C₆-halogenalkynyl | C₁-C₆-halogenalkyl |
| E.90 | (X.5) | 0, 1, or 2 | C₂-C₆-halogenalkynyl | C₁-C₆-halogenalkyl |
| E.91 | (X.1) | 0, 1, or 2 | O-C₁-C₆-alkyl | C₁-C₆-halogenalkyl |
| E.92 | (X.2) | 0, 1, or 2 | O-C₁-C₆-alkyl | C₁-C₆-halogenalkyl |
| E.93 | (X.3) | 0, 1, or 2 | O-C₁-C₆-alkyl | C₁-C₆-halogenalkyl |
| E.94 | (X.4) | 0, 1, or 2 | O-C₁-C₆-alkyl | C₁-C₆-halogenalkyl |
| E.95 | (X.5) | 0, 1, or 2 | O-C₁-C₆-alkyl | C₁-C₆-halogenalkyl |
| E.96 | (X.1) | 0, 1, or 2 | O-C₂-C₆-alkenyl | C₁-C₆-halogenalkyl |
| E.97 | (X.2) | 0, 1, or 2 | O-C₂-C₆-alkenyl | C₁-C₆-halogenalkyl |
| E.98 | (X.3) | 0, 1, or 2 | O-C₂-C₆-alkenyl | C₁-C₆-halogenalkyl |
| E.99 | (X.4) | 0, 1, or 2 | O-C₂-C₆-alkenyl | C₁-C₆-halogenalkyl |
| E.100 | (X.5) | 0, 1, or 2 | O-C₂-C₆-alkenyl | C₁-C₆-halogenalkyl |
| E.101 | (X.1) | 0, 1, or 2 | O-C₂-C₆-alkynyl | C₁-C₆-halogenalkyl |
| E.102 | (X.2) | 0, 1, or 2 | O-C₂-C₆-alkynyl | C₁-C₆-halogenalkyl |
| E.103 | (X.3) | 0, 1, or 2 | O-C₂-C₆-alkynyl | C₁-C₆-halogenalkyl |
| E.104 | (X.4) | 0, 1, or 2 | O-C₂-C₆-alkynyl | C₁-C₆-halogenalkyl |
| E.105 | (X.5) | 0, 1, or 2 | O-C₂-C₆-alkynyl | C₁-C₆-halogenalkyl |
| E.106 | (X.1) | 0, 1, or 2 | C₃-C₆-cycloalkyl | C₁-C₆-halogenalkyl |
| E.107 | (X.2) | 0, 1, or 2 | C₃-C₆-cycloalkyl | C₁-C₆-halogenalkyl |
| E.108 | (X.3) | 0, 1, or 2 | C₃-C₆-cycloalkyl | C₁-C₆-halogenalkyl |
| E.109 | (X.4) | 0, 1, or 2 | C₃-C₆-cycloalkyl | C₁-C₆-halogenalkyl |
| E.110 | (X.5) | 0, 1, or 2 | C₃-C₆-cycloalkyl | C₁-C₆-halogenalkyl |
| E.111 | (X.1) | 0, 1, or 2 | C₁-C₆-alkyl | O-C₁-C₆-alkyl |
| E.112 | (X.2) | 0, 1, or 2 | C₁-C₆-alkyl | O-C₁-C₆-alkyl |
| E.113 | (X.3) | 0, 1, or 2 | C₁-C₆-alkyl | O-C₁-C₆-alkyl |
| E.114 | (X.4) | 0, 1, or 2 | C₁-C₆-alkyl | O-C₁-C₆-alkyl |
| E.115 | (X.5) | 0, 1, or 2 | C₁-C₆-alkyl | O-C₁-C₆-alkyl |
| E.116 | (X.1) | 0, 1, or 2 | halogen | O-C₁-C₆-alkyl |
| E.117 | (X.2) | 0, 1, or 2 | halogen | O-C₁-C₆-alkyl |
| E.118 | (X.3) | 0, 1, or 2 | halogen | O-C₁-C₆-alkyl |
| E.119 | (X.4) | 0, 1, or 2 | halogen | O-C₁-C₆-alkyl |
| E.120 | (X.5) | 0, 1, or 2 | halogen | O-C₁-C₆-alkyl |
| E.121 | (X.1) | 0, 1, or 2 | C₁-C₆-halogenalkyl | O-C₁-C₆-alkyl |
| E.122 | (X.2) | 0, 1, or 2 | C₁-C₆-halogenalkyl | O-C₁-C₆-alkyl |
| E.123 | (X.3) | 0, 1, or 2 | C₁-C₆-halogenalkyl | O-C₁-C₆-alkyl |
| E.124 | (X.4) | 0, 1, or 2 | C₁-C₆-halogenalkyl | O-C₁-C₆-alkyl |
| E.125 | (X.5) | 0, 1, or 2 | C₁-C₆-halogenalkyl | O-C₁-C₆-alkyl |
| E.126 | (X.1) | 0, 1, or 2 | C₂-C₆-alkenyl | O-C₁-C₆-alkyl |
| E.127 | (X.2) | 0, 1, or 2 | C₂-C₆-alkenyl | O-C₁-C₆-alkyl |
| E.128 | (X.3) | 0, 1, or 2 | C₂-C₆-alkenyl | O-C₁-C₆-alkyl |
| E.129 | (X.4) | 0, 1, or 2 | C₂-C₆-alkenyl | O-C₁-C₆-alkyl |
| E.130 | (X.5) | 0, 1, or 2 | C₂-C₆-alkenyl | O-C₁-C₆-alkyl |
| E.131 | (X.1) | 0, 1, or 2 | C₂-C₆-halogenalkenyl | O-C₁-C₆-alkyl |
| E.132 | (X.2) | 0, 1, or 2 | C₂-C₆-halogenalkenyl | O-C₁-C₆-alkyl |
| E.133 | (X.3) | 0, 1, or 2 | C₂-C₆-halogenalkenyl | O-C₁-C₆-alkyl |
| E.134 | (X.4) | 0, 1, or 2 | C₂-C₆-halogenalkenyl | O-C₁-C₆-alkyl |
| E.135 | (X.5) | 0, 1, or 2 | C₂-C₆-halogenalkenyl | O-C₁-C₆-alkyl |
| E.136 | (X.1) | 0, 1, or 2 | C₂-C₆-alkynyl | O-C₁-C₆-alkyl |
| E.137 | (X.2) | 0, 1, or 2 | C₂-C₆-alkynyl | O-C₁-C₆-alkyl |
| E.138 | (X.3) | 0, 1, or 2 | C₂-C₆-alkynyl | O-C₁-C₆-alkyl |
| E.139 | (X.4) | 0, 1, or 2 | C₂-C₆-alkynyl | O-C₁-C₆-alkyl |
| E.140 | (X.5) | 0, 1, or 2 | C₂-C₆-alkynyl | O-C₁-C₆-alkyl |
| E.141 | (X.1) | 0, 1, or 2 | C₂-C₆-halogenalkynyl | O-C₁-C₆-alkyl |
| E.142 | (X.2) | 0, 1, or 2 | C₂-C₆-halogenalkynyl | O-C₁-C₆-alkyl |
| E.143 | (X.3) | 0, 1, or 2 | C₂-C₆-halogenalkynyl | O-C₁-C₆-alkyl |
| E.144 | (X.4) | 0, 1, or 2 | C₂-C₆-halogenalkynyl | O-C₁-C₆-alkyl |
| E.145 | (X.5) | 0, 1, or 2 | C₂-C₆-halogenalkynyl | O-C₁-C₆-alkyl |
| E.146 | (X.1) | 0, 1, or 2 | O-C₁-C₆-alkyl | O-C₁-C₆-alkyl |
| E.147 | (X.2) | 0, 1, or 2 | O-C₁-C₆-alkyl | O-C₁-C₆-alkyl |
| E.148 | (X.3) | 0, 1, or 2 | O-C₁-C₆-alkyl | O-C₁-C₆-alkyl |
| E.149 | (X.4) | 0, 1, or 2 | O-C₁-C₆-alkyl | O-C₁-C₆-alkyl |
| E.150 | (X.5) | 0, 1, or 2 | O-C₁-C₆-alkyl | O-C₁-C₆-alkyl |
| E.151 | (X.1) | 0, 1, or 2 | O-C₂-C₆-alkenyl | O-C₁-C₆-alkyl |
| E.152 | (X.2) | 0, 1, or 2 | O-C₂-C₆-alkenyl | O-C₁-C₆-alkyl |
| E.153 | (X.3) | 0, 1, or 2 | O-C₂-C₆-alkenyl | O-C₁-C₆-alkyl |
| E.154 | (X.4) | 0, 1, or 2 | O-C₂-C₆-alkenyl | O-C₁-C₆-alkyl |
| E.155 | (X.5) | 0, 1, or 2 | O-C₂-C₆-alkenyl | O-C₁-C₆-alkyl |
| E.156 | (X.1) | 0, 1, or 2 | O-C₂-C₆-alkynyl | O-C₁-C₆-alkyl |
| E.157 | (X.2) | 0, 1, or 2 | O-C₂-C₆-alkynyl | O-C₁-C₆-alkyl |
| E.158 | (X.3) | 0, 1, or 2 | O-C₂-C₆-alkynyl | O-C₁-C₆-alkyl |
| E.159 | (X.4) | 0, 1, or 2 | O-C₂-C₆-alkynyl | O-C₁-C₆-alkyl |
| E.160 | (X.5) | 0, 1, or 2 | O-C₂-C₆-alkynyl | O-C₁-C₆-alkyl |
| E.161 | (X.1) | 0, 1, or 2 | C₃-C₆-cycloalkyl | O-C₁-C₆-alkyl |
| E.162 | (X.2) | 0, 1, or 2 | C₃-C₆-cycloalkyl | O-C₁-C₆-alkyl |
| E.163 | (X.3) | 0, 1, or 2 | C₃-C₆-cycloalkyl | O-C₁-C₆-alkyl |
| E.164 | (X.4) | 0, 1, or 2 | C₃-C₆-cycloalkyl | O-C₁-C₆-alkyl |
| E.165 | (X.5) | 0, 1, or 2 | C₃-C₆-cycloalkyl | O-C₁-C₆-alkyl |
| E.166 | (X.1) | 0, 1, or 2 | C₁-C₆-alkyl | C₃-C₆-cycloalkyl |
| E.167 | (X.2) | 0, 1, or 2 | C₁-C₆-alkyl | C₃-C₆-cycloalkyl |
| E.168 | (X.3) | 0, 1, or 2 | C₁-C₆-alkyl | C₃-C₆-cycloalkyl |
| E.169 | (X.4) | 0, 1, or 2 | C₁-C₆-alkyl | C₃-C₆-cycloalkyl |
| E.170 | (X.5) | 0, 1, or 2 | C₁-C₆-alkyl | C₃-C₆-cycloalkyl |
| E.171 | (X.1) | 0, 1, or 2 | halogen | C₃-C₆-cycloalkyl |
| E.172 | (X.2) | 0, 1, or 2 | halogen | C₃-C₆-cycloalkyl |
| E.173 | (X.3) | 0, 1, or 2 | halogen | C₃-C₆-cycloalkyl |
| E.174 | (X.4) | 0, 1, or 2 | halogen | C₃-C₆-cycloalkyl |
| E.175 | (X.5) | 0, 1, or 2 | halogen | C₃-C₆-cycloalkyl |
| E.176 | (X.1) | 0, 1, or 2 | C₁-C₆-halogenalkyl | C₃-C₆-cycloalkyl |
| E.177 | (X.2) | 0, 1, or 2 | C₁-C₆-halogenalkyl | C₃-C₆-cycloalkyl |
| E.178 | (X.3) | 0, 1, or 2 | C₁-C₆-halogenalkyl | C₃-C₆-cycloalkyl |
| E.179 | (X.4) | 0, 1, or 2 | C₁-C₆-halogenalkyl | C₃-C₆-cycloalkyl |
| E.180 | (X.5) | 0, 1, or 2 | C₁-C₆-halogenalkyl | C₃-C₆-cycloalkyl |
| E.181 | (X.1) | 0, 1, or 2 | C₂-C₆-alkenyl | C₃-C₆-cycloalkyl |
| E.182 | (X.2) | 0, 1, or 2 | C₂-C₆-alkenyl | C₃-C₆-cycloalkyl |
| E.183 | (X.3) | 0, 1, or 2 | C₂-C₆-alkenyl | C₃-C₆-cycloalkyl |
| E.184 | (X.4) | 0, 1, or 2 | C₂-C₆-alkenyl | C₃-C₆-cycloalkyl |
| E.185 | (X.5) | 0, 1, or 2 | C₂-C₆-alkenyl | C₃-C₆-cycloalkyl |
| E.186 | (X.1) | 0, 1, or 2 | C₂-C₆-halogenalkenyl | C₃-C₆-cycloalkyl |
| E.187 | (X.2) | 0, 1, or 2 | C₂-C₆-halogenalkenyl | C₃-C₆-cycloalkyl |
| E.188 | (X.3) | 0, 1, or 2 | C₂-C₆-halogenalkenyl | C₃-C₆-cycloalkyl |
| E.189 | (X.4) | 0, 1, or 2 | C₂-C₆-halogenalkenyl | C₃-C₆-cycloalkyl |
| E.190 | (X.5) | 0, 1, or 2 | C₂-C₆-halogenalkenyl | C₃-C₆-cycloalkyl |
| E.191 | (X.1) | 0, 1, or 2 | C₂-C₆-alkynyl | C₃-C₆-cycloalkyl |
| E.192 | (X.2) | 0, 1, or 2 | C₂-C₆-alkynyl | C₃-C₆-cycloalkyl |
| E.193 | (X.3) | 0, 1, or 2 | C₂-C₆-alkynyl | C₃-C₆-cycloalkyl |
| E.194 | (X.4) | 0, 1, or 2 | C₂-C₆-alkynyl | C₃-C₆-cycloalkyl |
| E.195 | (X.5) | 0, 1, or 2 | C₂-C₆-alkynyl | C₃-C₆-cycloalkyl |
| E.196 | (X.1) | 0, 1, or 2 | C₂-C₆-halogenalkynyl | C₃-C₆-cycloalkyl |
| E.197 | (X.2) | 0, 1, or 2 | C₂-C₆-halogenalkynyl | C₃-C₆-cycloalkyl |
| E.198 | (X.3) | 0, 1, or 2 | C₂-C₆-halogenalkynyl | C₃-C₆-cycloalkyl |
| E.199 | (X.4) | 0, 1, or 2 | C₂-C₆-halogenalkynyl | C₃-C₆-cycloalkyl |
| E.200 | (X.5) | 0, 1, or 2 | C₂-C₆-halogenalkynyl | C₃-C₆-cycloalkyl |
| E.201 | (X.1) | 0, 1, or 2 | O-C₁-C₆-alkyl | C₃-C₆-cycloalkyl |
| E.202 | (X.2) | 0, 1, or 2 | O-C₁-C₆-alkyl | C₃-C₆-cycloalkyl |
| E.203 | (X.3) | 0, 1, or 2 | O-C₁-C₆-alkyl | C₃-C₆-cycloalkyl |
| E.204 | (X.4) | 0, 1, or 2 | O-C₁-C₆-alkyl | C₃-C₆-cycloalkyl |
| E.205 | (X.5) | 0, 1, or 2 | O-C₁-C₆-alkyl | C₃-C₆-cycloalkyl |
| E.206 | (X.1) | 0, 1, or 2 | O-C₂-C₆-alkenyl | C₃-C₆-cycloalkyl |
| E.207 | (X.2) | 0, 1, or 2 | O-C₂-C₆-alkenyl | C₃-C₆-cycloalkyl |
| E.208 | (X.3) | 0, 1, or 2 | O-C₂-C₆-alkenyl | C₃-C₆-cycloalkyl |
| E.209 | (X.4) | 0, 1, or 2 | O-C₂-C₆-alkenyl | C₃-C₆-cycloalkyl |
| E.210 | (X.5) | 0, 1, or 2 | O-C₂-C₆-alkenyl | C₃-C₆-cycloalkyl |
| E.211 | (X.1) | 0, 1, or 2 | O-C₂-C₆-alkynyl | C₃-C₆-cycloalkyl |
| E.212 | (X.2) | 0, 1, or 2 | O-C₂-C₆-alkynyl | C₃-C₆-cycloalkyl |
| E.213 | (X.3) | 0, 1, or 2 | O-C₂-C₆-alkynyl | C₃-C₆-cycloalkyl |
| E.214 | (X.4) | 0, 1, or 2 | O-C₂-C₆-alkynyl | C₃-C₆-cycloalkyl |
| E.215 | (X.5) | 0, 1, or 2 | O-C₂-C₆-alkynyl | C₃-C₆-cycloalkyl |
| E.216 | (X.1) | 0, 1, or 2 | C₃-C₆-cycloalkyl | C₃-C₆-cycloalkyl |
| E.217 | (X.2) | 0, 1, or 2 | C₃-C₆-cycloalkyl | C₃-C₆-cycloalkyl |
| E.218 | (X.3) | 0, 1, or 2 | C₃-C₆-cycloalkyl | C₃-C₆-cycloalkyl |
| E.219 | (X.4) | 0, 1, or 2 | C₃-C₆-cycloalkyl | C₃-C₆-cycloalkyl |
| E.220 | (X.5) | 0, 1, or 2 | C₃-C₆-cycloalkyl | C₃-C₆-cycloalkyl |
| E.221 | (X.1) | 0, 1, or 2 | C₁-C₆-alkyl | C₂-C₆-alkenyl |
| E.222 | (X.2) | 0, 1, or 2 | C₁-C₆-alkyl | C₂-C₆-alkenyl |
| E.223 | (X.3) | 0, 1, or 2 | C₁-C₆-alkyl | C₂-C₆-alkenyl |
| E.224 | (X.4) | 0, 1, or 2 | C₁-C₆-alkyl | C₂-C₆-alkenyl |
| E.225 | (X.5) | 0, 1, or 2 | C₁-C₆-alkyl | C₂-C₆-alkenyl |
| E.226 | (X.1) | 0, 1, or 2 | halogen | C₂-C₆-alkenyl |
| E.227 | (X.2) | 0, 1, or 2 | halogen | C₂-C₆-alkenyl |
| E.228 | (X.3) | 0, 1, or 2 | halogen | C₂-C₆-alkenyl |
| E.229 | (X.4) | 0, 1, or 2 | halogen | C₂-C₆-alkenyl |
| E.230 | (X.5) | 0, 1, or 2 | halogen | C₂-C₆-alkenyl |
| E.231 | (X.1) | 0, 1, or 2 | C₁-C₆-halogenalkyl | C₂-C₆-alkenyl |
| E.232 | (X.2) | 0, 1, or 2 | C₁-C₆-halogenalkyl | C₂-C₆-alkenyl |
| E.233 | (X.3) | 0, 1, or 2 | C₁-C₆-halogenalkyl | C₂-C₆-alkenyl |
| E.234 | (X.4) | 0, 1, or 2 | C₁-C₆-halogenalkyl | C₂-C₆-alkenyl |
| E.235 | (X.5) | 0, 1, or 2 | C₁-C₆-halogenalkyl | C₂-C₆-alkenyl |
| E.236 | (X.1) | 0, 1, or 2 | C₂-C₆-alkenyl | C₂-C₆-alkenyl |
| E.237 | (X.2) | 0, 1, or 2 | C₂-C₆-alkenyl | C₂-C₆-alkenyl |
| E.238 | (X.3) | 0, 1, or 2 | C₂-C₆-alkenyl | C₂-C₆-alkenyl |
| E.239 | (X.4) | 0, 1, or 2 | C₂-C₆-alkenyl | C₂-C₆-alkenyl |
| E.240 | (X.5) | 0, 1, or 2 | C₂-C₆-alkenyl | C₂-C₆-alkenyl |
| E.241 | (X.1) | 0, 1, or 2 | C₂-C₆-halogenalkenyl | C₂-C₆-alkenyl |
| E.242 | (X.2) | 0, 1, or 2 | C₂-C₆-halogenalkenyl | C₂-C₆-alkenyl |
| E.243 | (X.3) | 0, 1, or 2 | C₂-C₆-halogenalkenyl | C₂-C₆-alkenyl |
| E.244 | (X.4) | 0, 1, or 2 | C₂-C₆-halogenalkenyl | C₂-C₆-alkenyl |
| E.245 | (X.5) | 0, 1, or 2 | C₂-C₆-halogenalkenyl | C₂-C₆-alkenyl |
| E.246 | (X.1) | 0, 1, or 2 | C₂-C₆-alkynyl | C₂-C₆-alkenyl |
| E.247 | (X.2) | 0, 1, or 2 | C₂-C₆-alkynyl | C₂-C₆-alkenyl |
| E.248 | (X.3) | 0, 1, or 2 | C₂-C₆-alkynyl | C₂-C₆-alkenyl |
| E.249 | (X.4) | 0, 1, or 2 | C₂-C₆-alkynyl | C₂-C₆-alkenyl |
| E.250 | (X.5) | 0, 1, or 2 | C₂-C₆-alkynyl | C₂-C₆-alkenyl |
| E.251 | (X.1) | 0, 1, or 2 | C₂-C₆-halogenalkynyl | C₂-C₆-alkenyl |
| E.252 | (X.2) | 0, 1, or 2 | C₂-C₆-halogenalkynyl | C₂-C₆-alkenyl |
| E.253 | (X.3) | 0, 1, or 2 | C₂-C₆-halogenalkynyl | C₂-C₆-alkenyl |
| E.254 | (X.4) | 0, 1, or 2 | C₂-C₆-halogenalkynyl | C₂-C₆-alkenyl |
| E.255 | (X.5) | 0, 1, or 2 | C₂-C₆-halogenalkynyl | C₂-C₆-alkenyl |
| E.256 | (X.1) | 0, 1, or 2 | O-C₁-C₆-alkyl | C₂-C₆-alkenyl |
| E.257 | (X.2) | 0, 1, or 2 | O-C₁-C₆-alkyl | C₂-C₆-alkenyl |
| E.258 | (X.3) | 0, 1, or 2 | O-C₁-C₆-alkyl | C₂-C₆-alkenyl |
| E.259 | (X.4) | 0, 1, or 2 | O-C₁-C₆-alkyl | C₂-C₆-alkenyl |
| E.260 | (X.5) | 0, 1, or 2 | O-C₁-C₆-alkyl | C₂-C₆-alkenyl |
| E.261 | (X.1) | 0, 1, or 2 | O-C₂-Cₑ-alkenyl | C₂-C₆-alkenyl |
| E.262 | (X.2) | 0, 1, or 2 | O-C₂-Cₑ-alkenyl | C₂-C₆-alkenyl |
| E.263 | (X.3) | 0, 1, or 2 | O-C₂-C₆-alkenyl | C₂-C₆-alkenyl |
| E.264 | (X.4) | 0, 1, or 2 | O-C₂-Cₑ-alkenyl | C₂-C₆-alkenyl |
| E.265 | (X.5) | 0, 1, or 2 | O-C₂-C₆-alkenyl | C₂-C₆-alkenyl |
| E.266 | (X.1) | 0, 1, or 2 | O-C₂-C₆-alkynyl | C₂-C₆-alkenyl |
| E.267 | (X.2) | 0, 1, or 2 | O-C₂-C₆-alkynyl | C₂-C₆-alkenyl |
| E.268 | (X.3) | 0, 1, or 2 | O-C₂-C₆-alkynyl | C₂-C₆-alkenyl |
| E.269 | (X.4) | 0, 1, or 2 | O-C₂-C₆-alkynyl | C₂-C₆-alkenyl |
| E.270 | (X.5) | 0, 1, or 2 | O-C₂-C₆-alkynyl | C₂-C₆-alkenyl |
| E.271 | (X.1) | 0, 1, or 2 | C₃-C₆-cycloalkyl | C₂-C₆-alkenyl |
| E.272 | (X.2) | 0, 1, or 2 | C₃-C₆-cycloalkyl | C₂-C₆-alkenyl |
| E.273 | (X.3) | 0, 1, or 2 | C₃-C₆-cycloalkyl | C₂-C₆-alkenyl |
| E.274 | (X.4) | 0, 1, or 2 | C₃-C₆-cycloalkyl | C₂-C₆-alkenyl |
| E.275 | (X.5) | 0, 1, or 2 | C₃-C₆-cycloalkyl | C₂-C₆-alkenyl |

In further aspects the present invention relates to the embodiments E.1 to E.275 listed in Table E, wherein R⁵ is represented by embodiment 5.1 and R⁶ is represented by embodiment 6.1.

In further aspects the present invention relates to the embodiments E.1 to E.275 listed in Table E, wherein R⁵ is represented by embodiment 5.2 and R⁶ is represented by embodiment 6.1.

In further aspects the present invention relates to the embodiments E.1 to E.275 listed in Table E, wherein R⁵ is represented by embodiment 5.3 and R⁶ is represented by embodiment 6.1.

In further aspects the present invention relates to the embodiments E.1 to E.275 listed in Table E, wherein R⁵ is represented by embodiment 5.4 and R⁶ is represented by embodiment 6.1.

In further aspects the present invention relates to the embodiments E.1 to E.275 listed in Table E, wherein R⁵ is represented by embodiment 5.1 and R⁶ is represented by embodiment 6.2.

In further aspects the present invention relates to the embodiments E.1 to E.275 listed in Table E, wherein R⁵ is represented by embodiment 5.2 and R⁶ is represented by embodiment 6.2.

In further aspects the present invention relates to the embodiments E.1 to E.275 listed in Table E, wherein R⁵ is represented by embodiment 5.3 and R⁶ is represented by embodiment 6.2.

In further aspects the present invention relates to the embodiments E.1 to E.275 listed in Table E, wherein R⁵ is represented by embodiment 5.4 and R⁶ is represented by embodiment 6.2.

In further aspects the present invention relates to the embodiments E.1 to E.275 listed in Table E, wherein R⁵ is represented by embodiment 5.1 and R⁶ is represented by embodiment 6.3.

In further aspects the present invention relates to the embodiments E.1 to E.275 listed in Table E, wherein R⁵ is represented by embodiment 5.2 and R⁶ is represented by embodiment 6.3.

In further aspects the present invention relates to the embodiments E.1 to E.275 listed in Table E, wherein R⁵ is represented by embodiment 5.3 and R⁶ is represented by embodiment 6.3.

In further aspects the present invention relates to the embodiments E.1 to E.275 listed in Table E, wherein R⁵ is represented by embodiment 5.4 and R⁶ is represented by embodiment 6.3.

In further aspects the present invention relates to the embodiments E.1 to E.275 listed in Table E, wherein R⁵ and R⁶ arerepresented by embodiment 6.4.

In further aspects the present invention relates to the embodiments E.1 to E.275 listed in Table E, wherein R⁵ and R⁶ arerepresented by embodiment 6.5.

In further aspects the present invention relates to the embodiments E.1 to E.275 listed in Table E, wherein R⁷ is represented by embodiment 7.1 and R⁸ is represented by embodiment 8.1.

In further aspects the present invention relates to the embodiments E.1 to E.275 listed in Table E, wherein R⁷ is represented by embodiment 7.2 and R⁸ is represented by embodiment 8.1.

In further aspects the present invention relates to the embodiments E.1 to E.275 listed in Table E, wherein R⁷ is represented by embodiment 7.3 and R⁸ is represented by embodiment 8.1.

In further aspects the present invention relates to the embodiments E.1 to E.275 listed in Table E, wherein R⁷ is represented by embodiment 7.4 and R⁸ is represented by embodiment 8.1.

In further aspects the present invention relates to the embodiments E.1 to E.275 listed in Table E, wherein R⁷ is represented by embodiment 7.1 and R⁸ is represented by embodiment 8.2.

In further aspects the present invention relates to the embodiments E.1 to E.277 listed in Table E, wherein R⁷ is represented by embodiment 7.2 and R⁸ is represented by embodiment 8.2.

In further aspects the present invention relates to the embodiments E.1 to E.277 listed in Table E, wherein R⁷ is represented by embodiment 7.3 and R⁸ is represented by embodiment 8.2.

In further aspects the present invention relates to the embodiments E.1 to E.277 listed in Table E, wherein R⁷ is represented by embodiment 7.4 and R⁸ is represented by embodiment 8.2.

In further aspects the present invention relates to the embodiments E.1 to E.277 listed in Table E, wherein R⁷ is represented by embodiment 7.1 and R⁸ is represented by embodiment 8.3.

In further aspects the present invention relates to the embodiments E.1 to E.277 listed in Table E, wherein R⁷ is represented by embodiment 7.2 and R⁸ is represented by embodiment 8.3.

In further aspects the present invention relates to the embodiments E.1 to E.277 listed in Table E, wherein R⁷ is represented by embodiment 7.3 and R⁸ is represented by embodiment 8.3.

In further aspects the present invention relates to the embodiments E.1 to E.277 listed in Table E, wherein R⁷ is represented by embodiment 7.4 and R⁸ is represented by embodiment 8.3.

In further aspects the present invention relates to the embodiments E.1 to E.277 listed in Table E, wherein R⁷ and R⁸ arerepresented by embodiment 8.4.

In further aspects the present invention relates to the embodiments E.1 to E.277 listed in Table E, wherein R⁷ and R⁸ arerepresented by embodiment 8.7.

Preferred embodiments of the present invention are the following compounds I.A-1, I.A-2, I.A-3, I.A-4. In these formulae, the substituents R⁵, R⁶, R⁷ and R⁸ and Xn are independently as defined above or preferably defined herein:

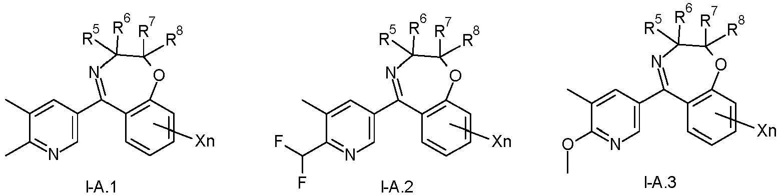

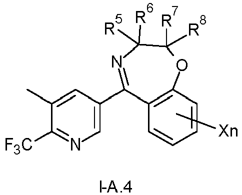

In particular with a view to their use, according to one embodiment, preference is given to the compounds of the compounds I.A-1, I.A-2, I.A-3, I.A-4; that are compiled in the Tables 1a to 7a. Each of the groups mentioned for a substituent in the tables is furthermore per se, independently of the combination in which it is mentioned, a particularly preferred aspect of the substituent in question.

Table 1a Compounds of the formula I.A-1, I.A-2, I.A-3, I.A-4 in which Xn is H and the meaning for the combination of R⁵ and R⁶ for each individual compound corresponds in each case to one line of Table B (compounds I.A-1.1a.B-1 to I.A-1.1a.B-25, I.A-2.1a.B-1 to I.A-2.1a.B-25, I.A-3.1a.B-1 to I.A-3.1a.B-25, I.A-4.1a.B-1 to I.A-4.1a.B-25).

Table 2a Compounds of the formula I.A-1, I.A-2, I.A-3, I.A-4; in which Xn is 8-F and the meaning for the combination of R⁵ and R⁶ for each individual compound corresponds in each case to one line of Table B (compounds I.A-1.2a.B-1 to I.A-1.2a.B-25, I.A-2.2a.B-1 to I.A-2.2a.B-25, I.A-3.2a.B-1 to I.A-3.2a.B-25, I.A-4.2a.B-1 to I.A-4.2a.B-25).

Table 3a Compounds of the formula I.A-1, I.A-2, I.A-3, I.A-4; in which Xn is 8-Cl and the meaning for the combination of R⁵ and R⁶ for each individual compound corresponds in each case to one line of Table B (compounds I.A-1.3a.B-1 to I.A-1.3a.B-25, I.A-2.3a.B-1 to I.A-2.3a.B-25, I.A-3.3a.B-1 to I.A-3.3a.B-25, I.A-4.3a.B-1 to I.A-4.3a.B-25)

Table 4a Compounds of the formula I.A-1, I.A-2, I.A-3, I.A-4; in which Xn is 7,8-F₂ and the meaning for the combination of R⁵ and R⁶ for each individual compound corresponds in each case to one line of Table B (compounds I.A-1.4a.B-1 to I.A-1.4a.B-25, I.A-2.4a.B-1 to I.A-2.4a.B-25, I.A-3.4a.B-1 to I.A-3.4a.B-25, I.A-4.4a.B-1 to I.A-4.4a.B-25).

Table 5a Compounds of the formula I.A-1, I.A-2, I.A-3, I.A-4; in which Xn is 5,8-F₂ and the meaning for the combination of R⁵ and R⁶ for each individual compound corresponds in each case to one line of Table B (compounds I.A-1.5a.B-1 to I.A-1.5a.B-25, I.A-2.5a.B-1 to I.A-2.5a.B-25, I.A-3.5a.B-1 to I.A-3.5a.B-25, I.A-4.5a.B-1 to I.A-4.5a.B-25)

Table 6a Compounds of the formula I.A-1, I.A-2, I.A-3, I.A-4; in which Xn is 7-OCH₃ and the meaning for the combination of R⁵ and R⁶ for each individual compound corresponds in each case to one line of Table B (compounds I.A-1.6a.B-1 to I.A-1.6a.B-25, I.A-2.6a.B-1 to I.A-2.6a.B-25, I.A-3.6a.B-1 to I.A-3.6a.B-25, I.A-4.6a.B-1 to I.A-4.6a.B-25

Table 7a Compounds of the formula I.A-1, I.A-2, I.A-3, I.A-4; in which Xn is 6,8-F₂ and the meaning for the combination of R⁵ and R⁶ for each individual compound corresponds in each case to one line of Table B (compounds I.A-1.7a.B-1 to I.A-1.7a.B-25, I.A-2.7a.B-1 to I.A-2.7a.B-25, I.A-3.7a.B-1 to I.A-3.7a.B-25, I.A-4.7a.B-1 to I.A-4.7a.B-25)

Preferred embodiments of the present invention are the following compounds I.B-1, I.B-2, I.B-3, I.B-4. In these formulae, the substituents R⁵, R⁶, R⁷ and R⁸ and Xn are independently as defined above or preferably defined herein:

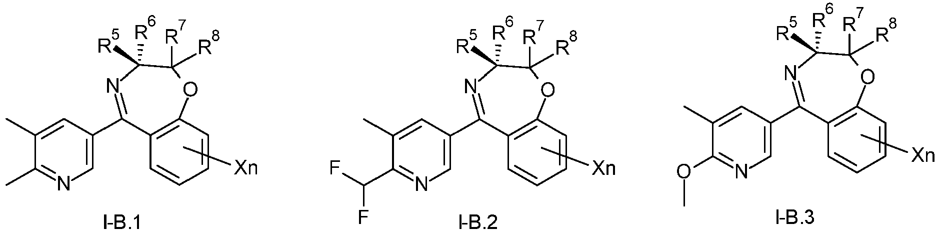

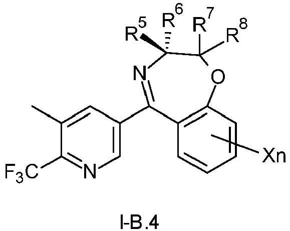

In particular with a view to their use, according to one embodiment, preference is given to the compounds of the compounds I.B-1, I.B-2, I.B-3, I.B-4; that are compiled in the Tables 1b to 7b. Each of the groups mentioned for a substituent in the tables is furthermore per se, independently of the combination in which it is mentioned, a particularly preferred aspect of the substituent in question.

Table 1b Compounds of the formula I.B-1, I.B-2, I.B-3, I.B-4 in which Xn is H and the meaning for the combination of R⁵ and R⁶ for each individual compound corresponds in each case to one line of Table B (compounds I.B-1.1b.B-1 to I.B-1.1b.B-25, I.B-2.1b.B-1 to I.B-2.1b.B-25, I.B-3.1b.B-1 to I.B-3.1b.B-25, I.B-4.1b.B-1 to I.B-4.1b.B-25).

Table 2b Compounds of the formula I.B-1, I.B-2, I.B-3, I.B-4; in which Xn is 8-F and the meaning for the combination of R⁵ and R⁶ for each individual compound corresponds in each case to one line of Table B (compounds I.B-1.2b.B-1 to I.B-1.2b.B-25, I.B-2.2b.B-1 to I.B-2.2b.B-25, I.B-3.2b.B-1 to I.B-3.2b.B-25, I.B-4.2b.B-1 to I.B-4.2b.B-25).

Table 3b Compounds of the formula I.B-1, I.B-2, I.B-3, I.B-4; in which Xn is 8-Cl and the meaning for the combination of R⁵ and R⁶ for each individual compound corresponds in each case to one line of Table B (compounds I.B-1.3b.B-1 to I.B-1.3b.B-25, I.B-2.3b.B-1 to I.B-2.3b.B-25, I.B-3.3b.B-1 to I.B-3.3b.B-25, I.B-4.3b.B-1 to I.B-4.3b.B-25)

Table 4b Compounds of the formula I.B-1, I.B-2, I.B-3, I.B-4; in which Xn is 7,8-F₂ and the meaning for the combination of R⁵ and R⁶ for each individual compound corresponds in each case to one line of Table B (compounds I.B-1.4b.B-1 to I.B-1.4b.B-25, I.B-2.4b.B-1 to I.B-2.4b.B-25, I.B-3.4b.B-1 to I.B-3.4b.B-25, I.B-4.4b.B-1 to I.B-4.4b.B-25).

Table 5b Compounds of the formula I.B-1, I.B-2, I.B-3, I.B-4; in which Xn is 5,8-F₂ and the meaning for the combination of R⁵ and R⁶ for each individual compound corresponds in each case to one line of Table B (compounds I.B-1.5b.B-1 to I.B-1.5b.B-25, I.B-2.5b.B-1 to I.B-2.5b.B-25, I.B-3.5b.B-1 to I.B-3.5b.B-25, I.B-4.5b.B-1 to I.B-4.5b.B-25)

Table 6b Compounds of the formula I.B-1, I.B-2, I.B-3, I.B-4; in which Xn is 7-OCH₃ and the meaning for the combination of R⁵ and R⁶ for each individual compound corresponds in each case to one line of Table B (compounds I.B-1.6b.B-1 to I.B-1.6b.B-25, I.B-2.6b.B-1 to I.B-2.6b.B-25, I.B-3.6b.B-1 to I.B-3.6b.B-25, I.B-4.6b.B-1 to I.B-4.6b.B-25

Table 7b Compounds of the formula I.B-1, I.B-2, I.B-3, I.B-4; in which Xn is 6,8-F₂ and the meaning for the combination of R⁵ and R⁶ for each individual compound corresponds in each case to one line of Table B (compounds I.B-1.7b.B-1 to I.B-1.7b.B-25, I.B-2.7b.B-1 to I.B-2.7b.B-25, I.B-3.7b.B-1 to I.B-3.7b.B-25, I.B-4.7b.B-1 to I.B-4.7b.B-25)

**Table B**

| **No.** | **R⁵** | | **R⁶** | **R⁷** | **R⁸** |
|---|---|---|---|---|---|
| B-1 | H | | H | H | H |
| B-2 | H | | CH₃ | H | H |
| B-3 | CH₃ | | CH₃ | H | H |
| B-4 | -CH₂-CH₂- | | | H | H |
| B-5 | -CH₂-CH₂-CH₂- | | | H | H |
| B-6 | H | H | | CH₃ | H |
| B-7 | H | CH₃ | | CH₃ | H |
| B-8 | CH₃ | CH₃ | | CH₃ | H |
| B-9 | -CH₂-CH₂- | | | CH₃ | H |
| B-10 | -CH₂-CH₂-CH₂- | | | CH₃ | H |
| B-11 | H | H | | CH₃ | CH₃ |
| B-12 | H | CH₃ | | CH₃ | CH₃ |
| B-13 | CH₃ | CH₃ | | CH₃ | CH₃ |
| B-14 | -CH₂-CH₂- | | | CH₃ | CH₃ |
| B-15 | -CH₂-CH₂-CH₂- | | | CH₃ | CH₃ |
| B-16 | H | H | | -CH₂-CH₂- | |
| B-17 | H | CH₃ | | -CH₂-CH₂- | |
| B-18 | CH₃ | CH₃ | | -CH₂-CH₂- | |
| B-19 | -CH₂-CH₂- | | | -CH₂-CH₂- | |
| B-20 | -CH₂-CH₂-CH₂- | | | -CH₂-CH₂- | |
| B-21 | H | H | | -CH₂-CH₂-CH₂- | |
| B-22 | H | CH₃ | | -CH₂-CH₂-CH₂- | |
| B-23 | CH₃ | CH₃ | | -CH₂-CH₂-CH₂- | |
| B-24 | -CH₂-CH₂- | | | -CH₂-CH₂-CH₂- | |
| B-25 | -CH₂-CH₂-CH₂- | | | -CH₂-CH₂-CH₂- | |

Compounds of the present invention can be made as shown in the following schemes, in which, unless otherwise stated, the definition of each variable is as defined above for a compound of formula I. The compounds of the formula I can be prepared according to methods or in analogy to methods that are described in the prior art. The synthesis takes advantage of starting materials that are commercially available or may be prepared according to conventional procedures starting from readily available compounds.

For example, compounds I can be prepared by a by palladium catalyzed Suzuki coupling reaction between a boronic acid derivative represented by formula (2) and an imidoyl halide derivative represented by formula (6) using a palladium complex in an organic solvent. It is preferred to conduct the reaction at elevated temperature, preferably between 60 and 160 °C, and using 1-3 equivalents of boronic acid derivative represented by formula 3 per 1 imidoyl halide derivative (6), as described in WO2009119089A1.

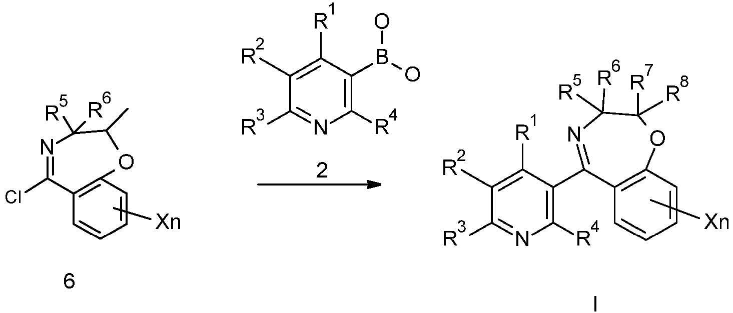

An imidoyl compound represented by formula (6) can be prepared by a method in which a cyclic amide represented by formula (7) is reacting in the presence of an suitable halogenating agent such as triphenylphosphine and a carbon tetrahalide, triphenylphosphine dichloride, phosgene, oxalyl chloride or thionyl chloride as described in US 2011/0136782 A1.

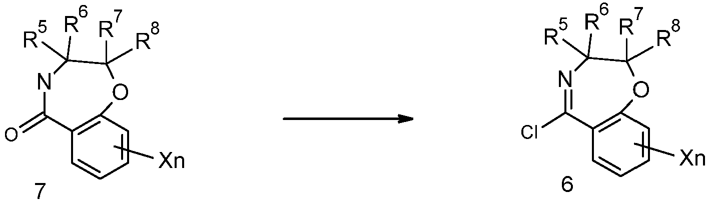

A compound represented by formula (7) can be prepared by deriving a cyclic acetophenone derivative represented by formula (9) to an oxime by a Schmidt reaction, and then carrying out a Beckmann rearrangement. Various variations have been reported for both reactions. The Schmidt reaction can be carried out by, for example, reacting a ketone in sodium azide and a strong acid, such as concentrated hydrochloric acid, sulfuric acid, trifluoroacetic acid or methane sulfonic acid, and in the absence of a solvent or in a solvent such as acetonitrile, chloroform or methylene chloride. In the Beckmann rearrangement, an oxime of a carbonyl compound is reacted with polyphosphoric acid or a trimethylsilyl ester thereof, or reacting at a high temperature with a Lewis acid such as aluminum triiodide or iron (III) chloride-impregnated montmoril-lonite or with thionyl chloride in the absence of solvent or in the presence of a solvent such as acetonitrile. In addition, it can also be prepared by forming a mesylate or tosylate of an oxime followed by treating with a base such as aqueous sodium hydroxide solution or treating with a Lewis acid such as diethyl aluminum chloride, as described in Heterocycles (1994), 38(2), 305-18; US 2011/0136782 A1.

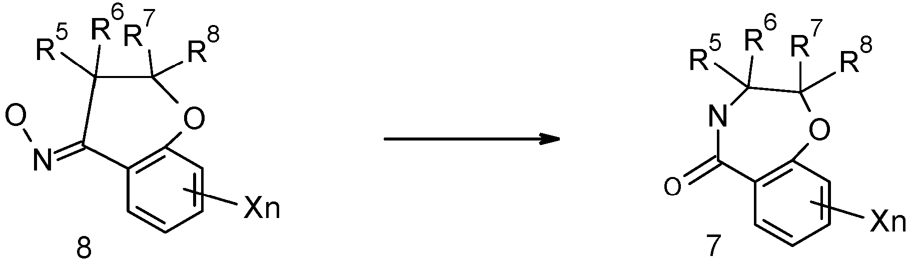

Alternatively, the compound represented by formula (7) can be prepared in an one pot by copper(II)-catalyzed Beckmann rearrangement of ketones (9) under mild reaction conditions using hydroxylamine-O-sulfonic acid as aminating agent, as described in Synthesis 2019, 51(19), 3709-3714.

The oxime (8) can be prepared using a known method by reacting with hydroxylamine hydrochloride in a solvent such as ethanol followed by adding a base such as pyridine or sodium acetate or aqueous sodium hydroxide solution as necessary at a temperature up to the boiling point of the solvent, as described in Bioorganic & Medicinal Chemistry (2008), 16(11), 6124-6130; Heterocyclic Communications (1998), 4(6), 547-557.

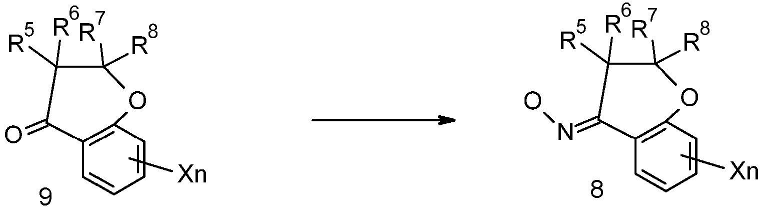

The cyclic acetophenone derivatives represented by formula (9) are commercially available or can be accessed starting from 2-hydroxyacetophenone via a classical ring closure reaction using the corresponding ketone in the presence of pyrrolidine, as described in Bioorganic & Medicinal Chemistry (2008), 16(11), 6124-6130; Journal of the Chemical Society, Perkin Transactions 1: Organic and Bio-Organic Chemistry (1995).

The pending invention relates further to a process for preparing compounds of formula I, comprising a reaction of the compounf of the formula Y: 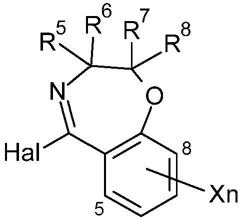 wherein R⁵, R⁶, R⁷, R⁸ and Xn are as defined above..

The pending invention relates further to compounds of the formula Y 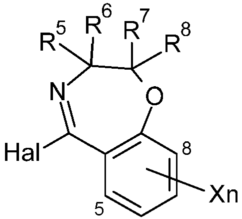 wherein
R⁵ is selected from the group consisting of H, C₁-C₆-alkyl,
R⁶ is selected from the group consisting of H, C₁-C₆-alkyl, or
R⁵ and R⁶ form together with the C atoms to which they are bound a C₃-C₆-cycloalkyl;
R⁷ is selected from the group consisting of H**,** C₁-C₆-alkyl,
R⁸ is selected from the group consisting of H, C₁-C₆-alkyl, or
R⁷ and R⁸ form together with the C atoms to which they are bound a C₃-C₆-cycloalkyl;
X is halogene, C₁-C₆-alkyl,
n is 0, 1 or 2.

According to one embodiment
R⁵ is selected from the group consisting of H, CH₃,
R⁶ is selected from the group consisting of H, CH₃, or
R⁵ and R⁶ form together with the C atoms to which they are bound a cyclopropyl;
R⁷ is selected from the group consisting of **H,** CH₃,
R³ is selected from the group consisting of H, CH₃, or
R⁷ and R⁸ form together with the C atoms to which they are bound a cyclopropyl;
X is Cl, F or CH₃,
n is 0, 1 or 2.

According to one further embodiment following compounds Y are most preferred:

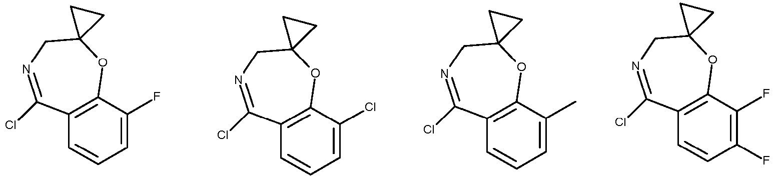

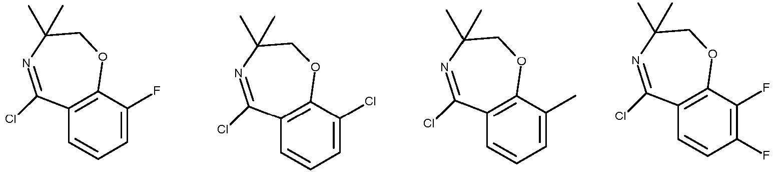

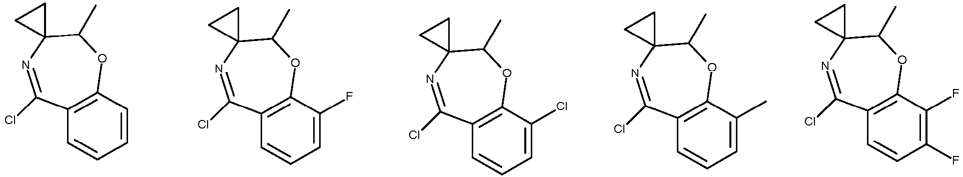

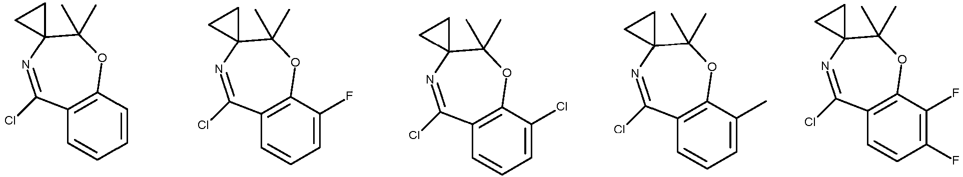

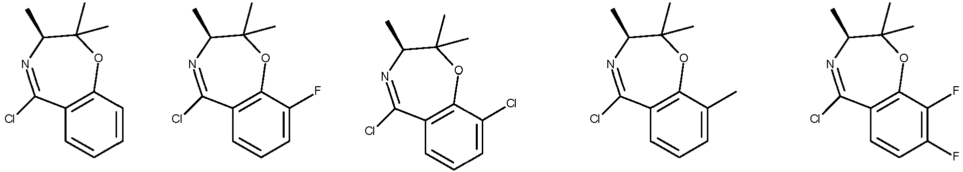

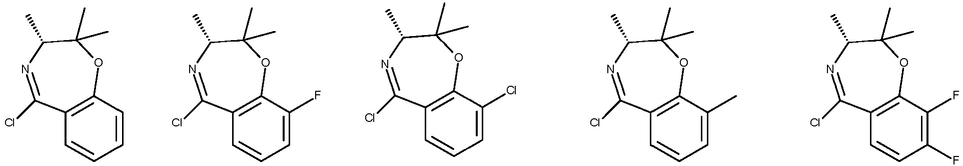

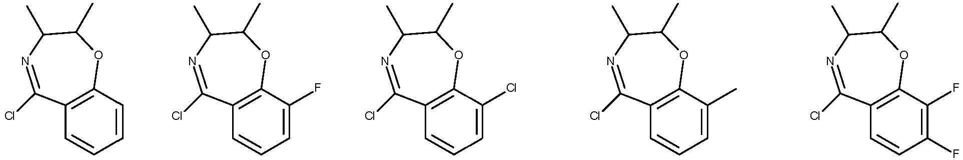

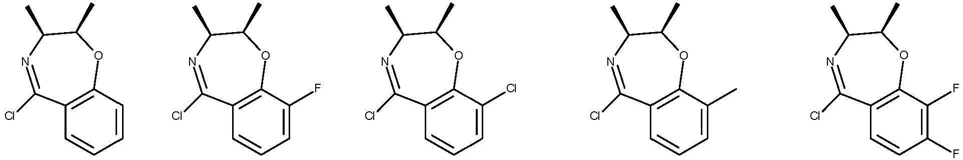

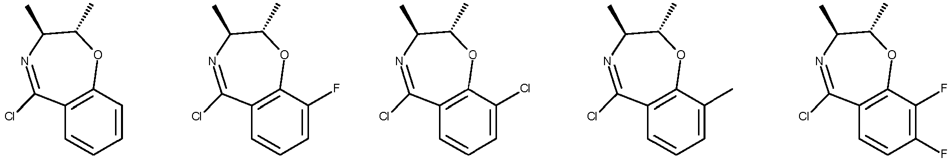

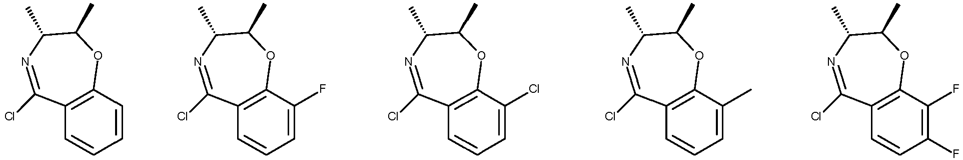

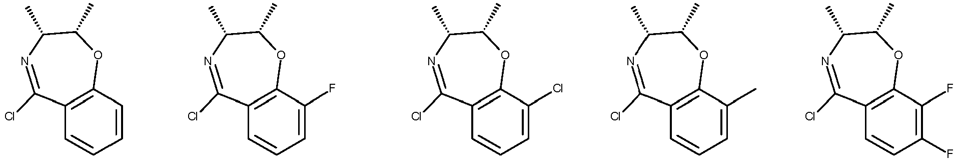

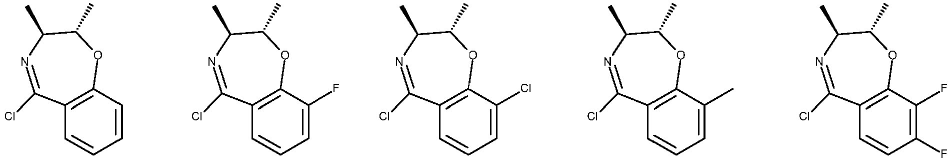

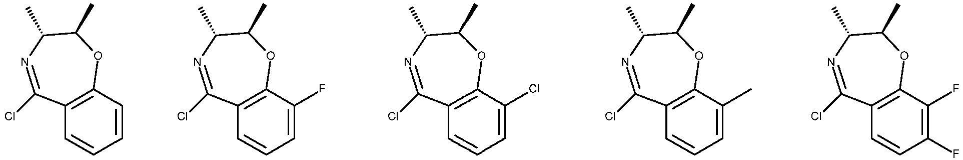

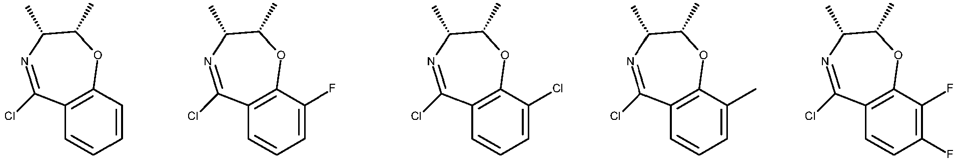

The compounds I and the compositions thereof, respectively, are suitable as fungicides effective against a broad spectrum of phytopathogenic fungi, including soil-borne fungi, in particular from the classes of Plasmodiophoromycetes, Peronosporomycetes (syn. Oomycetes), Chytridiomycetes, Zygomycetes, Ascomycetes, Basidiomycetes, and Deuteromycetes (syn. Fungi imperfecti). They can be used in crop protection as foliar fungicides, fungicides for seed dressing, and soil fungicides.

The compounds I and the compositions thereof are preferably useful in the control of phytopathogenic fungi on various cultivated plants, such as cereals, e. g. wheat, rye, barley, triticale, oats, or rice; beet, e. g. sugar beet or fodder beet; fruits, e. g. pomes (apples, pears, etc.), stone fruits (e.g. plums, peaches, almonds, cherries), or soft fruits, also called berries (strawberries, raspberries, blackberries, gooseberries, etc.); leguminous plants, e. g. lentils, peas, alfalfa, or soybeans; oil plants, e. g. oilseed rape, mustard, olives, sunflowers, coconut, cocoa beans, castor oil plants, oil palms, ground nuts, or soybeans; cucurbits, e. g. squashes, cucumber, or melons; fiber plants, e. g. cotton, flax, hemp, or jute; citrus fruits, e. g. oranges, lemons, grapefruits, or mandarins; vegetables, e. g. spinach, lettuce, asparagus, cabbages, carrots, onions, tomatoes, potatoes, cucurbits, or paprika; lauraceous plants, e. g. avocados, cinnamon, or camphor; energy and raw material plants, e. g. corn, soybean, oilseed rape, sugar cane, or oil palm; corn; tobacco; nuts; coffee; tea; bananas; vines (table grapes and grape juice grape vines); hop; turf; sweet leaf (also called Stevia); natural rubber plants; or ornamental and forestry plants, e. g. flowers, shrubs, broad-leaved trees, or evergreens (conifers, eucalypts, etc.); on the plant propagation material, such as seeds; and on the crop material of these plants.

More preferably, compounds I and compositions thereof, respectively are used for controlling fungi on field crops, such as potatoes, sugar beets, tobacco, wheat, rye, barley, oats, rice, corn, cotton, soybeans, oilseed rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant, such as seeds; and vegetative plant materials, such as cuttings and tubers (e. g. potatoes), which can be used for the multiplication of the plant. This includes seeds, roots, fruits, tubers, bulbs, rhizomes, shoots, sprouts and other parts of plants; including seedlings and young plants to be transplanted after germination or after emergence from soil.

Preferably, treatment of plant propagation materials with compounds I and compositions thereof, respectively, is used for controlling fungi on cereals, such as wheat, rye, barley and oats; rice, corn, cotton and soybeans.

According to the invention all of the above cultivated plants are understood to comprise all species, subspecies, variants, varieties and/or hybrids which belong to the respective cultivated plants, including but not limited to winter and spring varieties, in particular in cereals such as wheat and barley, as well as oilseed rape, e.g. winter wheat, spring wheat, winter barley etc.

Corn is also known as Indian corn or maize (*Zea mays*) which comprises all kinds of corn such as field corn and sweet corn. According to the invention all maize or corn subspecies and/or varieties are comprised, in particular flour corn (*Zea mays var. amylacea*)*,* popcorn (*Zea mays var. everta*), dent corn (*Zea mays var. indentata*)*,* flint corn (*Zea mays var. indurata*)*,* sweet corn (*Zea mays var. saccharata* and var. *rugosa*)*,* waxy corn (*Zea mays var. ceratina*)*,* amylomaize (high amylose *Zea mays* varieties), pod corn or wild maize (*Zea mays var. tunicata*) and striped maize (*Zea mays var. japonica*)*.*

Most soybean cultivars are classifiable into indeterminate and determinate growth habit, whereas *Glycine soja,* the wild progenitor of soybean, is indeterminate (PNAS 2010, 107 (19) 8563-856). The indeterminate growth habit (Maturity Group, MG 00 to MG 4.9) is characterized by a continuation of vegetative growth after flowering begins whereas determinate soybean varieties (MG 5 to MG 8) characteristically have finished most of their vegetative growth when flowering begins. According to the invention all soybean cultivars or varieties are comprised, in particular indeterminate and determinate cultivars or varieties.

The term "cultivated plants" is to be understood as including plants which have been modified by mutagenesis or genetic engineering to provide a new trait to a plant or to modify an already present trait. Mutagenesis includes random mutagenesis using X-rays or mutagenic chemicals, but also targeted mutagenesis to create mutations at a specific locus of a plant genome. Targeted mutagenesis frequently uses oligonucleotides or proteins like CRISPR/Cas, zinc-finger nucleases, TALENs or meganucleases. Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant to add a trait or improve or modify a trait. These integrated genes are also referred to as transgenes, while plant comprising such transgenes are referred to as transgenic plants. The process of plant transformation usually produces several transformation events, wich differ in the genomic locus in which a transgene has been integrated. Plants comprising a specific transgene on a specific genomic locus are usually described as comprising a specific "event", which is referred to by a specific event name. Traits which have been introduced in plants or have been modified include herbicide tolerance, insect resistance, increased yield and tolerance to abiotic conditions, like drought.

Herbicide tolerance has been created by using mutagenesis and genetic engineering. Plants which have been rendered tolerant to acetolactate synthase (ALS) inhibitor herbicides by mutagenesis and breeding are e.g. available under the name Clearfield^{®}. Herbicide tolerance to glyphosate, glufosinate, 2,4-D, dicamba, oxynil herbicides, like bromoxynil and ioxynil, sulfonylurea herbicides, ALS inhibitors and 4-hydroxyphenylpyruvate dioxygenase (HPPD) inhibitors, like isoxaflutole and mesotrione, has been created via the use of transgenes.

Transgenes to provide herbicide tolerance traits comprise: for tolerance to glyphosate: cp4 epsps, epsps grg23ace5, mepsps, 2mepsps, gat4601, gat4621, goxv247; for tolerance to glufosinate: pat and bar, for tolerance to 2,4-D: aad-1, aad-12; for tolerance to dicamba: dmo; for tolerance to oxynil herbicies: bxn; for tolerance to sulfonylurea herbicides: zm-hra, csr1-2, gm-hra, S4-HrA; for tolerance to ALS inhibitors: csr1-2; and for tolerance to HPPD inhibitors: hppdPF, W336, avhppd-03.

Transgenic corn events comprising herbicide tolerance genes include, but are not limited to, DAS40278, MON801, MON802, MON809, MON810, MON832, MON87411, MON87419, MON87427, MON88017, MON89034, NK603, GA21, MZHG0JG, HCEM485, VCO-Ø1981-5, 676, 678, 680, 33121, 4114, 59122, 98140, Bt10, Bt176, CBH-351, DBT418, DLL25, MS3, MS6, MZIR098, T25, TC1507 and TC6275. Transgenic soybean events comprising herbicide tolerance genes include, but are not limited to, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHTØH2, W62, W98, FG72 and CV127. Transgenic cotton events comprising herbicide tolerance genes include, but are not limited to, 19-51a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40. Transgenic canola events comprising herbicide tolerance genes are for example, but not excluding others, MON88302, HCR-1, HCN10, HCN28, HCN92, MS1, MS8, PHY14, PHY23, PHY35, PHY36, RF1, RF2 and RF3.

Transgenes to provide insect resistance preferably are toxin genes of *Bacillus* spp. and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. In addition, transgenes of plant origin, such as genes coding for protease inhibitors, like CpTI and pinll, can be used. A further approach uses transgenes such as dvsnf7 to produce double-stranded RNA in plants.

Transgenic corn events comprising genes for insecticidal proteins or double stranded RNA include, but are not limited to, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098. Transgenic soybean events comprising genes for insecticidal proteins include, but are not limited to, MON87701, MON87751 and DAS-81419. Transgenic cotton events comprising genes for insecticidal proteins include, but are not limited to, SGK321, MON531, MON757, MON1076, MON15985, 31707, 3253, 3257, 3258, 42317, BNLA-601, Event1, COT67B, COT102, T303-3, T304-40, GFM Cry1A, GK12, MLS 9124, 281-24-236, 3006-210-23, GHB119 and SGK321.

Cultivated plants with increased yield have been created by using the transgene athb17 (e.g. corn event MON87403), or bbx32 (e.g. soybean event MON87712).

Cultivated plants comprising a modified oil content have been created by using the transgenes: gm-fad2-1, Pj.D6D, Nc.Fad3, fad2-1A and fatb1-A (e.g. soybean events 260-05, MON87705 and MON87769).

Tolerance to abiotic conditions, such as drought, has been created by using the transgene cspB (corn event MON87460) and Hahb-4 (soybean event IND-ØØ41Ø-5).

Traits are frequently combined by combining genes in a transformation event or by combining different events during the breeding process resulting in a cultivated plant with stacked traits. Preferred combinations of traits are combinations of herbicide tolerance traits to different groups of herbicides, combinations of insect tolerance to different kind of insects, in particular tolerance to lepidopteran and coleopteran insects, combinations of herbicide tolerance with one or several types of insect resistance, combinations of herbicide tolerance with increased yield as well as combinations of herbicide tolerance and tolerance to abiotic conditions.

Plants comprising singular or stacked traits as well as the genes and events providing these traits are well known in the art. For example, detailed information as to the mutagenized or integrated genes and the respective events are available from websites of the organizations "International Service for the Acquisition of Agri-biotech Applications (ISAAA)" (http://www.isaaa.org/gmapprovaldatabase) and the "Center for Environmental Risk Assessment (CERA)" (http://cera-gmc.org/GMCropDatabase). Further information on specific events and methods to detect them can be found for canola events MS1, MS8, RF3, GT73, MON88302, KK179 in WO01/031042, WO01/041558, WO01/041558, WO02/036831, WO11/153186, WO13/003558; for cotton events MON1445, MON15985, MON531 (MON15985), LLCotton25, MON88913, COT102, 281-24-236, 3006-210-23, COT67B, GHB614, T304-40, GHB119, MON88701, 81910 in WO02/034946, WO02/100163, WO02/100163, WO03/013224, WO04/072235, WO04/039986, WO05/103266, WO05/103266, WO06/128573, WO07/017186, WO08/122406, WO08/151780, WO12/134808, WO13/112527; for corn events GA21, MON810, DLL25, TC1507, MON863, MIR604, LY038, MON88017, 3272, 59122, NK603, MIR162, MON89034, 98140, 32138, MON87460, 5307, 4114, MON87427, DAS40278, MON87411, 33121, MON87403, MON87419 in WO98/044140, US02/102582, US03/126634, WO04/099447, WO04/011601, WO05/103301, WO05/061720, WO05/059103, WO06/098952, WO06/039376, US2007/292854, WO07/142840, WO07/140256, WO08/112019, WO09/103049, WO09/111263, WO10/077816, WO11/084621, WO11/062904, WO11/022469, WO13/169923, WO14/116854, WO15/053998, WO15/142571; for potato events E12, F10, J3, J55, V11, X17, Y9 in WO14/178910, WO14/178913, WO14/178941, WO14/179276, WO16/183445, WO17/062831, WO17/062825; for rice events LLRICE06, LLRICE601, LLRICE62 in WO00/026345, WO00/026356, WO00/026345; and for soybean events H7-1, MON89788, A2704-12, A5547-127, DP305423, DP356043, MON87701, MON87769, CV127, MON87705, DAS68416-4, MON87708, MON87712, SYHT0H2, DAS81419, DAS81419 x DAS44406-6, MON87751 in WO04/074492, WO06/130436, WO06/108674, WO06/108675, WO08/054747, WO08/002872, WO09/064652, WO09/102873, WO10/080829, WO10/037016, WO11/066384, WO11/034704, WO12/051199, WO12/082548, WO13/016527, WO13/016516, WO14/201235.

The use of compounds I and compositions thereof, respectively, on cultivated plants may result in effects which are specific to a cultivated plant comprising a certain transgene or event. These effects might involve changes in growth behavior or changed resistance to biotic or abiotic stress factors. Such effects may in particular comprise enhanced yield, enhanced resistance or tolerance to insects, nematodes, fungal, bacterial, mycoplasma, viral or viroid pathogens as well as early vigour, early or delayed ripening, cold or heat tolerance as well as changed amino acid or fatty acid spectrum or content.

The compounds I and compositions thereof, respectively, are particularly suitable for controlling the following causal agents of plant diseases:
*Albugo* spp. (white rust) on ornamentals, vegetables (e. g. *A. candida*) and sunflowers (e. g. *A. tragopogonis*); *Alternaria* spp. (Alternaria leaf spot) on vegetables (e.g. *A. dauci* or *A. porn*)*,* oilseed rape (*A. brassicicola* or *brassicae*)*,* sugar beets (*A. tenuis*)*,* fruits (e.g. *A. grandis*)*,* rice, soybeans, potatoes and tomatoes (e. g. *A. solani, A. grandis* or *A. alternata*)*,* tomatoes (e. g. *A. solani* or *A. alternata*) and wheat (e.g. A. *triticina*); *Aphanomyces* spp. on sugar beets and vegetables; *Ascochyta* spp. on cereals and vegetables, e. g. *A. tritici* (anthracnose) on wheat and A. *hordei* on barley; *Aureobasidium zeae* (*syn. Kapatiella* zeae) on corn; *Bipolaris* and *Drechslera* spp. (teleomorph: *Cochliobolus* spp.), e. g. Southern leaf blight (*D. maydis*) or Northern leaf blight (*B. zeicola*) on corn, e. g. spot blotch (*B. sorokiniana*) on cereals and e. g. *B*. *oryzae* on rice and turfs; *Blumeria* (formerly *Erysiphe*) *graminis* (powdery mildew) on cereals (e. g. on wheat or barley); *Botrytis cinerea* (teleomorph: *Botryotinia fuckeliana:* grey mold) on fruits and berries (e. g. strawberries), vegetables (e. g. lettuce, carrots, celery and cabbages); *B. squamosa* or *B*. *allii* on onion family), oilseed rape, ornamentals (e.g. *B eliptica*)*,* vines, forestry plants and wheat; *Bremia lactucae* (downy mildew) on lettuce; *Ceratocystis* (*syn. Ophiostoma*) spp. (rot or wilt) on broad-leaved trees and evergreens, e. g. *C*. *ulmi* (Dutch elm disease) on elms; *Cercospora* spp. (Cercospora leaf spots) on corn (e. g. Gray leaf spot: *C*. *zeae-maydis*)*,* rice, sugar beets (e. g. *C*. *beticola*)*,* sugar cane, vegetables, coffee, soybeans (e. g. *C*. *sojina* or *C. kikuchii*) and rice; *Cladobotryum* (*syn. Dactylium*) spp. (e.g. *C*. *mycophilum*
(formerly *Dactylium dendroides,* teleomorph: *Nectria albertinii, Nectria rosella syn. Hypomyces rosellus*) on mushrooms; *Cladosporium* spp. on tomatoes (e. g. *C*. *fulvum:* leaf mold) and cereals, e. g. *C*. *herbarum* (black ear) on wheat; *Claviceps purpurea* (ergot) on cereals; *Cochliobolus* (anamorph: *Helminthosporium* of *Bipolaris*) spp. (leaf spots) on corn (*C*. *carbonum*)*,* cereals (e. g. *C*. *sativus,* anamorph: *B. sorokiniana*) and rice (e. g. *C*. *miyabeanus,* anamorph: *H. oryzae*); *Colletotrichum* (teleomorph: *Glomerella*) spp. (anthracnose) on cotton (e. g. *C*. *gossypii*)*,* corn (e. g. *C*. *graminicola:* Anthracnose stalk rot), soft fruits, potatoes (e. g. *C*. *coccodes:* black dot), beans (e. g. *C*. *lindemuthianum*)*,* soybeans (e. g. *C*. *truncatum* or *C*. *gloeosporioides*)*,* vegetables (e.g. *C*. *lagenarium* or *C*. *capsici*)*,* fruits (e.g. *C*. *acutatum),* coffee (e.g. *C*. *coffeanum* or *C*. *kahawae)* and *C*. *gloeosporioides* on various crops; *Corticium* spp., e. g. *C*. *sasakii* (sheath blight) on rice; *Corynespora cassiicola* (leaf spots) on soybeans, cotton and ornamentals; *Cycloconium* spp., e. g. *C*. *oleaginum* on olive trees; *Cylindrocarpon* spp. (e. g. fruit tree canker or young vine decline, teleomorph: *Nectria* or *Neonectria* spp.) on fruit trees, vines (e. g. *C*. *liriodendri,* teleomorph: *Neonectria liriodendri:* Black Foot Disease) and ornamentals; *Dematophora* (teleomorph: *Rosellinia*) *necatrix* (root and stem rot) on soybeans; *Diaporthe* spp., e. g. *D*. *phaseolorum* (damping off) on soybeans; *Drechslera* (*syn. Helminthosporium,* teleomorph: *Pyrenophora*) spp. on corn, cereals, such as barley (e. g. *D. teres,* net blotch) and wheat (e. g. *D*. *tritici-repentis:* tan spot), rice and turf; Esca (dieback, apoplexy) on vines, caused by *Formitiporia* (*syn. Phellinus*) *punctata, F. mediterranea, Phaeomoniella chlamydospora* (formerly *Phaeoacremonium chlamydosporum*)*, Phaeoacremonium aleophilum* and/or *Botryosphaeria obtusa; Elsinoe* spp. on pome fruits (*E. pyri*)*,* soft fruits (*E. veneta:* anthracnose) and vines (*E. ampelina:* anthracnose); *Entyloma oryzae* (leaf smut) on rice; *Epicoccum* spp. (black mold) on wheat; *Erysiphe* spp. (powdery mildew) on sugar beets (*E. betae*)*,* vegetables (e. g. *E. pisi*)*,* such as cucurbits (e. g. *E. cichoracearum*)*,* cabbages, oilseed rape (e. g. *E. cruciferarum*); *Eutypa lata* (Eutypa canker or dieback, anamorph: *Cytosporina lata, syn. Libertella blepharis*) on fruit trees, vines and ornamental woods; *Exserohilum* (syn*. Helminthosporium*) spp. on corn (e. g. *E. turcicum*); *Fusarium* (teleomorph: *Gibberella*) spp. (wilt, root or stem rot) on various plants, such as *F. graminearum* or *F. culmorum* (root rot, scab or head blight) on cereals (e. g. wheat or barley), *F. oxysporum* on tomatoes, *F. solani* (f. sp. *glycines* now syn. *F. virguliforme* ) and *F. tucumaniae* and *F. brasiliense* each causing sudden death syndrome on soybeans, and *F. verticillioides* on corn; *Gaeumannomyces graminis* (take-all) on cereals (e. g. wheat or barley) and corn; *Gibberella* spp. on cereals (e. g. *G. zeae*) and rice (e. g. *G. fujikuroi:* Bakanae disease); *Glomerella cingulata* on vines, pome fruits and other plants and *G. gossypii* on cotton; Grain-staining complex on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera,* teleomorph: *Cochliobolus*) on corn, cereals, potatoes and rice; *Hemileia* spp., e. g. *H. vastatrix* (coffee leaf rust) on coffee; *Isariopsis clavispora* (syn*. Cladosporium vitis*) on vines; *Macrophomina phaseolina* (syn*. phaseoli*) (root and stem rot) on soybeans and cotton; *Microdochium* (syn*. Fusarium*) *nivale* (pink snow mold) on cereals (e. g. wheat or barley); *Microsphaera diffusa* (powdery mildew) on soybeans; *Monilinia* spp., e. g. *M. laxa, M. fructicola* and *M. fructigena* (syn*. Monilia* spp.: bloom and twig blight, brown rot) on stone fruits and other rosaceous plants; *Mycosphaerella* spp. on cereals, bananas, soft fruits and ground nuts, such as e. g. *M. graminicola* (anamorph: *Zymoseptoria tritici* formerly *Septoria tritici:* Septoria blotch) on wheat or *M. fijiensis* (*syn. Pseudocercospora fijiensis:* black Sigatoka disease) and *M. musicola* on bananas, *M. arachidicola* (*syn. M. arachidis* or *Cercospora arachidis*)*, M. berkeleyi* on peanuts, *M*. *pisi* on peas and *M. brassiciola* on brassicas; *Peronospora* spp. (downy mildew) on cabbage (e. g. *P. brassicae*)*,* oilseed rape (e. g. *P. parasitica*)*,* onions (e. g. *P. destructor*)*,* tobacco (P. *tabacina*) and soybeans (e. g. *P. manshurica*); *Phakopsora pachyrhizi* and *P*. *meibomiae* (*soy*bean rust) on soybeans; *Phialophora* spp. e. g. on vines (e. g. *P*. *tracheiphila* and *P*. *tetraspora*) and soybeans (e. g. *P. gregata:* stem rot); *Phoma lingam* (syn*. Leptosphaeria biglobosa* and L. *maculans:* root and stem rot) on oilseed rape and cabbage, *P. betae* (root rot, leaf spot and damping-off) on sugar beets and *P*. *zeae-maydis* (syn*. Phyllostica zeae*) on corn; *Phomopsis* spp. on sunflowers, vines (e. g. *P*. *viticola:* can and leaf spot) and soybeans (e. g. stem rot: *P*. *phaseoli,* teleomorph: *Diaporthe phaseolorum*)*; Physoderma maydis* (brown spots) on corn; *Phytophthora* spp. (wilt, root, leaf, fruit and stem root) on various plants, such as paprika and cucurbits (e. g. *P. capsici*)*,* soybeans (e. g. *P. megasperma, syn. P. sojae*)*,* potatoes and tomatoes (e. g. *P. infestans:* late blight) and broad-leaved trees (e. g. *P*. *ramorum:* sudden oak death); *Plasmodiophora brassicae* (club root) on cabbage, oilseed rape, radish and other plants; *Plasmopara* spp., e. g. *P*. *viticola* (grapevine downy mildew) on vines and *P*. *halstedii* on sunflowers; *Podosphaera* spp. (powdery mildew) on rosaceous plants, hop, pome and soft fruits (e. g. *P. leucotricha* on apples) and curcurbits (*P. xanthii*); *Polymyxa* spp., e. g. on cereals, such as barley and wheat (*P*. *graminis)* and sugar beets (*P*. *betae*) and thereby transmitted viral diseases; *Pseudocercosporella herpotrichoides* (syn*. Oculimacula yallundae, O. acuformis: eye-*spot, teleomorph: *Tapesia yallundae*) on cereals, e. g. wheat or barley; *Pseudoperonospora* (downy mildew) on various plants, e. g. *P. cubensis* on cucurbits or *P*. *humili* on hop; *Pseudopezicula tracheiphila* (red fire disease or ,rotbrenner', anamorph: *Phialophora*) on vines; *Puccinia* spp. (rusts) on various plants, e. g. *P. triticina* (brown or leaf rust), *P. striiformis* (stripe or yellow rust), *P. hordei* (dwarf rust), *P. graminis* (stem or black rust) or *P*. *recondita* (brown or leaf rust) on cereals, such as e. g. wheat, barley or rye, *P. kuehnii* (orange rust) on sugar cane and *P. asparagi* on asparagus; *Pyrenopeziza spp.,* e.g. *P. brassicae* on oilseed rape; *Pyrenophora* (anamorph: *Drechslera*) *tritici-repentis* (tan spot) on wheat or *P*. *teres* (net blotch) on barley; *Pyricularia* spp., e. g. *P*. *oryzae* (teleomorph: *Magnaporthe grisea:* rice blast) on rice and *P*. *grisea* on turf and cereals; *Pythium* spp. (damping-off) on turf, rice, corn, wheat, cotton, oilseed rape, sunflowers, soybeans, sugar beets, vegetables and various other plants (e. g. *P*. *ultimum* or *P*. *aphanidermatum*) and *P*. *oligandrum* on mushrooms; *Ramularia* spp., e. g. *R. collo-cygni* (Ramularia leaf spots, Physiological leaf spots) on barley, *R*. *areola* (teleomorph: *Mycosphaerella areola*) on cotton and *R*. *beticola* on sugar beets; *Rhizoctonia* spp. on cotton, rice, potatoes, turf, corn, oilseed rape, potatoes, sugar beets, vegetables and various other plants, e. g. *R. solani* (root and stem rot) on soybeans, *R. solani* (sheath blight) on rice or *R*. *cerealis* (Rhizoctonia spring blight) on wheat or barley; *Rhizopus stolonifer* (black mold, soft rot) on strawberries, carrots, cabbage, vines and tomatoes; *Rhynchosporium secalis* and *R*. *commune* (scald) on barley, rye and triticale; *Sarocladium oryzae* and *S. attenuatum* (sheath rot) on rice; *Sclerotinia* spp. (stem rot or white mold) on vegetables (*S. minor* and *S. sclerotiorum)* and field crops, such as oilseed rape, sunflowers (e. g. *S. sclerotiorum)* and soybeans, *S. rolfsii* (*syn. Athelia rolfsii*) on soybeans, peanut, vegetables, corn, cereals and ornamentals; *Septoria* spp. on various plants, e. g. *S. glycines* (brown spot) on soybeans, *S. tritici* (syn*. Zymoseptoria tritici,* Septoria blotch) on wheat and *S*. (syn. *Stagonospora*) *nodorum* (Stagonospora blotch) on cereals; *Uncinula* (syn*. Erysiphe*) *necator* (powdery mildew, anamorph: *Oidium tuckeri*) on vines; *Setosphaeria* spp. (leaf blight) on corn (e. g. *S. turcicum, syn. Helminthosporium turcicum)* and turf; *Sphacelotheca* spp. (smut) on corn, (e. g. *S. reiliana, syn. Ustilago reiliana:* head smut), sorghum und sugar cane; *Sphaerotheca fuliginea* (syn*. Podosphaera xanthii:* powdery mildew) on cucurbits; *Spongospora subterranea* (powdery scab) on potatoes and thereby transmitted viral diseases; *Stagonospora* spp. on cereals, e. g. *S. nodorum* (Stagonospora blotch, teleomorph: *Leptosphaeria* [syn*. Phaeosphaeria*] *nodorum, syn. Septoria nodorum*) on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis* spp. (black root rot) on tobacco, pome fruits, vegetables, soybeans and cotton, e. g. *T. basicola* (syn*. Chalara elegans*); *Tilletia* spp. (common bunt or stinking smut) on cereals, such as e. g. *T. tritici* (syn. *T. caries,* wheat bunt) and *T. controversa* (dwarf bunt) on wheat; *Trichoderma harzianum on mushrooms; Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus,* syn. *U. phaseoli*)*,* sugar beets (e. g. *U. betae* or *U. beticola*) and on pulses (e.g. *U*. *vignae, U. pisi, U. viciae-fabae* and *U. fabae*); *Ustilago* spp. (loose smut) on cereals (e. g. *U. nuda* and *U. avaenae*)*,* corn (e. g. *U. maydis:* corn smut) and sugar cane; *Venturia* spp. (scab) on apples (e. g. *V. inaequalis*) and pears; and *Verticillium* spp. (wilt) on various plants, such as fruits and ornamentals, vines, soft fruits, vegetables and field crops, e. g. *V. longisporum* on oilseed rape, *V. dahliae* on strawberries, oilseed rape, potatoes and tomatoes, and *V. fungicola* on mushrooms; *Zymoseptoria tritici* on cereals.

The compounds I and compositions thereof, respectively, are particularly suitable for controlling the following causal agents of plant diseases: rusts on soybean and cereals (e.g. *Phakopsora pachyrhizi* and *P*. *meibomiae* on soy; *Puccinia tritici* and *P*. *striiformis* on wheat); molds on specialty crops, soybean, oil seed rape and sunflowers (e.g. *Botrytis cinerea* on strawberries and vines, *Sclerotinia sclerotiorum, S*. *minor* and *S*. *rolfsii* on oil seed rape, sunflowers and soybean); Fusarium diseases on cereals (e.g. *Fusarium culmorum* and *F. graminearum* on wheat); downy mildews on specialty crops (e.g. *Plasmopara viticola* on vines, *Phytophthora infestans* on potatoes); powdery mildews on specialty crops and cereals (e.g. *Uncinula necator* on vines, *Erysiphe* spp. on various specialty crops, *Blumeria graminis* on cereals); and leaf spots on cereals, soybean and corn (e.g. *Septoria tritici* and *S. nodorum* on cereals, *S. glycines* on soybean, *Cercospora* spp. on corn and soybean).

According to one embodiment compounds I.A-1.1a.B-1 to I.A-1.1a.B-25 are particularly suitable for controlling the causal agents of plant diseases according to the list Z.

According to one embodiment compounds I.A-2.1a.B-1 to I.A-2.1a.B-25 are particularly suitable for controlling the causal agents of plant diseases according to the list Z.

According to one embodiment compounds I.A-3.1a.B-1 to I.A-3.1a.B-25 are particularly suitable for controlling the causal agents of plant diseases according to the list Z.

According to one embodiment compounds I.A-4.1a.B-1 to I.A-4.1a.B-25 are particularly suitable for controlling the causal agents of plant diseases according to the list Z.

According to one embodiment compounds I.A-1.2a.B-1 to I.A-1.2a.B-25 are particularly suitable for controlling the causal agents of plant diseases according to the list Z.

According to one embodiment compounds I.A-2.2a.B-1 to I.A-2.2a.B-25 are particularly suitable for controlling the causal agents of plant diseases according to the list Z.

According to one embodiment compounds I.A-3.2a.B-1 to I.A-3.2a.B-25 are particularly suitable for controlling the causal agents of plant diseases according to the list Z.

According to one embodiment compounds I.A-4.2a.B-1 to I.A-4.2a.B-25 are particularly suitable for controlling the causal agents of plant diseases according to the list Z.

According to one embodiment compounds I.A-1.3a.B-1 to I.A-1.3a.B-25 are particularly suitable for controlling the causal agents of plant diseases according to the list Z.

According to one embodiment compounds I.A-2.3a.B-1 to I.A-2.3a.B-25 are particularly suitable for controlling the causal agents of plant diseases according to the list Z.

According to one embodiment compounds I.A-3.3a.B-1 to I.A-3.3a.B-25 are particularly suitable for controlling the causal agents of plant diseases according to the list Z.

According to one embodiment compounds I.A-4.3a.B-1 to I.A-4.3a.B-25 are particularly suitable for controlling the causal agents of plant diseases according to the list Z.

According to one embodiment compounds I.A-1.4a.B-1 to I.A-1.4a.B-25 are particularly suitable for controlling the causal agents of plant diseases according to the list Z.

According to one embodiment compounds I.A-2.4a.B-1 to I.A-2.4a.B-25 are particularly suitable for controlling the causal agents of plant diseases according to the list Z.

According to one embodiment compounds I.A-3.4a.B-1 to I.A-3.4a.B-25 are particularly suitable for controlling the causal agents of plant diseases according to the list Z.

According to one embodiment compounds I.A-4.4a.B-1 to I.A-4.4a.B-25 are particularly suitable for controlling the causal agents of plant diseases according to the list Z.

According to one embodiment compounds I.A-1.5a.B-1 to I.A-1.5a.B-25 are particularly suitable for controlling the causal agents of plant diseases according to the list Z.

According to one embodiment compounds I.A-2.5a.B-1 to I.A-2.5a.B-25 are particularly suitable for controlling the causal agents of plant diseases according to the list Z.

According to one embodiment compounds I.A-3.5a.B-1 to I.A-3.5a.B-25 are particularly suitable for controlling the causal agents of plant diseases according to the list Z.

According to one embodiment compounds I.A-4.5a.B-1 to I.A-4.5a.B-25 are particularly suitable for controlling the causal agents of plant diseases according to the list Z.

According to one embodiment compounds I.A-1.6a.B-1 to I.A-1.6a.B-25 are particularly suitable for controlling the causal agents of plant diseases according to the list Z.

According to one embodiment compounds I.A-2.6a.B-1 to I.A-2.6a.B-25 are particularly suitable for controlling the causal agents of plant diseases according to the list Z.

According to one embodiment compounds I.A-3.6a.B-1 to I.A-3.6a.B-25 are particularly suitable for controlling the causal agents of plant diseases according to the list Z.

According to one embodiment compounds I.A-4.6a.B-1 to I.A-4.6a.B-25 are particularly suitable for controlling the causal agents of plant diseases according to the list Z.

According to one embodiment compounds I.A-1.7a.B-1 to I.A-1.7a.B-25 are particularly suitable for controlling the causal agents of plant diseases according to the list Z.

According to one embodiment compounds I.A-2.7a.B-1 to I.A-2.7a.B-25 are particularly suitable for controlling the causal agents of plant diseases according to the list Z.

According to one embodiment compounds I.A-3.7a.B-1 to I.A-3.7a.B-25 are particularly suitable for controlling the causal agents of plant diseases according to the list Z.

According to one embodiment compounds I.A-4.7a.B-1 to I.A-4.7a.B-25 are particularly suitable for controlling the causal agents of plant diseases according to the list Z.

According to one embodiment compounds I.B-1.1a.B-1 to I.B-1.1a.B-25 are particularly suitable for controlling the causal agents of plant diseases according to the list Z.

According to one embodiment compounds I.B-2.1a.B-1 to I.B-2.1a.B-25 are particularly suitable for controlling the causal agents of plant diseases according to the list Z.

According to one embodiment compounds I.B-3.1a.B-1 to I.B-3.1a.B-25 are particularly suitable for controlling the causal agents of plant diseases according to the list Z.

According to one embodiment compounds I.B-4.1a.B-1 to I.B-4.1a.B-25 are particularly suitable for controlling the causal agents of plant diseases according to the list Z.

According to one embodiment compounds I.B-1.2a.B-1 to I.B-1.2a.B-25 are particularly suitable for controlling the causal agents of plant diseases according to the list Z.

According to one embodiment compounds I.B-2.2a.B-1 to I.B-2.2a.B-25 are particularly suitable for controlling the causal agents of plant diseases according to the list Z.

According to one embodiment compounds I.B-3.2a.B-1 to I.B-3.2a.B-25 are particularly suitable for controlling the causal agents of plant diseases according to the list Z.

According to one embodiment compounds I.B-4.2a.B-1 to I.B-4.2a.B-25 are particularly suitable for controlling the causal agents of plant diseases according to the list Z.

According to one embodiment compounds I.B-1.3a.B-1 to I.B-1.3a.B-25 are particularly suitable for controlling the causal agents of plant diseases according to the list Z.

According to one embodiment compounds I.B-2.3a.B-1 to I.B-2.3a.B-25 are particularly suitable for controlling the causal agents of plant diseases according to the list Z.

According to one embodiment compounds I.B-3.3a.B-1 to I.B-3.3a.B-25 are particularly suitable for controlling the causal agents of plant diseases according to the list Z.

According to one embodiment compounds I.B-4.3a.B-1 to I.B-4.3a.B-25 are particularly suitable for controlling the causal agents of plant diseases according to the list Z.

According to one embodiment compounds I.B-1.4a.B-1 to I.B-1.4a.B-25 are particularly suitable for controlling the causal agents of plant diseases according to the list Z.

According to one embodiment compounds I.B-2.4a.B-1 to I.B-2.4a.B-25 are particularly suitable for controlling the causal agents of plant diseases according to the list Z.

According to one embodiment compounds I.B-3.4a.B-1 to I.B-3.4a.B-25 are particularly suitable for controlling the causal agents of plant diseases according to the list Z.

According to one embodiment compounds I.B-4.4a.B-1 to I.B-4.4a.B-25 are particularly suitable for controlling the causal agents of plant diseases according to the list Z.

According to one embodiment compounds I.B-1.5a.B-1 to I.B-1.5a.B-25 are particularly suitable for controlling the causal agents of plant diseases according to the list Z.

According to one embodiment compounds I.B-2.5a.B-1 to I.B-2.5a.B-25 are particularly suitable for controlling the causal agents of plant diseases according to the list Z.

According to one embodiment compounds I.B-3.5a.B-1 to I.B-3.5a.B-25 are particularly suitable for controlling the causal agents of plant diseases according to the list Z.

According to one embodiment compounds I.B-4.5a.B-1 to I.B-4.5a.B-25 are particularly suitable for controlling the causal agents of plant diseases according to the list Z.

According to one embodiment compounds I.B-1.6a.B-1 to I.B-1.6a.B-25 are particularly suitable for controlling the causal agents of plant diseases according to the list Z.

According to one embodiment compounds I.B-2.6a.B-1 to I.B-2.6a.B-25 are particularly suitable for controlling the causal agents of plant diseases according to the list Z.

According to one embodiment compounds I.B-3.6a.B-1 to I.B-3.6a.B-25 are particularly suitable for controlling the causal agents of plant diseases according to the list Z.

According to one embodiment compounds I.B-4.6a.B-1 to I.B-4.6a.B-25 are particularly suitable for controlling the causal agents of plant diseases according to the list Z.

According to one embodiment compounds I.B-1.7a.B-1 to I.B-1.7a.B-25 are particularly suitable for controlling the causal agents of plant diseases according to the list Z.

According to one embodiment compounds I.B-2.7a.B-1 to I.B-2.7a.B-25 are particularly suitable for controlling the causal agents of plant diseases according to the list Z.

According to one embodiment compounds I.B-3.7a.B-1 to I.B-3.7a.B-25 are particularly suitable for controlling the causal agents of plant diseases according to the list Z.

According to one embodiment compounds I.B-4.7a.B-1 to I.B-4.7a.B-25 are particularly suitable for controlling the causal agents of plant diseases according to the list Z.

According to one embodiment compounds Ex-1 to Ex-101 are particularly suitable for controlling the causal agents of plant diseases according to the list Z.

List Z:
*Albugo* spp. (white rust) on ornamentals, vegetables (e. g. *A. candida*) and sunflowers (e. g. *A. tragopogonis*); *Alternaria* spp. (Alternaria leaf spot) on vegetables (e.g. *A. dauci* or *A. porri*), oilseed rape (*A. brassicicola* or *brassicae*)*,* sugar beets (*A. tenuis*)*,* fruits (e.g. *A. grandis*)*,* rice, soybeans, potatoes and tomatoes (e. g. *A. solani, A. grandis* or *A. alternata*), tomatoes (e. g. *A. solani* or *A. alternata*) and wheat (e.g. *A*. *triticina*); *Aphanomyces* spp. on sugar beets and vegetables; *Ascochyta* spp. on cereals and vegetables, e. g. *A. tritici* (anthracnose) on wheat and *A*. *hordei* on barley; *Aureobasidium zeae* (*syn. Kapatiella* zeae) on corn; *Bipolaris* and *Drechslera* spp. (teleomorph: *Cochliobolus* spp.), e. g. Southern leaf blight (*D. maydis*) or Northern leaf blight (*B. zeicola*) on corn, e. g. spot blotch (*B. sorokiniana*) on cereals and e. g. *B*. *oryzae* on rice and turfs; *Blumeria* (formerly *Erysiphe*) *graminis* (powdery mildew) on cereals (e. g. on wheat or barley); *Botrytis cinerea* (teleomorph: *Botryotinia fuckeliana:* grey mold) on fruits and berries (e. g. strawberries), vegetables (e. g. lettuce, carrots, celery and cabbages); *B*. *squamosa* or *B*. *allii* on onion family), oilseed rape, ornamentals (e.g. *B eliptica*)*,* vines, forestry plants and wheat; *Bremia lactucae* (downy mildew) on lettuce; *Ceratocystis* (syn*. Ophiostoma*) spp. (rot or wilt) on broad-leaved trees and evergreens, e. g. *C*. *ulmi* (Dutch elm disease) on elms; *Cercospora* spp. (Cercospora leaf spots) on corn (e. g. Gray leaf spot: *C*. *zeae-maydis*)*,* rice, sugar beets (e. g. *C*. *beticola*)*,* sugar cane, vegetables, coffee, soybeans (e. g. *C*. *sojina* or *C. kikuchii*) and rice; *Cladobotryum* (*syn. Dactylium*) spp. (e*.*g. *C*. *mycophilum*
(formerly *Dactylium dendroides,* teleomorph: *Nectria albertinii, Nectria rosella syn. Hypomyces rosellus)* on mushrooms; *Cladosporium* spp. on tomatoes (e. g. *C*. *fulvum:* leaf mold) and cereals, e. g. *C*. *herbarum* (black ear) on wheat; *Claviceps purpurea* (ergot) on cereals; *Cochliobolus* (anamorph: *Helminthosporium* of *Bipolaris*) spp. (leaf spots) on corn (*C*. *carbonum*)*,* cereals (e. g. *C*. *sativus,* anamorph: *B. sorokiniana*) and rice (e. g. *C*. *miyabeanus,* anamorph: *H. oryzae*); *Colletotrichum* (teleomorph: *Glomerella*) spp. (anthracnose) on cotton (e. g. *C*. *gossypii*)*,* corn (e. g. *C*. *graminicola:* Anthracnose stalk rot), soft fruits, potatoes (e. g. *C*. *coccodes:* black dot), beans (e. g. *C*. *lindemuthianum*)*,* soybeans (e. g. *C. truncatum* or *C*. *gloeosporioides*)*,* vegetables (e.g. *C*. *lagenarium* or *C*. *capsici*)*,* fruits (e.g. *C*. *acutatum*)*,* coffee (e.g. *C*. *coffeanum* or *C*. *kahawae*) and *C*. *gloeosporioides* on various crops; *Corticium* spp., e. g. *C*. *sasakii* (sheath blight) on rice; *Corynespora cassiicola* (leaf spots) on soybeans, cotton and ornamentals; *Cycloconium* spp., e. g. *C*. *oleaginum* on olive trees; *Cylindrocarpon* spp. (e. g. fruit tree canker or young vine decline, teleomorph: *Nectria* or *Neonectria* spp.) on fruit trees, vines (e. g. *C*. *liriodendri,* teleomorph: *Neonectria liriodendri:* Black Foot Disease) and ornamentals; *Dematophora* (teleomorph: *Rosellinia*) *necatrix* (root and stem rot) on soybeans; *Diaporthe* spp., e. g. *D*. *phaseolorum* (damping off) on soybeans; *Drechslera* (syn*. Helminthosporium,* teleomorph: *Pyrenophora*) spp. on corn, cereals, such as barley (e. g. *D. teres,* net blotch) and wheat (e. g. *D*. *tritici-repentis:* tan spot), rice and turf; Esca (dieback, apoplexy) on vines, caused by *Formitiporia* (syn*. Phellinus) punctata, F. mediterranea, Phaeomoniella chlamydospora* (formerly *Phaeoacremonium chlamydosporum*)*, Phaeoacremonium aleophilum* and/or *Botryosphaeria obtusa; Elsinoe* spp. on pome fruits (*E. pyri*)*,* soft fruits (*E. veneta:* anthracnose) and vines (*E. ampelina:* anthracnose); *Entyloma oryzae* (leaf smut) on rice; *Epicoccum* spp. (black mold) on wheat; *Erysiphe* spp. (powdery mildew) on sugar beets (*E. betae*)*,* vegetables (e. g. *E. pisi*)*,* such as cucurbits (e. g. *E. cichoracearum*)*,* cabbages, oilseed rape (e. g. *E. cruciferarum*)*; Eutypa lata* (Eutypa canker or dieback, anamorph: *Cytosporina lata, syn. Libertella blepharis*) on fruit trees, vines and ornamental woods; *Exserohilum* (syn*. Helminthosporium*) spp. on corn (e. g. *E. turcicum*); *Fusarium* (teleomorph: *Gibberella*) spp. (wilt, root or stem rot) on various plants, such as *F. graminearum* or *F. culmorum* (root rot, scab or head blight) on cereals (e. g. wheat or barley), *F. oxysporum* on tomatoes, *F. solani* (f. sp. *glycines* now syn. *F. virguliforme* ) and *F. tucumaniae* and *F. brasiliense* each causing sudden death syndrome on soybeans, and *F. verticillioides* on corn; *Gaeumannomyces graminis* (take-all) on cereals (e. g. wheat or barley) and corn; *Gibberella* spp. on cereals (e. g. *G*. *zeae*) and rice (e. g. *G. fujikuroi:* Bakanae disease); *Glomerella cingulata* on vines, pome fruits and other plants and *G. gossypii* on cotton; Grain-staining complex on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera,* teleomorph: *Cochliobolus*) on corn, cereals, potatoes and rice; *Hemileia* spp., e. g. *H. vastatrix* (coffee leaf rust) on coffee; *Isariopsis clavispora* (syn*. Cladosporium vitis)* on vines; *Macrophomina phaseolina* (syn*. phaseoli*) (root and stem rot) on soybeans and cotton; *Microdochium* (syn*. Fusarium*) *nivale* (pink snow mold) on cereals (e. g. wheat or barley); *Microsphaera diffusa* (powdery mildew) on soybeans; *Monilinia* spp., e. g. *M. laxa, M. fructicola* and *M. fructigena* (syn*. Monilia* spp.: bloom and twig blight, brown rot) on stone fruits and other rosaceous plants; *Mycosphaerella* spp. on cereals, bananas, soft fruits and ground nuts, such as e. g. *M. graminicola* (anamorph: *Zymoseptoria tritici* formerly *Septoria tritici:* Septoria blotch) on wheat or *M. fijiensis (syn. Pseudocercospora fijiensis:* black Sigatoka disease) and *M. musicola* on bananas, *M. arachidicola* (syn. *M. arachidis* or *Cercospora arachidis*)*, M. berkeleyi* on peanuts, *M*. *pisi* on peas and *M. brassiciola* on brassicas; *Peronospora* spp. (downy mildew) on cabbage (e. g. *P. brassicae*)*,* oilseed rape (e. g. *P. parasitica*)*,* onions (e. g. *P. destructor*)*,* tobacco (P. *tabacina*) and soybeans (e. g. *P. manshurica*); *Phakopsora pachyrhizi* and *P*. *meibomiae* (soybean rust) on soybeans; *Phialophora* spp. e. g. on vines (e. g. *P*. *tracheiphila* and *P*. *tetraspora*) and soybeans (e. g. *P. gregata:* stem rot); *Phoma lingam* (*syn. Leptosphaeria biglobosa* and L. *maculans:* root and stem rot) on oilseed rape and cabbage, *P. betae* (root rot, leaf spot and damping-off) on sugar beets and *P*. *zeae-maydis* (syn*. Phyllostica zeae*) on corn; *Phomopsis* spp. on sunflowers, vines (e. g. *P*. *viticola:* can and leaf spot) and soybeans (e. g. stem rot: *P*. *phaseoli,* teleomorph: *Diaporthe phaseolorum*); *Physoderma maydis* (brown spots) on corn; *Phytophthora* spp. (wilt, root, leaf, fruit and stem root) on various plants, such as paprika and cucurbits (e. g. *P. capsici*)*,* soybeans (e. g. *P. megasperma, syn. P. sojae*)*,* potatoes and tomatoes (e. g. *P. infestans:* late blight) and broad-leaved trees (e. g. *P*. *ramorum:* sudden oak death); *Plasmodiophora brassicae* (club root) on cabbage, oilseed rape, radish and other plants; *Plasmopara* spp., e. g. *P*. *viticola* (grapevine downy mildew) on vines and *P*. *halstedii* on sunflowers; *Podosphaera* spp. (powdery mildew) on rosaceous plants, hop, pome and soft fruits (e. g. *P. leucotricha* on apples) and curcurbits (*P. xanthii*); *Polymyxa* spp., e. g. on cereals, such as barley and wheat (*P*. *graminis*) and sugar beets (*P*. *betae*) and thereby transmitted viral diseases; *Pseudocercosporella herpotrichoides* (*syn. Oculimacula yallundae, O. acuformis: eye-*spot, teleomorph: *Tapesia yallundae*) on cereals, e. g. wheat or barley; *Pseudoperonospora* (downy mildew) on various plants, e. g. *P. cubensis* on cucurbits or *P*. *humili* on hop; *Pseudopezicula tracheiphila* (red fire disease or ,rotbrenner', anamorph: *Phialophora*) on vines; *Puccinia* spp. (rusts) on various plants, e. g. *P. triticina* (brown or leaf rust), *P. striiformis* (stripe or yellow rust), *P. hordei* (dwarf rust), *P. graminis* (stem or black rust) or *P*. *recondita* (brown or leaf rust) on cereals, such as e. g. wheat, barley or rye, *P*. *kuehnii* (orange rust) on sugar cane and *P. asparagi* on asparagus; *Pyrenopeziza spp.,* e.g. *P. brassicae* on oilseed rape; *Pyrenophora* (anamorph: *Drechslera*) *tritici-repentis* (tan spot) on wheat or *P*. *teres* (net blotch) on barley; *Pyricularia* spp., e. g. *P*. *oryzae* (teleomorph: *Magnaporthe grisea:* rice blast) on rice and *P*. *grisea* on turf and cereals; *Pythium* spp. (damping-off) on turf, rice, corn, wheat, cotton, oilseed rape, sunflowers, soybeans, sugar beets, vegetables and various other plants (e. g. *P*. *ultimum* or *P*. *aphanidermatum*) and *P*. *oligandrum* on mushrooms; *Ramularia* spp., e. g. *R. collo-cygni* (Ramularia leaf spots, Physiological leaf spots) on barley, *R*. *areola* (teleomorph: *Mycosphaerella areola*) on cotton and *R*. *beticola* on sugar beets; *Rhizoctonia* spp. on cotton, rice, potatoes, turf, corn, oilseed rape, potatoes, sugar beets, vegetables and various other plants, e. g. *R. solani* (root and stem rot) on soybeans, *R. solani* (sheath blight) on rice or *R*. *cerealis* (Rhizoctonia spring blight) on wheat or barley; *Rhizopus stolonifer* (black mold, soft rot) on strawberries, carrots, cabbage, vines and tomatoes; *Rhynchosporium secalis* and *R*. *commune* (scald) on barley, rye and triticale; *Sarocladium oryzae* and *S. attenuatum* (sheath rot) on rice; *Sclerotinia* spp. (stem rot or white mold) on vegetables (*S. minor* and *S. sclerotiorum*) and field crops, such as oilseed rape, sunflowers (e. g. *S. sclerotiorum)* and soybeans, *S. rolfsii* (syn*. Athelia rolfsii*) on soybeans, peanut, vegetables, corn, cereals and ornamentals; *Septoria* spp. on various plants, e. g. *S. glycines* (brown spot) on soybeans, *S. tritici* (syn*. Zymoseptoria tritici,* Septoria blotch) on wheat and *S*. (syn. *Stagonospora*) *nodorum* (Stagonospora blotch) on cereals; *Uncinula* (*syn. Erysiphe*) *necator* (powdery mildew, anamorph: *Oidium tuckeri*) on vines; *Setosphaeria* spp. (leaf blight) on corn (e. g. *S. turcicum,* syn. *Helminthosporium turcicum*) and turf; *Sphacelotheca* spp. (smut) on corn, (e. g. *S. reiliana, syn. Ustilago reiliana:* head smut), sorghum und sugar cane; *Sphaerotheca fuliginea* (*syn. Podosphaera xanthii:* powdery mildew) on cucurbits; *Spongospora subterranea* (powdery scab) on potatoes and thereby transmitted viral diseases; *Stagonospora* spp. on cereals, e. g. *S. nodorum* (Stagonospora blotch, teleomorph: *Leptosphaeria* [syn*. Phaeosphaeria*] *nodorum, syn. Septoria nodorum*) on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis* spp. (black root rot) on tobacco, pome fruits, vegetables, soybeans and cotton, e. g. *T. basicola* (syn*. Chalara elegans*); *Tilletia* spp. (common bunt or stinking smut) on cereals, such as e. g. *T. tritici* (syn. *T. caries,* wheat bunt) and *T. controversa* (dwarf bunt) on wheat; *Trichoderma harzianum on mushrooms; Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus, syn. U. phaseoli*)*,* sugar beets (e. g. *U. betae* or *U. beticola*) and on pulses (e.g. *U*. *vignae, U. pisi, U. viciae-fabae* and *U. fabae*)*; Ustilago* spp. (loose smut) on cereals (e. g. *U. nuda* and *U. avaenae*)*,* corn (e. g. *U. maydis:* corn smut) and sugar cane; *Venturia* spp. (scab) on apples (e. g. *V. inaequalis*) and pears; and *Verticillium* spp. (wilt) on various plants, such as fruits and ornamentals, vines, soft fruits, vegetables and field crops, e. g. *V. longisporum* on oilseed rape, *V. dahliae* on strawberries, oilseed rape, potatoes and tomatoes, and *V. fungicola* on mushrooms; *Zymoseptoria tritici* on cereals.

The compounds I and compositions thereof, respectively, are also suitable for controlling harmful microorganisms in the protection of stored products or harvest, and in the protection of materials.

The term "stored products or harvest" is understood to denote natural substances of plant or animal origin and their processed forms for which long-term protection is desired. Stored products of plant origin, for example stalks, leafs, tubers, seeds, fruits or grains, can be protected in the freshly harvested state or in processed form, such as pre-dried, moistened, comminuted, ground, pressed or roasted, which process is also known as post-harvest treatment. Also falling under the definition of stored products is timber, whether in the form of crude timber, such as construction timber, electricity pylons and barriers, or in the form of finished articles, such as furniture or objects made from wood. Stored products of animal origin are hides, leather, furs, hairs and alike. Preferably, "stored products" is understood to denote natural substances of plant origin and their processed forms, more preferably fruits and their processed forms, such as pomes, stone fruits, soft fruits and citrus fruits and their processed forms, where application of compounds I and compositions thereof can also prevent disadvantageous effects such as decay, discoloration or mold.

The term "protection of materials" is to be understood to denote the protection of technical and non-living materials, such as adhesives, glues, wood, paper, paperboard, textiles, leather, paint dispersions, plastics, cooling lubricants, fiber, or fabrics against the infestation and destruction by harmful microorganisms, such as fungi and bacteria.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

The compounds I and compositions thereof, respectively, may be used for improving the health of a plant. The invention also relates to a method for improving plant health by treating a plant, its propagation material, and/or the locus where the plant is growing or is to grow with an effective amount of compounds I and compositions thereof, respectively.

The term "plant health" is to be understood to denote a condition of the plant and/or its products which is determined by several indicators alone or in combination with each other, such as yield (e. g. increased biomass and/or increased content of valuable ingredients), plant vigor (e. g. improved plant growth and/or greener leaves ("greening effect")), quality (e. g. improved content or composition of certain ingredients), and tolerance to abiotic and/or biotic stress. The above identified indicators for the health condition of a plant may be interdependent or may result from each other.

The compounds I are employed as such or in form of compositions by treating the fungi, the plants, plant propagation materials, such as seeds; soil, surfaces, materials, or rooms to be protected from fungal attack with a fungicidally effective amount of the active substances. The application can be carried out both before and after the infection of the plants, plant propagation materials, such as seeds; soil, surfaces, materials or rooms by the fungi.

An agrochemical composition comprises a fungicidally effective amount of a compound I. The term "fungicidally effective amount" denotes an amount of the composition or of the compounds I, which is sufficient for controlling harmful fungi on cultivated plants or in the protection of stored products or harvest or of materials and which does not result in a substantial damage to the treated plants, the treated stored products or harvest, or to the treated materials. Such an amount can vary in a broad range and is dependent on various factors, such as the fungal species to be controlled, the treated cultivated plant, stored product, harvest or material, the climatic conditions and the specific compound I used.

Plant propagation materials may be treated with compounds I as such or a composition comprising at least one compound I prophylactically either at or before planting or transplanting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials, such as seeds, e. g. by dusting, coating, or drenching, amounts of active substance of generally from 0.1 to 1000 g, preferably from 1 to 1000 g, more preferably from 1 to 100 g and most preferably from 5 to 100 g, per 100 kg of plant propagation material (preferably seeds) are required.

The user applies the agrochemical composition usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

The compounds I, their N-oxides and salts can be converted into customary types of agrochemical compositions, e. g. solutions, emulsions, suspensions, dusts, powders, pastes, granules, pressings, capsules, and mixtures thereof. Examples for composition types (see also "Catalogue of pesticide formulation types and international coding system", Technical Monograph No. 2, 6th Ed. May 2008, CropLife International) are suspensions (e. g. SC, OD, FS), emulsifiable concentrates (e. g. EC), emulsions (e. g. EW, EO, ES, ME), capsules (e. g. CS, ZC), pastes, pastilles, wettable powders or dusts (e. g. WP, SP, WS, DP, DS), pressings (e. g. BR, TB, DT), granules (e. g. WG, SG, GR, FG, GG, MG), insecticidal articles (e. g. LN), as well as gel formulations for the treatment of plant propagation materials, such as seeds (e. g. GF). The compositions are prepared in a known manner, such as described by Mollet and Grubemann, Formulation technology, Wiley VCH, Weinheim, 2001; or by Knowles, New developments in crop protection product formulation, Agrow Reports DS243, T&F Informa, London, 2005. The invention also relates to agrochemical compositions comprising an auxiliary and at least one compound I.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers, and binders.

Suitable solvents and liquid carriers are water and organic solvents, such as mineral oil fractions of medium to high boiling point, e. g. kerosene, diesel oil; oils of vegetable or animal origin; aliphatic, cyclic and aromatic hydrocarbons, e. g. toluene, paraffin, tetrahydronaphthalene, and alkylated naphthalenes; alcohols, e. g. ethanol, propanol, butanol, benzyl alcohol, cyclohexanol, glycols; DMSO; ketones, e. g. cyclohexanone; esters, e. g. lactates, carbonates, fatty acid esters, gamma-butyrolactone; fatty acids; phosphonates; amines; amides, e. g. *N*-methyl pyrrolidone, fatty acid dimethyl amides; and mixtures thereof.

Suitable solid carriers or fillers are mineral earths, e. g. silicates, silica gels, talc, kaolins, limestone, lime, chalk, clays, dolomite, diatomaceous earth, bentonite, calcium sulfate, magnesium sulfate, magnesium oxide; polysaccharides, e. g. cellulose, starch; fertilizers, e. g. ammonium sulfate, ammonium phosphate, ammonium nitrate, ureas; products of vegetable origin, e. g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylaryl sulfonates, diphenyl sulfonates, alpha-olefin sulfonates, lignin sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and of alkyl naphthalenes, sulfosuccinates, or sulfosuccinamates. Examples of sulfates are sulfates of fatty acids, of oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of *N*-substituted fatty acid amides are fatty acid glucamides or fatty acid alkanolamides. Examples of esters are fatty acid esters, glycerol esters, or monoglycerides. Examples of sugar-based surfactants are sorbitans, ethoxylated sorbitans, sucrose and glucose esters, or alkylpolyglucosides. Examples of polymeric surfactants are home- or copolymers of vinyl pyrrolidone, vinyl alcohols, or vinyl acetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide, and polypropylene oxide. Suitable polyelectrolytes are polyacids or polybases. Examples of polyacids are alkali salts of polyacrylic acid or polyacid comb polymers. Examples of polybases are polyvinyl amines or polyethylene amines.

Suitable adjuvants are compounds, which have a negligible or even no pesticidal activity themselves, and which improve the biological performance of the compound I on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e. g. xanthan gum, carboxymethyl cellulose), inorganic clays (organically modified or unmodified), polycarboxylates, and silicates.

Suitable bactericides are bronopol and isothiazolinone derivatives, such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e. g. in red, blue, or green) are pigments of low water solubility and water-soluble dyes. Examples are inorganic colorants (e. g. iron oxide, titan oxide, iron hexacyanoferrate) and organic colorants (e. g. alizarin-, azo- and phthalocyanine colorants).

Suitable tackifiers or binders are polyvinyl pyrrolidones, polyvinyl acetates, polyvinyl alcohols, polyacrylates, biological or synthetic waxes, and cellulose ethers.

The agrochemical compositions generally comprise between 0.01 and 95 %, preferably between 0.1 and 90 %, more preferably between 1 and 70 %, and in particular between 10 and 60 %, by weight of active substances (e.g. at least one compound I). The agrochemical compositions generally comprise between 5 and 99.9 %, preferably between 10 and 99.9 %, more preferably between 30 and 99 %, and in particular between 40 and 90 %, by weight of at least one auxiliary. The active substances (e.g. compounds I) are employed in a purity of from 90 % to 100 %, preferably from 95-% to 100 % (according to NMR spectrum).

For the purposes of treatment of plant propagation materials, particularly seeds, solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC), and gels (GF) are usually employed. The compositions in question give, after two-to-tenfold dilution, active substance concentrations of from 0.01 to 60 % by weight, preferably from 0.1 to 40 %, in the ready-to-use preparations. Application can be carried out before or during sowing. Methods for applying compound I and compositions thereof, respectively, onto plant propagation material, especially seeds, include dressing, coating, pelleting, dusting, soaking, as well as in-furrow application methods. Preferably, compound I or the compositions thereof, respectively, are applied on to the plant propagation material by a method such that germination is not induced, e. g. by seed dressing, pelleting, coating, and dusting.

Various types of oils, wetters, adjuvants, fertilizers, or micronutrients, and further pesticides (e. g. fungicides, growth regulators, herbicides, insecticides, safeners) may be added to the compounds I or the compositions thereof as premix, or, not until immediately prior to use (tank mix). These agents can be admixed with the compositions according to the invention in a weight ratio of 1:100 to 100:1, preferably 1:10 to 10:1.

A pesticide is generally a chemical or biological agent (such as pestidal active ingredient, compound, composition, virus, bacterium, antimicrobial, or disinfectant) that through its effect deters, incapacitates, kills or otherwise discourages pests. Target pests can include insects, plant pathogens, weeds, mollusks, birds, mammals, fish, nematodes (roundworms), and microbes that destroy property, cause nuisance, spread disease or are vectors for disease. The term "pesticide" includes also plant growth regulators that alter the expected growth, flowering, or reproduction rate of plants; defoliants that cause leaves or other foliage to drop from a plant, usually to facilitate harvest; desiccants that promote drying of living tissues, such as unwanted plant tops; plant activators that activate plant physiology for defense of against certain pests; safeners that reduce unwanted herbicidal action of pesticides on crop plants; and plant growth promoters that affect plant physiology e.g. to increase plant growth, biomass, yield or any other quality parameter of the harvestable goods of a crop plant.

Biopesticides have been defined as a form of pesticides based on microorganisms (bacteria, fungi, viruses, nematodes, etc.) or natural products (compounds, such as metabolites, proteins, or extracts from biological or other natural sources) (U.S. Environmental Protection Agency: http://www.epa.gov/pesticides/biopesticides/). Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classified as microbial pesticides, even though they are multi-cellular.
(2) Biochemical pesticides are naturally occurring substances that control pests or provide other crop protection uses as defined below, but are relatively non-toxic to mammals.

Mixing the compounds I or the compositions comprising them in the use form as fungicides with other fungicides results in many cases in an expansion of the fungicidal spectrum of activity or in a prevention of fungicide resistance development. Furthermore, in many cases, synergistic effects are obtained (synergistic mixtures).

The following list of pesticides II, in conjunction with which the compounds I can be used, is intended to illustrate the possible combinations but does not limit them:
A) Respiration inhibitors
   - Inhibitors of complex III at Qₒ site: azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxystrobin (A.1.17), 2-(2-(3-(2,6-dichlorophenyl)-1-methyl-allylideneaminooxymethyl)-phenyl)-2-methoxyimino-*N*-methyl-acetamide (A.1.18), pyribencarb (A.1.19), triclopyricarb/chlorodincarb (A.1.20), famoxadone (A.1.21), fenamidone (A.1.21), methyl-*N*-[2-[(1,4-dimethyl-5-phenylpyrazol-3-yl)oxylmethyl]phenyl]-*N*-methoxy-carbamate (A.1.22), metyltetraprole (A.1.25), (*Z*,2*E*)-5-[1-(2,4-dichlorophenyl)pyrazol-3-yl]-oxy-2-methoxyimino-*N*,3-dimethyl-pent-3-enamide (A.1.34), (*Z*,2*E*)-5-[1-(4-chlorophenyl)pyrazol-3-yl]oxy-2-methoxyimino-*N*,3-dimethylpent-3-enamide (A.1.35), pyriminostrobin (A.1.36), bifujunzhi (A.1.37), 2-(ortho-((2,5-dimethylphenyl-oxymethylen)phenyl)-3-methoxy-acrylic acid methylester (A.1.38);
   - inhibitors of complex III at Qᵢ site: cyazofamid (A.2.1), amisulbrom (A.2.2), [(6*S*,7*R*,8*R*)-8-benzyl-3-[(3-hydroxy-4-methoxy-pyridine-2-carbonyl)amino]-6-methyl-4,9-dioxo-1,5-dioxonan-7-yl] 2-methylpropanoate (A.2.3), fenpicoxamid (A.2.4), florylpicoxamid (A.2.5), metarylpicoxamid (A.2.6);
   - inhibitors of complex II: benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.15), penthiopyrad (A.3.16), pydiflumetofen (A.3.17), pyraziflumid (A.3.18), sedaxane (A.3.19), tecloftalam (A.3.20), thifluzamide (A.3.21), inpyrfluxam (A.3.22), pyrapropoyne (A.3.23), fluindapyr (A.3.28), N-[2-[2-chloro-4-(trifluoromethyl)phenoxy]phenyl]-3-(difluoromethyl)-5-fluoro-1-methyl-pyrazole-4-carboxamide (A.3.29), methyl (*E*)-2-[2-[(5-cyano-2-methyl-phenoxy)methyl]phenyl]-3-methoxy-prop-2-enoate (A.3.30), isoflucypram (A.3.31), 2-(difluoromethyl)-*N*-(1,1,3-trimethyl-indan-4-yl)pyridine-3-carboxamide (A.3.32), 2-(difluoromethyl)-*N*-[(3*R*)-1,1,3-trimethylindan-4-yl]pyridine-3-carboxamide (A.3.33), 2-(difluoromethyl)-*N*-(3-ethyl-1,1-dimethyl-indan-4-yl)pyridine-3-carboxamide (A.3.34), 2-(difluoromethyl)-*N*-[(3*R*)-3-ethyl-1,1-dimethyl-indan-4-yl]pyridine-3-carboxamide (A.3.35), 2-(difluoromethyl)-*N*-(1,1-dimethyl-3-propyl-indan-4-yl)pyridine-3-carboxamide (A.3.36), 2-(difluoromethyl)-*N*-[(3*R*)-1,1-dimethyl-3-propyl-indan-4-yl]pyridine-3-carboxamide (A.3.37), 2-(difluoromethyl)-*N*-(3-isobutyl-1,1-dimethyl-indan-4-yl)pyridine-3-carboxamide (A.3.38), 2-(difluoromethyl)-*N*-[(3R)-3-isobutyl-1,1-dimethyl-indan-4-yl]pyridine-3-carboxamide (A.3.39) cyclobutrifluram (A.3.24);
   - other respiration inhibitors: diflumetorim (A.4.1); nitrophenyl derivates: binapacryl (A.4.2), dinobuton (A.4.3), dinocap (A.4.4), fluazinam (A.4.5), meptyldinocap (A.4.6), ferimzone (A.4.7); organometal compounds: fentin salts, e. g. fentin-acetate (A.4.8), fentin chloride (A.4.9) or fentin hydroxide (A.4.10); ametoctradin (A.4.11); silthiofam (A.4.12);
B) Sterol biosynthesis inhibitors (SBI fungicides)
   - C14 demethylase inhibitors: triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 2-(2,4-difluorophenyl)-1,1-difluoro-3-(tetrazol-1-yl)-1-[5-[4-(2,2,2-trifluoroethoxy)phenyl]-2-pyridyl]propan-2-ol (B.1.31), 2-(2,4-difluorophenyl)-1,1-difluoro-3-(tetrazol-1-yl)-1-[5-[4-(trifluoromethoxy)phenyl]-2-pyridyl]propan-2-ol (B.1.32), 4-[[6-[2-(2,4-difluorophenyl)-1,1-difluoro-2-hydroxy-3-(5-sulfanyl-1,2,4-triazol-1-yl)propyl]-3-pyridyl]oxy]benzonitrile (B.1.33), ipfentrifluconazole (B.1.37), mefentrifluconazole (B.1.38), (2*R*)-2-[4-(4-chlorophenoxy)-2-(trifluoromethyl)phenyl]-1-(1,2,4-triazol-1-yl)propan-2-ol, (2*S*)-2-[4-(4-chlorophenoxy)-2-(trifluoromethyl)phenyl]-1-(1,2,4-triazol-1-yl)propan-2-ol, 2-(chloromethyl)-2-methyl-5-(*p*-tolylmethyl)-1-(1,2,4-triazol-1-ylmethyl)cyclopentanol (B.1.43); imidazoles: imazalil (B.1.44), pefurazoate (B.1.45), prochloraz (B.1.46), triflumizol (B.1.47); pyrimidines, pyridines, piperazines: fenarimol (B.1.49), pyrifenox (B.1.50), triforine (B.1.51), [3-(4-chloro-2-fluoro-phenyl)-5-(2,4-difluorophenyl)isoxazol-4-yl]-(3-pyridyl)methanol (B.1.52), 4-[[6-[2-(2,4-difluorophenyl)-1,1-difluoro-2-hydroxy-3-(1,2,4-triazol-1-yl)propyl]-3-pyridyl]oxy]benzonitrile (B.1.53), 2-[6-(4-bromophenoxy)-2-(trifluoromethyl)-3-pyridyl]-1-(1,2,4-triazol-1-yl)propan-2-ol (B.1.54), 2-[6-(4-chlorophenoxy)-2-(trifluoromethyl)-3-pyridyl]-1-(1,2,4-triazol-1-yl)propan-2-ol (B.1.55);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8);
   - Inhibitors of 3-keto reductase: fenhexamid (B.3.1);
   - Other Sterol biosynthesis inhibitors: chlorphenomizole (B.4.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - other nucleic acid synthesis inhibitors: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7), 5-fluoro-2-(4-chlorophenylmethoxy)pyrimidin-4 amine (C.2.8);
D) Inhibitors of cell division and cytoskeleton
   - tubulin inhibitors: benomyl (D.1.1), carbendazim (D.1.2), fuberidazole (D1.3), thiabendazole (D.1.4), thiophanate-methyl (D.1.5), pyridachlometyl (D.1.6), N-ethyl-2-[(3-ethynyl-8-methyl-6-quinolyl)oxy]butanamide (D.1.8), *N*-ethyl-2-[(3-ethynyl-8-methyl-6-quinolyl)oxy]-2-methylsulfanyl-acetamide (D.1.9), 2-[(3-ethynyl-8-methyl-6-quinolyl)oxy]-*N*-(2-fluoroethyl)butanamide (D.1.10), 2-[(3-ethynyl-8-methyl-6-quinolyl)oxy]-*N*-(2-fluoroethyl)-2-methoxy-acetamide (D.1.11), 2-[(3-ethynyl-8-methyl-6-quinolyl)oxy]-*N*-propyl-butanamide (D.1.12), 2-[(3-ethynyl-8-methyl-6-quinolyl)oxy]-2-methoxy-*N*-propyl-acetamide (D.1.13), 2-[(3-ethynyl-8-methyl-6-quinolyl)oxy]-2-methylsulfanyl-*N*-propyl-acetamide (D.1.14), 2-[(3-ethynyl-8-methyl-6-quinolyl)oxy]-*N*-(2-fluoroethyl)-2-methylsulfanyl-acetamide (D.1.15), 4-(2-bromo-4-fluorophenyl)-*N*-(2-chloro-6-fluoro-phenyl)-2,5-dimethyl-pyrazol-3-amine (D.1.16);
   - other cell division inhibitors: diethofencarb (D.2.1), ethaboxam (D.2.2), pencycuron (D.2.3), fluopicolide (D.2.4), zoxamide (D.2.5), metrafenone (D.2.6), pyriofenone (D.2.7), phenamacril (D.2.8);
E) Inhibitors of amino acid and protein synthesis
   - methionine synthesis inhibitors: cyprodinil (E.1.1), mepanipyrim (E.1.2), pyrimethanil (E.1.3);
   - protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6);
F) Signal transduction inhibitors
   - MAP / histidine kinase inhibitors: fluoroimid (F.1.1), iprodione (F.1.2), procymidone (F.1.3), vinclozolin (F.1.4), fludioxonil (F.1.5);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7), zinc thiazole (G.2.8);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7);
   - compounds affecting cell membrane permeability and fatty acides: propamocarb (G.4.1);
   - inhibitors of oxysterol binding protein: oxathiapiprolin (G.5.1), fluoxapiprolin (G.5.3), 4-[1-[2-[3-(difluoromethyl)-5-methyl-pyrazol-1-yl]acetyl]-4-piperidyl]-*N*-tetralin-1-yl-pyridine-2-carboxamide (G.5.4), 4-[1-[2-[3,5-bis(difluoromethyl)pyrazol-1-yl]acetyl]-4-piperidyl]-*N*-tetralin-1-yl-pyridine-2-carboxamide (G.5.5), 4-[1-[2-[3-(difluoromethyl)-5-(trifluoromethyl)pyrazol-1-yl]acetyl]-4-piperidyl]-*N*-tetralin-1-yl-pyridine-2-carboxamide (G.5.6), 4-[1-[2-[5-cyclopropyl-3-(difluoromethyl)pyrazol-1-yl]acetyl]-4-piperidyl]- *N*-tetralin-1-yl-pyridine-2-carboxamide (G.5.7), 4-[1-[2-[5-methyl-3-(trifluoromethyl)pyrazol-1-yl]acetyl]-4-piperidyl]-*N*-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-*N*-tetralin-1-yl-pyridine-2-carboxamide (G.5.9), 4-[1-[2-[3,5-bis(trifluoromethyl)pyrazol-1-yl]acetyl]-4-piperidyl]-*N*-tetralin-1-yl-pyridine-2-carboxamide (G.5.10), (4-[1-[2-[5-cyclopropyl-3-(trifluoromethyl)pyrazol-1-yl]acetyl]-4-piperidyl]-*N*-tetralin-1-yl-pyridine-2-carboxamide (G.5.11);
H) Inhibitors with Multi Site Action
   - inorganic active substances: Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds: anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1*H*,5*H*-[1,4]dithiino[2,3-c:5,6-c']dipyrrole-1,3,5,7(2*H*,6*H*)-tetraone (H.4.10);
I) Cell wall synthesis inhibitors
   - inhibitors of glucan synthesis: validamycin (I.1.1), polyoxin B (I.1.2);
   - melanin synthesis inhibitors: pyroquilon (I.2.1), tricyclazole (I.2.2), carpropamid (I.2.3), dicyclomet (I.2.4), fenoxanil (I.2.5);
J) Plant defence inducers
   - acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), calcium phosphonate (J.1.11), potassium phosphonate (J.1.12), potassium or sodium bicarbonate (J.1.9), 4-cyclopropyl-*N*-(2,4-dimethoxyphenyl)thiadiazole-5-carboxamide (J.1.10);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclocymet (K.1.7), diclomezine (K.1.8), difenzoquat (K.1.9), difenzoquat-methylsulfate (K.1.10), diphenylamin (K.1.11), fenitropan (K.1.12), fenpyrazamine (K.1.13), flumetover (K.1.14), flusulfamide (K.1.15), flutianil (K.1.16), harpin (K.1.17), methasulfocarb (K.1.18), nitrapyrin (K.1.19), nitrothal-isopropyl (K.1.20), tolprocarb (K.1.21), oxincopper (K.1.22), proquinazid (K.1.23), tebufloquin (K.1.24), tecloftalam (K.1.25), triazoxide (K.1.26), N'-(4-(4-chloro-3-trifluoromethyl-phenoxy)-2,5-dimethyl-phenyl)-*N*-ethyl-*N*-methyl formamidine (K.1.27), N'-(4-(4-fluoro-3-trifluoromethyl-phenoxy)-2,5-dimethyl-phenyl)-*N*-ethyl-*N-*methyl formamidine (K.1.28), *N*'-[4-[[3-[(4-chlorophenyl)methyl]-1,2,4-thiadiazol-5-yl]-oxy]-2,5-dimethyl-phenyl]-*N*-ethyl-*N*-methyl-formamidine (K.1.29), *N*'-(5-bromo-6-indan-2-yloxy-2-methyl-3-pyridyl)-*N*-ethyl-*N*-methyl-formamidine (K.1.30), *N*'-[5-bromo-6-[1-(3,5-difluorophenyl)ethoxy]-2-methyl-3-pyridyl]-*N*-ethyl-*N*-methyl-formamidine (K.1.31), *N*'-[5-bromo-6-(4-isopropylcyclohexoxy)-2-methyl-3-pyridyl]-*N*-ethyl-*N*-methyl-formamidine (K.1.32), *N*'-[5-bromo-2-methyl-6-(1-phenylethoxy)-3-pyridyl]-*N*-e*t*hyl-*N*-methyl-formamidine (K.1.33), *N'*-(2-methyl-5-trifluoromethyl-4-(3-trimethylsilanyl-propoxy)-phenyl)-*N*-ethyl-*N*-methyl formamidine (K.1.34), *N*'-(5-difluoromethyl-2-methyl-4-(3-trimethylsilanyl-propoxy)-phenyl)-N-ethyl-*N*-methyl formamidine (K.1.35), 2-(4-chloro-phenyl)-*N*-[4-(3,4-dimethoxy-phenyl)-isoxazol-5-yl]-2-prop-2-ynyloxy-acetamide (K.1.36), 3-[5-(4-chloro-phenyl)-2,3-dimethyl-isoxazolidin-3-yl]-pyridine (pyrisoxazole) (K.1.37), 3-[5-(4-methylphenyl)-2,3-dimethyl-isoxazolidin-3-yl]-pyridine (K.1.38), 5-chloro-1-(4,6-dimethoxy-pyrimidin-2-yl)-2-methyl-1*H*-benzoimidazole (K.1.39), ethyl (*Z*)-3-amino-2-cyano-3-phenyl-prop-2-enoate (K.1.40), picarbutrazox (K.1.41), pentyl *N*-[B-[[(2)-[(1-methyltetrazol-5-yl)-phenyl-methylene]amino]oxymethyl]-2-pyridyl]carbamate (K.1.42), but-3-ynyl *N*-[6-[[(Z)-[(1-methyltetrazol-5-yl)-phenyl-methylene]amino]oxymethyl]-2-pyridyl]carbamate (K.1.43), ipflufenoquin (K.1.44), quinofumelin (K.1.47), benziothiazolinone (K.1.48), bromothalonil (K.1.49), 2-(6-benzyl-2-pyridyl)quinazoline (K.1.50), 2-[6-(3-fluoro-4-methoxy-phenyl)-5-methyl-2-pyridyl]quinazoline (K.1.51), dichlobentiazox (K.1.52), *N*'-(2,5-dimethyl-4-phenoxy-phenyl)-*N*-ethyl-*N*-methyl-formamidine (K.1.53), aminopyrifen (K.1.54), fluopimomide (K.1.55), *N*'-[5-bromo-2-methyl-6-(1-methyl-2-propoxy-ethoxy)-3-pyridyl]*-N*-ethyl-*N*-methyl-formamidine (K.1.56), *N*'-[4-(4,5-dichlorothiazol-2-yl)oxy-2,5-dimethyl-phenyl]*-N*-ethyl-*N*-methyl-formamidine (K.1.57), *N*-(2-fluorophenyl)-4-[5-(trifluoromethyl)-1,2,4-oxadiazol-3-yl]benzamide (K.1.58), *N*-methyl-4-[5-(trifluoromethyl)-1,2,4-oxadiazol-3-yl]benzenecarbothioamide (K.1.59), *N*-methoxy-*N*-[[4-[5-(trifluoromethyl)-1,2,4-oxadiazol-3-yl]phenyl]methyl]cyclopropanecarboxamide (WO2018/177894, WO 2020/212513);
L) Biopesticides
   L1) Microbial pesticides with fungicidal, bactericidal, viricidal and/or plant defense activator activity: *Ampelomyces quisqualis, Aspergillus flavus, Aureobasidium pullulans, Bacillus altitudinis, B. amyloliquefaciens, B. amyloliquefaciens* ssp. *plantarum* (also referred to as *B*. *velezensis*)*, B. megaterium, B. mojavensis, B. mycoides, B. pumilus, B. simplex, B. solisalsi, B. subtilis, B. subtilis var. amyloliquefaciens, B. velezensis, Candida oleophila, C*. *saitoana, Clavibacter michiganensis* (bacteriophages), *Coniothyrium minitans, Cryphonectria parasitica, Cryptococcus albidus, Dilophosphora alopecuri, Fusarium oxysporum, Clonostachys rosea* f. *catenulate* (also named *Gliocladium catenulatum*)*, Gliocladium roseum, Lysobacter antibioticus, L. enzymogenes, Metschnikowia fructicola, Microdochium dimerum, Microsphaeropsis ochracea, Muscodor albus, Paenibacillus alvei, Paenibacillus epiphyticus, P. polymyxa, Pantoea vagans, Penicillium bilaiae, Phlebiopsis gigantea, Pseudomonas* sp., *Pseudomonas chloraphis, Pseudozyma flocculosa, Pichia anomala, Pythium oligandrum, Sphaerodes mycoparasitica, Streptomyces griseoviridis, S. lydicus, S. violaceusniger, Talaromyces flavus, Trichoderma asperelloides, T. asperellum, T. atroviride, T. fertile, T. gamsii, T. harmatum, T. harzianum, T. polysporum, T. stromaticum, T. virens, T. viride, Typhula phacorrhiza, Ulocladium oudemansii, Verticillium dahlia,* zucchini yellow mosaic virus (avirulent strain);
   L2) Biochemical pesticides with fungicidal, bactericidal, viricidal and/or plant defense activator activity: harpin protein, *Reynoutria sachalinensis* extract;
   L3) Microbial pesticides with insecticidal, acaricidal, molluscidal and/or nematicidal activity: *Agrobacterium radiobacter, Bacillus cereus, B. firmus, B. thuringiensis, B. thuringiensis* ssp. *aizawai, B. t.* ssp. *israelensis, B. t.* ssp. *galleriae, B. t.* ssp. *kurstaki, B. t.* ssp. *tenebrionis, Beauveria bassiana, B. brongniartii, Burkholderia* spp., *Chromobacterium subtsugae, Cydia pomonella* granulovirus (CpGV), *Cryptophlebia leucotreta* granulovirus (CrleGV), *Flavobacterium* spp., *Helicoverpa armigera* nucleopolyhedrovirus (HearNPV), *Helicoverpa zea* nucleopolyhedrovirus (HzNPV), *Helicoverpa zea* single capsid nucleopolyhedrovirus (HzSNPV), *Heterorhabditis bacteriophora, Isaria fumosorosea, Lecanicillium longisporum, L. muscarium, Metarhizium anisopliae, M. anisopliae* var. *anisopliae, M. anisopliae* var. *acridum, Nomuraea rileyi, Paecilomyces fumosoroseus, P. lilacinus, Paenibacillus popilliae, Pasteuria* spp., *P. nishizawae, P. penetrans,* P. *ramosa, P. thornea, P. usgae, Pseudomonas fluorescens, Spodoptera littoralis* nucleopolyhedrovirus (SpliNPV), *Steinernema carpocapsae, S. feltiae, S. kraussei, Streptomyces galbus, S. microflavus;*
   L4) Biochemical pesticides with insecticidal, acaricidal, molluscidal, pheromone and/or nematicidal activity: L-carvone, citral, (*E,Z*)-7,9-dodecadien-1-yl acetate, ethyl formate, (*E,Z*)-2,4-ethyl decadienoate (pear ester), (*Z,Z,E*)-7, 11, 13-hexadecatrienal, heptyl butyrate, isopropyl myristate, lavanulyl senecioate, cis-jasmone, 2-methyl 1-butanol, methyl eugenol, methyl jasmonate, (*E,Z*)-2,13-octadecadien-1-ol, (*E,Z*)-2,13-octadecadien-1-ol acetate, (*E,Z*)-3,13-octadecadien-1-ol, (*R*)-1-octen-3-ol, pentatermanone, (*E,Z,Z*)-3,8,11-tetradecatrienyl acetate, (*Z,E*)-9,12-tetradecadien-1-yl acetate, (*Z*)-7-tetradecen-2-one, (*Z*)-9-tetradecen-1-yl acetate, (*Z*)-11-tetradecenal, (*Z*)-11-tetradecen-1-ol, extract of *Chenopodium ambrosiodes,* Neem oil, Quillay extract;
   L5) Microbial pesticides with plant stress reducing, plant growth regulator, plant growth promoting and/or yield enhancing activity: *Azospirillum amazonense, A. brasilense, A. lipoferum, A. irakense, A. halopraeferens, Bradyrhizobium* spp., *B. elkanii, B. japonicum, B. liaoningense, B. lupini, Delftia acidovorans, Glomus intraradices, Mesorhizobium* spp., *Rhizobium leguminosarum bv. phaseoli, R. I. bv. trifolii, R. I. bv. viciae, R. tropici, Sinorhizobium meliloti;*
O) Insecticides from classes O.1 to O.29
   O.1 Acetylcholine esterase (AChE) inhibitors: aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb, triazamate; acephate, azamethiphos, azinphos-ethyl, azinphosmethyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos- methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion;
   O.2 GABA-gated chloride channel antagonists: endosulfan, chlordane; ethiprole, fipronil, flufiprole, pyrafluprole, pyriprole;
   O.3 Sodium channel modulators: acrinathrin, allethrin, d-cis-trans allethrin, d-trans allethrin, bifenthrin, kappa-bifenthrin, bioallethrin, bioallethrin S-cylclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, heptafluthrin, imiprothrin, meperfluthrin, metofluthrin, momfluorothrin, epsilon-momfluorothrin, permethrin, phenothrin, prallethrin, profluthrin, pyrethrin (pyrethrum), resmethrin, silafluofen, tefluthrin, kappa-tefluthrin, tetramethylfluthrin, tetramethrin, tralomethrin, transfluthrin; DDT, methoxychlor;
   O.4 Nicotinic acetylcholine receptor (nAChR) agonists: acetamiprid, clothianidin, cycloxaprid, dinotefuran, imidacloprid, nitenpyram, thiacloprid, thiamethoxam; 4,5-dihydro-*N*-nitro-1-(2-oxiranylmethyl)-1*H*-imidazol-2-amine, (2*E*)-1-[(6-chloropyridin-3-yl)methyl]-*N*'-nitro-2-pentylidenehydrazinecarboximidamide; 1-[(6-chloropyridin-3-yl)methyl]-7-methyl-8-nitro-5-propoxy-1,2,3,5,6,7-hexahydroimidazo[1,2-a]pyridine; nicotine; sulfoxaflor, flupyradifurone, triflumezopyrim, (3*R*)-3-(2-chlorothiazol-5-yl)-8-methyl-5-oxo-6-phenyl-2,3-dihydrothiazolo-[3,2-a]pyrimidin-8-ium-7-olate, (3*S*)-3-(6-chloro-3-pyridyl)-8-methyl-5-oxo-6-phenyl-2,3-dihydrothiazolo[3,2-a]pyrimidin-8-ium-7-olate, (3*S*)-8-methyl-5-oxo-6-phenyl-3-pyrimidin-5-yl-2,3-dihydrothiazolo[3,2-a]pyrimidin-8-ium-7-olate, (3*R*)-3-(2-chlorothiazol-5-yl)-8-methyl-5-oxo-6-[3-(trifluoromethyl)phenyl]-2,3-dihydrothiazolo[3,2-a]pyrimidin-8-ium-7-olate; (3*R*)-3-(2-chlorothiazol-5-yl)-6-(3,5-dichlorophenyl)-8-methyl-5-oxo-2,3-dihydrothiazolo[3,2-a]pyrimidin-8ium-7-olate, (3*R*)-3-(2-chlorothiazol-5-yl)-8-ethyl-5-oxo-6-phenyl-2,3-dihydrothiazolo[3,2-a]pyrimidin-8-ium-7-olate;
   O.5 Nicotinic acetylcholine receptor allosteric activators: spinosad, spinetoram;
   O.6 Chloride channel activators: abamectin, emamectin benzoate, ivermectin, lepimectin, milbemectin;
   O.7 Juvenile hormone mimics: hydroprene, kinoprene, methoprene; fenoxycarb, pyriproxyfen;
   O.8 miscellaneous non-specific (multi-site) inhibitors: methyl bromide and other alkyl halides; chloropicrin, sulfuryl fluoride, borax, tartar emetic;
   O.9 Chordotonal organ TRPV channel modulators: pymetrozine, pyrifluquinazon;
   O.10 Mite growth inhibitors: clofentezine, hexythiazox, diflovidazin; etoxazole;
   O.11 Microbial disruptors of insect midgut membranes: *Bacillus thuringiensis, Bacillus sphaericus* and the insecticdal proteins they produce: *Bacillus thuringiensis* subsp. *israelensis, Bacillus sphaericus, Bacillus thuringiensis* subsp. *aizawai, Bacillus thuringiensis* subsp. *kurstaki, Bacillus thuringiensis* subsp. *tenebrionis,* the Bt crop proteins: Cry1Ab, Cry1Ac, Cry1Fa, Cry2Ab, mCry3A, Cry3Ab, Cry3Bb, Cry34/35Ab1;
   0.12 Inhibitors of mitochondrial ATP synthase: diafenthiuron; azocyclotin, cyhexatin, fenbutatin oxide, propargite, tetradifon;
   0.13 Uncouplers of oxidative phosphorylation via disruption of the proton gradient: chlorfenapyr, DNOC, sulfluramid;
   0.14 Nicotinic acetylcholine receptor (nAChR) channel blockers: bensultap, cartap hydrochloride, thiocyclam, thiosultap sodium;
   0.15 Inhibitors of the chitin biosynthesis type 0: bistrifluron, chlorfluazuron, diflubenzuron, flucycloxuron, flufenoxuron, hexaflumuron, lufenuron, novaluron, noviflumuron, teflubenzuron, triflumuron;
   0.16 Inhibitors of the chitin biosynthesis type 1: buprofezin;
   0.17 Moulting disruptors: cyromazine;
   0.18 Ecdyson receptor agonists: methoxyfenozide, tebufenozide, halofenozide, fufenozide, chromafenozide;
   0.19 Octopamin receptor agonists: amitraz;
   O.20 Mitochondrial complex III electron transport inhibitors: hydramethylnon, acequinocyl, fluacrypyrim, bifenazate;
   0.21 Mitochondrial complex I electron transport inhibitors: fenazaquin, fenpyroximate, pyrimidifen, pyridaben, tebufenpyrad, tolfenpyrad; rotenone;
   O.22 Voltage-dependent sodium channel blockers: indoxacarb, metaflumizone, 2-[2-(4-cyanophenyl)-1-[3-(trifluoromethyl)phenyl]ethylidene]-N-[4-(difluoromethoxy)phenyl]-hydrazinecarboxamide, N-(3-chloro-2-methylphenyl)-2-[(4-chlorophenyl)-[4-[methyl(methylsulfonyl)-amino]phenyl]methylene]-hydrazinecarboxamide;
   O.23 Inhibitors of the of acetyl CoA carboxylase: spirodiclofen, spiromesifen, spirotetramat, spiropidion;
   O.24 Mitochondrial complex IV electron transport inhibitors: aluminium phosphide, calcium phosphide, phosphine, zinc phosphide, cyanide;
   O.25 Mitochondrial complex II electron transport inhibitors: cyenopyrafen, cyflumetofen;
   O.26 Ryanodine receptor-modulators: flubendiamide, chlorantraniliprole, cyantraniliprole, cyclaniliprole, tetraniliprole; (*R*)-3-chloro-*N*¹-{2-methyl-4-[1,2,2,2 -tetrafluoro-1-(trifluoromethyl)-ethyl]phenyl}-*N*²-(1-methyl-2-methylsulfonylethyl)phthalamide, (*S*)-3-chloro-*N*¹-{2-methyl-4-[1,2,2,2-tetrafluoro-1-(trifluoromethyl)ethyl]phenyl}-*N*²-(1-methyl-2-methylsulfonylethyl)-phthalamide, methyl-2-[3,5-dibromo-2-({[3-bromo-1-(3-chloropyridin-2-yl)-1*H*-pyrazol-5-yl]-carbonyl}amino)benzoyl]-1,2-dimethylhydrazinecarboxylate; *N*-[4,6-dichloro-2-[(diethyllambda-4-sulfanylidene)carbamoyl]-phenyl]-2-(3-chloro-2-pyridyl)-5-(trifluoromethyl)pyrazole-3-carboxamide; *N*-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide; *N*-[4-chloro-2-[(di-2-propyllambda-4-sulfanylidene)carbamoyl]-6-methyl-phenyl]-2-(3-chloro-2-pyridyl)-5-(trifluoromethyl)pyrazole-3-carboxamide; *N*-[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide; *N*-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide; *N*-[2-(5-amino-1,3,4-thiadiazol-2-yl)-4-chloro-6-methylphenyl]-3-bromo-1-(3-chloro-2-pyridinyl)-1*H*-pyrazole-5-carboxamide; 3-chloro-1-(3-chloro-2-pyridinyl)-*N*-[2,4-dichloro-6-[[(1-cyano-1-methylethyl)amino]carbonyl]phenyl]-1*H*-pyrazole-5-carboxamide; tetrachlorantraniliprole; *N-*[4-chloro-2-[[(1,1-dimethylethyl)amino]carbonyl]-6-methylphenyl]-1-(3-chloro-2-pyridinyl)-3-(fluoromethoxy)-1*H*-pyrazole-5-carboxamide; cyhalodiamide;
   O.27: Chordotonal organ modulators - undefined target site: flonicamid;
   O.28. insecticidal compounds of unknown or uncertain mode of action: afidopyropen, afoxolaner, azadirachtin, amidoflumet, benzoximate, broflanilide, bromopropylate, chinomethionat, cryolite, cyproflanilide, dicloromezotiaz, dicofol, flufenerim, flometoquin, fluensulfone, fluhexafon, fluopyram, fluralaner, metoxadiazone, piperonyl butoxide, pyflubumide, pyridalyl, tioxazafen, 11-(4-chloro-2,6-dimethylphenyl)-12-hydroxy-1,4-dioxa-9-azadispiro[4.2.4.2]-tetradec-11-en-10-one, 3-(4'-fluoro-2,4-dimethylbiphenyl-3-yl)-4-hydroxy-8-oxa-1-azaspiro[4.5]dec-3-en-2-one, 1-[2-fluoro-4-methyl-5-[(2,2,2-trifluoroethyl)sulfinyl]phenyl]-3-(trifluoromethyl)-1*H*-1,2,4-triazole-5-amine, *Bacillus firmus* I-1582; flupyrimin; fluazaindolizine; 4-[5-(3,5-dichlorophenyl)-5-(trifluoromethyl)-4*H*-isoxazol-3-yl]-2-methyl-*N*-(1-oxothietan-3-yl)benzamide; fluxametamide; 5-[3-[2,6-dichloro-4-(3,3-dichloroallyloxy)phenoxy]propoxy]-1*H*-pyrazole; 4-cyano-*N*-[2-cyano-5-[[2,6-dibromo-4-[1,2,2,3,3,3-hexafluoro-1-(trifluoromethyl)propyl]phenyl]carbamoyl]phenyl]-2-methyl-benzamide; 4-cyano-3-[(4-cyano-2-methyl-benzoyl)amino]-*N*-[2,6-dichloro-4-[1,2,2,3,3,3-hexafluoro-1-(trifluoromethyl)propyl]phenyl]-2-fluoro-benzamide; *N*-[5-[[2-chloro-6-cyano-4-[1,2,2,3,3,3-hexafluoro-1-(trifluoromethyl)propyl]phenyl]carbamoyl]-2-cyano-phenyl]-4-cyano-2-methyl-benzamide; *N*-[5-[[2-bromo-6-chloro-4-[2,2,2-trifluoro-1-hydroxy-1-(trifluoromethyl)ethyl]phenyl]carbamoyl]-2-cyano-phenyl]-4-cyano-2-methyl-benzamide; *N*-[5-[[2-bromo-6-chloro-4-[1,2,2,3,3,3-hexafluoro-1-(trifluoromethyl)propyl]phenyl]carbamoyl]-2-cyano-phenyl]-4-cyano-2-methyl-benzamide; 4-cyano-*N*-[2-cyano-5-[[2,6-dichloro-4-[1,2,2,3,3,3-hexafluoro-1-(trifluoromethyl)propyl]phenyl]-carbamoyl]phenyl]-2-methyl-benzamide; 4-cyano-*N*-[2-cyano-5-[[2,6-dichloro-4-[1,2,2,2-tetrafluoro-1-(trifluoromethyl)ethyl]phenyl]carbamoyl]phenyl]-2-methyl-benzamide; *N*-[5-[[2-bromo-6-chloro-4-[1,2,2,2-tetrafluoro-1-(trifluoromethyl)ethyl]phenyl]carbamoyl]-2-cyano-phenyl]-4-cyano-2-methyl-benzamide; 2-(1,3-dioxan-2-yl)-6-[2-(3-pyridinyl)-5-thiazolyl]-pyridine; 2-[6-[2-(5-fluoro-3-pyridinyl)-5-thiazolyl]-2-pyridinyl]-pyrimidine; 2-[6-[2-(3-pyridinyl)-5-thiazolyl]-2-pyridinyl]-pyrimidine; *N*-methylsulfonyl-6-[2-(3-pyridyl)thiazol-5-yl]pyridine-2-carboxamide; *N*-methylsulfonyl-6-[2-(3-pyridyl)thiazol-5-yl]pyridine-2-carboxamide; 1-[(6-chloro-3-pyridinyl)methyl]-1,2,3,5,6,7-hexahydro-5-methoxy-7-methyl-8-nitro-imidazo[1,2-a]pyridine; 1-[(6-chloropyridin-3-yl)methyl]-7-methyl-8-nitro-1,2,3,5,6,7-hexahydroimidazo[1,2-a]pyridin-5-ol; 1-isopropyl-N,5-dimethyl-*N*-pyridazin-4-yl-pyrazole-4-carboxamide; 1-(1,2-dimethylpropyl)-*N-*ethyl-5-methyl-*N*-pyridazin-4-yl-pyrazole-4-carboxamide; *N*,5-dimethyl-*N*-pyridazin-4-yl-1-(2,2,2-trifluoro-1-methyl-ethyl)pyrazole-4-carboxamide; 1-[1-(1-cyanocyclopropyl)ethyl]-N-ethyl-5-methyl-*N*-pyridazin-4-yl-pyrazole-4-carboxamide; *N*-ethyl-1-(2-fluoro-1-methyl-propyl)-5-meth-yl-*N*-pyridazin-4-yl-pyrazole-4-carboxamide; 1-(1,2-dimethylpropyl)-*N*,5-dimethyl-*N*-pyridazin-4-yl-pyrazole-4-carboxamide; 1-[1-(1-cyanocyclopropyl)ethyl]-*N*,5-dimethyl-*N*-pyridazin-4-yl-pyrazole-4-carboxamide; *N*-methyl-1-(2-fluoro-1-methyl-propyl]-5-methyl-N-pyridazin-4-yl-pyrazole-4-carboxamide; 1-(4,4-difluorocyclohexyl)-*N*-ethyl-5-methyl-*N*-pyridazin-4-yl-pyrazole-4-carboxamide; 1-(4,4-difluorocyclohexyl)*-N*,5-dimethyl-*N*-pyridazin-4-ylpyrazole-4-carboxamide, *N*-(1-methylethyl)-2-(3-pyridinyl)-2H-indazole-4-carboxamide; *N-*cyclopropyl-2-(3-pyridinyl)-2*H*-indazole-4-carboxamide; *N*-cyclohexyl-2-(3-pyridinyl)-2*H*-indazole-4-carboxamide; 2-(3-pyridinyl)-*N*-(2,2,2-trifluoroethyl)-2*H*-indazole-4-carboxamide; 2-(3-pyridinyl)-*N*-[(tetrahydro-2-furanyl)methyl]-2*H*-indazole-5-carboxamide; methyl 2-[[2-(3-pyridinyl)-2*H*-indazol-5-yl]carbonyl]hydrazinecarboxylate; *N*-[(2,2-difluorocyclopropyl)methyl]-2-(3-pyridinyl)-2*H*-indazole-5-carboxamide; *N*-(2,2-difluoropropyl)-2-(3-pyridinyl)-2H-indazole-5-carboxamide; 2-(3-pyridinyl )-*N*-(2-pyrimidinylmethyl )-2*H*-indazole-5-carboxamide; *N*-[(5-methyl-2-pyrazinyl)methyl]-2-(3-pyridinyl)-2*H*-indazole-5-carboxamide, tyclopyrazoflor; sarolaner, lotilaner, *N*-[4-chloro-3-[[(phenylmethyl)amino]carbonyl]phenyl]-1-methyl-3-(1,1,2,2,2-pentafluoroethyl)-4-(trifluoromethyl)-1*H*-pyrazole-5-carboxamide; 2-(3-ethylsulfonyl-2-pyridyl)-3-methyl-6-(trifluoromethyl)imidazo[4,5-b]pyridine, 2-[3-ethylsulfonyl-5-(trifluoromethyl)-2-pyridyl]-3-methyl-6-(trifluoromethyl)imidazo[4,5-b]pyridine, isocycloseram, *N-*[4-chloro-3-(cyclopropylcarbamoyl)phenyl]-2-methyl-5-(1,1,2,2,2-pentafluoroethyl)-4-(trifluoromethyl)pyrazole-3-carboxamide, *N*-[4-chloro-3-[(1-cyanocyclopropyl)carbamoyl]phenyl]-2-methyl-5-(1,1,2,2,2-pentafluoroethyl)-4-(trifluoromethyl)pyrazole-3-carboxamide; acynonapyr; benzpyrimoxan; tigolaner; chloro-*N*-(1-cyanocyclopropyl)-5-[1-[2-methyl-5-(1,1,2,2,2-pentafluoroethyl)-4-(trifluoromethyl)pyrazol-3-yl]pyrazol-4-yl]benzamide, oxazosulfyl, [(2S,3R,4R,5S,6S)-3,5-dimethoxy-6-methyl-4-propoxy-tetrahydropyran-2-yl]-*N*-[4-[1-[4-(trifluoromethoxy)phenyl]-1,2,4-triazol-3-yl]phenyl]carbamate, [(2*S*,3*R*,4*R*,5*S*,6*S*)-3,4,5-trimethoxy-6-methyl-tetrahydropyran-2-yl] N-[4-[1-[4-(trifluoromethoxy)phenyl]-1,2,4-triazol-3-yl]phenyl]carbamate, [(2*S*,3*R*,4*R*,5*S*,6*S*)-3,5-dimethoxy-6-methyl-4-propoxy-tetrahydropyran-2-yl]-*N*-[4-[1-[4-(1,1,2,2,2-pentafluoroethoxy)phenyl]-1,2,4-triazol-3-yl]phenyl]carbamate, [(2*S*,3*R*,4*R*,5*S*,6*S*)-3,4,5-trimethoxy-6-methyl-tetrahydropyran-2-yl]-*N*-[4-[1-[4-(1,1,2,2,2-pentafluoroethoxy)phenyl]-1,2,4-triazol-3-yl]phenyl]carbamate, (2*Z*)-3-(2-isopropylphenyl)-2-[(*E*)-[4-[1-[4-(1,1,2,2,2-pentafluoroethoxy)phenyl]-1,2,4-triazol-3-yl]phenyl]methylenehydrazono]thiazolidin-4-one; 2-(6-chloro-3-ethylsulfonyl-imidazo[1,2-a]pyridin-2-yl)-3-methyl-6-(trifluoromethyl)imidazo[4,5-b]pyridine, 2-(6-bromo-3-ethylsulfonyl-imidazo[1,2-a]pyridin-2-yl)-3-methyl-6-(trifluoromethyl)imidazo[4,5-b]pyridine, 2-(3-ethylsulfonyl-6-iodo-imidazo[1,2-a]pyridin-2-yl)-3-methyl-6-(trifluoromethyl)imidazo[4,5-b]pyridine, 2-[3-ethylsulfonyl-6-(trifluoromethyl)imidazo[1,2-a]pyridin-2-yl]-3-methyl-6-(trifluoromethyl)imidazo[4,5-b]pyridine, 2-(7-chloro-3-ethylsulfonyl-imidazo[1,2-a]pyridin-2-yl)-3-methyl-6-(trifluoromethyl)imidazo[4,5-b]pyridine, 2-(3-ethylsulfonyl-7-iodo-imidazo[1,2-a]pyridin-2-yl)-3-methyl-6-(trifluoromethyl)imidazo-[4,5-b]pyridine, 3-ethylsulfonyl-6-iodo-2-[3-methyl-6-(trifluoromethyl)imidazo[4,5-b]pyridin-2-yl]imidazo[1,2-a]pyridine-8-carbonitrile, 2-[3-ethylsulfonyl-8-fluoro-6-(trifluoromethyl)imidazo[1,2-a]pyridin-2-yl]-3-methyl-6-(trifluoromethyl)imidazo[4,5-b]pyridine, 2-[3-ethylsulfonyl-7-(trifluoromethyl)imidazo[1,2-a]pyridin-2-yl]-3-methyl-6-(trifluoromethylsulfinyl)imidazo-[4,5-b]pyridine, 2-[3-ethylsulfonyl-7-(trifluoromethyl)imidazo[1,2-a]pyridin-2-yl]-3-methyl-6-(trifluoromethyl)imidazo[4,5-c]pyridine, 2-(6-bromo-3-ethylsulfonyl-imidazo[1,2-a]pyridin-2-yl)-6-(trifluoromethyl)pyrazolo[4,3-c]pyridine.

The active substances referred to as component 2, their preparation and their activity e. g. against harmful fungi is known (cf.: http://www.alanwood.net/pesticides/); these substances are commercially available. The compounds described by IUPAC nomenclature, their preparation and their pesticidal activity are also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 10/139271, WO 11/028657, WO 12/168188, WO 07/006670, WO 11/77514; WO 13/047749, WO 10/069882, WO 13/047441, WO 03/16303, WO 09/90181, WO 13/007767, WO 13/010862, WO 13/127704, WO 13/024009, WO 13/24010, WO 13/047441, WO 13/162072, WO 13/092224, WO 11/135833, CN 1907024, CN 1456054, CN 103387541, CN 1309897, WO 12/84812, CN 1907024, WO 09094442, WO 14/60177, WO 13/116251, WO 08/013622, WO 15/65922, WO 94/01546, EP 2865265, WO 07/129454, WO 12/165511, WO 11/081174, WO 13/47441, WO 16/156241, WO 16/162265). Some compounds are identified by their CAS Registry Number which is separated by hyphens into three parts, the first consisting from two up to seven digits, the second consisting of two digits, and the third consisting of a single digit.

According to the invention, the solid material (dry matter) of the biopesticides (with the exception of oils such as Neem oil) are considered as active components (e. g. to be obtained after drying or evaporation of the extraction or suspension medium in case of liquid formulations of the microbial pesticides). The weight ratios and percentages used for a biological extract such as Quillay extract are based on the total weight of the dry content (solid material) of the respective extract(s).

The total weight ratios of compositions comprising at least one microbial pesticide in the form of viable microbial cells including dormant forms, can be determined using the amount of CFU of the respective microorganism to calculate the total weight of the respective active component with the following equation that 1 x 10¹⁰ CFU equals one gram of total weight of the respective active component. Colony forming unit is measure of viable microbial cells. In addition, CFU may also be understood as the number of (juvenile) individual nematodes in case of nematode biopesticides, such as *Steinernema feltiae.*

In the binary mixtures the weight ratio of the component 1) and the component 2) generally depends from the properties of the components used, usually it is in the range of from 1:10,000 to 10,000:1, often from 1:100 to 100:1, regularly from 1:50 to 50:1, preferably from 1:20 to 20:1, more preferably from 1:10 to 10:1, even more preferably from 1:4 to 4:1 and in particular from 1:2 to 2:1. According to further embodiments, the weight ratio of the component 1) and the component 2) usually is in the range of from 1000:1 to 1:1, often from 100: 1 to 1:1, regularly from 50:1 to 1:1, preferably from 20:1 to 1:1, more preferably from 10:1 to 1:1, even more preferably from 4:1 to 1:1 and in particular from 2:1 to 1:1. According to further embodiments, the weight ratio of the component 1) and the component 2) usually is in the range of from 20,000:1 to 1:10, often from 10,000:1 to 1:1, regularly from 5,000:1 to 5:1, preferably from 5,000:1 to 10:1, more preferably from 2,000:1 to 30:1, even more preferably from 2,000:1 to 100:1 and in particular from 1,000:1 to 100:1. According to further embodiments, the weight ratio of the component 1) and the component 2) usually is in the range of from 1:1 to 1:1000, often from 1:1 to 1:100, regularly from 1:1 to 1:50, preferably from 1:1 to 1:20, more preferably from 1:1 to 1:10, even more preferably from 1:1 to 1:4 and in particular from 1:1 to 1:2. According to further embodiments, the weight ratio of the component 1) and the component 2) usually is in the range of from 10:1 to 1:20,000, often from 1:1 to 1:10,000, regularly from 1:5 to 1:5,000, preferably from 1:10 to 1:5,000, more preferably from 1:30 to 1:2,000, even more preferably from 1:100 to 1:2,000 to and in particular from 1:100 to 1:1,000.

In the ternary mixtures, i.e. compositions comprising the component 1) and component 2) and a compound III (component 3), the weight ratio of component 1) and component 2) depends from the properties of the active substances used, usually it is in the range of from 1:100 to 100:1, regularly from 1:50 to 50:1, preferably from 1:20 to 20:1, more preferably from 1:10 to 10:1 and in particular from 1:4 to 4:1, and the weight ratio of component 1) and component 3) usually it is in the range of from 1:100 to 100:1, regularly from 1:50 to 50:1, preferably from 1:20 to 20:1, more preferably from 1:10 to 10:1 and in particular from 1:4 to 4:1. Any further active components are, if desired, added in a ratio of from 20:1 to 1:20 to the component 1). These ratios are also suitable for mixtures applied by seed treatment.

When mixtures comprising microbial pesticides are employed in crop protection, the application rates range from 1 x 10⁶ to 5 x 10¹⁶ (or more) CFU/ha, preferably from 1 x 10⁸ to 1 x 10¹³ CFU/ha, and even more preferably from 1 x 10⁹ to 5 x 10¹⁵ CFU/ha and in particular from 1 x 10¹² to 5 x 10¹⁴ CFU/ha. In the case of nematodes as microbial pesticides (e. g. *Steinernema feltiae*)*,* the application rates regularly range from 1 x 10⁶ to 1 x 10¹² (or more), preferably from 1 x 10⁸ to 1 x 10¹¹, more preferably from 5 x 10⁸ to 1 x 10¹⁰ individuals (e. g. in the form of eggs, juvenile or any other live stages, preferably in an infetive juvenile stage) per ha.

When mixtures comprising microbial pesticides are employed in seed treatment, the application rates generally range from 1 x 10⁶ to 1 x 10¹² (or more) CFU/seed, preferably from 1 x 10⁶ to 1 x 10⁹ CFU/seed. Furthermore, the application rates with respect to seed treatment generally range from 1 x 10⁷ to 1 x 10¹⁴ (or more) CFU per 100 kg of seed, preferably from 1 x 10⁹ to 1 x 10¹² CFU per 100 kg of seed.

Preference is given to mixtures comprising as component 2) at least one active substance selected from inhibitors of complex III at Qₒ site in group A), more preferably selected from compounds (A.1.1), (A.1.4), (A.1.8), (A.1.9), (A.1.10), (A.1.12), (A.1.13), (A.1.14), (A.1.17), (A.1.21), (A.1.25), (A.1.34) and (A.1.35); particularly selected from (A.1.1), (A.1.4), (A.1.8), (A.1.9), (A.1.13), (A.1.14), (A.1.17), (A.1.25), (A.1.34) and (A.1.35).

Preference is also given to mixtures comprising as component 2) at least one active substance selected from inhibitors of complex III at Qᵢ site in group A), more preferably selected from compounds (A.2.1), (A.2.3), (A.2.4) and (A.2.6); particularly selected from (A.2.3), (A.2.4) and (A.2.6).

Preference is also given to mixtures comprising as component 2) at least one active substance selected from inhibitors of complex II in group A), more preferably selected from compounds (A.3.2), (A.3.3), (A.3.4), (A.3.7), (A.3.9), (A.3.11), (A.3.12), (A.3.15), (A.3.16), (A.3.17), (A.3.18), (A.3.19), (A.3.20), (A.3.21), (A.3.22), (A.3.23), (A.3.24), (A.3.28), (A.3.31), (A.3.32), (A.3.33), (A.3.34), (A.3.35), (A.3.36), (A.3.37), (A.3.38) and (A.3.39); particularly selected from (A.3.2), (A.3.3), (A.3.4), (A.3.7), (A.3.9), (A.3.12), (A.3.15), (A.3.17), (A.3.19), (A.3.22), (A.3.23), (A.3.24), (A.3.31), (A.3.32), (A.3.33), (A.3.34), (A.3.35), (A.3.36), (A.3.37), (A.3.38) and (A.3.39).

Preference is also given to mixtures comprising as component 2) at least one active substance selected from other respiration inhibitors in group A), more preferably selected from compounds (A.4.5) and (A.4.11); in particular (A.4.11).

Preference is also given to mixtures comprising as component 2) at least one active substance selected from C14 demethylase inhibitors in group B), more preferably selected from compounds (B.1.4), (B.1.5), (B.1.8), (B.1.10), (B.1.11), (B.1.12), (B.1.13), (B.1.17), (B.1.18), (B.1.21), (B.1.22), (B.1.23), (B.1.25), (B.1.26), (B.1.29), (B.1.34), (B.1.37), (B.1.38), (B.1.43), (B.1.46), (B.1.53), (B.1.54) and (B.1.55); particularly selected from (B.1.5), (B.1.8), (B.1.10), (B.1.17), (B.1.22), (B.1.23), (B.1.25), (B.1.33), (B.1.34), (B.1.37), (B.1.38), (B.1.43) and (B.1.46).

Preference is also given to mixtures comprising as component 2) at least one active substance selected from Delta14-reductase inhibitors in group B), more preferably selected from compounds (B.2.4), (B.2.5), (B.2.6) and (B.2.8); in particular (B.2.4).

Preference is also given to mixtures comprising as component 2) at least one active substance selected from phenylamides and acyl amino acid fungicides in group C), more preferably selected from compounds (C.1.1), (C.1.2), (C.1.4) and (C.1.5); particularly selected from (C.1.1) and (C.1.4).

Preference is also given to mixtures comprising as component 2) at least one active substance selected from other nucleic acid synthesis inhibitors in group C), more preferably selected from compounds (C.2.6), (C.2.7) and (C.2.8).

Preference is also given to mixtures comprising as component 2) at least one active substance selected from group D), more preferably selected from compounds (D.1.1), (D.1.2), (D.1.5), (D.2.4) and (D.2.6); particularly selected from (D.1.2), (D.1.5) and (D.2.6).

Preference is also given to mixtures comprising as component 2) at least one active substance selected from group E), more preferably selected from compounds (E.1.1), (E.1.3), (E.2.2) and (E.2.3); in particular (E.1.3).

Preference is also given to mixtures comprising as component 2) at least one active substance selected from group F), more preferably selected from compounds (F.1.2), (F.1.4) and (F.1.5).

Preference is also given to mixtures comprising as component 2) at least one active substance selected from group G), more preferably selected from compounds (G.3.1), (G.3.3), (G.3.6), (G.5.1), (G.5.3), (G.5.4), (G.5.5), G.5.6), G.5.7), (G.5.8), (G.5.9), (G.5.10) and (G.5.11); particularly selected from (G.3.1), (G.5.1) and (G.5.3).

Preference is also given to mixtures comprising as component 2) at least one active substance selected from group H), more preferably selected from compounds (H.2.2), (H.2.3), (H.2.5), (H.2.7), (H.2.8), (H.3.2), (H.3.4), (H.3.5), (H.4.9) and (H.4.10); particularly selected from (H.2.2), (H.2.5), (H.3.2), (H.4.9) and (H.4.10).

Preference is also given to mixtures comprising as component 2) at least one active substance selected from group I), more preferably selected from compounds (I.2.2) and (I.2.5).

Preference is also given to mixtures comprising as component 2) at least one active substance selected from group J), more preferably selected from compounds (J.1.2), (J.1.5), (J.1.8), (J.1.11) and (J.1.12); in particular (J.1.5).

Preference is also given to mixtures comprising as component 2) at least one active substance selected from group K), more preferably selected from compounds (K.1.41), (K.1.42), (K.1.44), (K.1.47), (K.1.57), (K.1.58) and (K.1.59); particularly selected from (K.1.41), (K.1.44), (K.1.47), (K.1.57), (K.1.58) and (K.1.59).

The biopesticides from group L1) and/or L2) may also have insecticidal, acaricidal, molluscidal, pheromone, nematicidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) may also have fungicidal, bactericidal, viricidal, plant defense activator, insecticidal, acaricidal, molluscidal, pheromone and/or nematicidal activity.

The microbial pesticides, in particular those from groups L1), L3) and L5), embrace not only the isolated, pure cultures of the respective microorganism as defined herein, but also its cell-free extract, its suspension in a whole broth culture and a metabolite-containing culture medium or a purified metabolite obtained from a whole broth culture of the microorganism.

Many of these biopesticides have been deposited under deposition numbers mentioned herein (the prefices such as ATCC or DSM refer to the acronym of the respective culture collection, for details see e. g. here: http://www. wfcc.info/ccinfo/collection/by acronym/), are referred to in literature, registered and/or are commercially available: mixtures of *Aureobasidium pullulans* DSM 14940 and DSM 14941 isolated in 1989 in Konstanz, Germany (e. g. blastospores in BlossomProtect^{®} from bio-ferm GmbH, Austria), *Azospirillum brasilense* Sp245 originally isolated in wheat reagion of South Brazil (Passo Fundo) at least prior to 1980 (BR 11005; e. g. GELFIX^{®} Gramineas from BASF Agricultural Specialties Ltd., Brazil), *A. brasilense* strains Ab-V5 and Ab-V6 (e. g. in AzoMax from Novozymes BioAg Produtos papra Agricultura Ltda., Quattro Barras, Brazil or Simbiose-Maiz^{®} from Simbiose-Agro, Brazil; Plant Soil 331, 413-425, 2010*), Bacillus amyloliquefaciens* strain AP-188 (NRRL B-50615 and B-50331; US 8,445,255); *B. amyloliquefaciens* ssp. *plantarum* strains formerly also sometimes referred to as *B*. *subtilis,* recently together with *B*. *methylotrophicus,* and *B*. *velezensis* classified as *B*. *velezensis* (Int. J. Syst. Evol. Microbiol. 66, 1212-1217, 2016): *B*. a. ssp. *plantarum* or *B*. *velezensis* D747 isolated from air in Kikugawa-shi, Japan (US 20130236522 A1; FERM BP-8234; e. g. Double Nickel^{™} 55 WDG from Certis LLC, USA), *B*. a. ssp. *plantarum* or *B*. *velezensis* FZB24 isolated from soil in Brandenburg, Germany (also called SB3615; DSM 96-2; J. Plant Dis. Prot. 105, 181-197, 1998; e. g. Taegro^{®} from Novozyme Biologicals, Inc., USA), *B*. a. ssp. *plantarum* or *B. velezensis* FZB42 isolated from soil in Brandenburg, Germany (DSM 23117; J. Plant Dis. Prot. 105, 181-197, 1998; e. g. RhizoVital^{®} 42 from AbiTEP GmbH, Germany), *B*. a. ssp. *plantarum* or *B*. *velezensis* MBI600 isolated from faba bean in Sutton Bonington, Nottinghamshire, U.K. at least before 1988 (also called 1430; NRRL B-50595; US 2012/0149571 A1; e. g. Integral^{®} from BASF Corp., USA), *B*. a. ssp. *plantarum* or *B*. *velezensis* QST-713 isolated from peach orchard in 1995 in California, U.S.A. (NRRL B-21661; e. g. Serenade^{®} MAX from Bayer Crop Science LP, USA), *B*. a. ssp. *plantarum* or *B*. *velezensis* TJ1000 isolated in 1992 in South Dakoda, U.S.A. (also called 1BE; ATCC BAA-390; CA 2471555 A1; e. g. QuickRoots^{™} from TJ Technologies, Watertown, SD, USA); *B. firmus* CNCM I-1582, a variant of parental strain EIP-N1 (CNCM I-1556) isolated from soil of central plain area of Israel (WO 2009/126473, US 6,406,690; e. g. Votivo^{®} from Bayer CropScience LP, USA), *B. pumilus* GHA 25 isolated from apple tree rhizosphere in Mexico (IDAC 260707-01; e. g. PRO-MIX^{®} BX from Premier Horticulture, Quebec, Canada), *B. pumilus* INR-7 otherwise referred to as BU-F22 and BU-F33 isolated at least before 1993 from cucumber infested by *Erwinia tracheiphila* (NRRL B-50185, NRRL B-50153; US 8,445,255), *B. pumilus* KFP9F isolated from the rhizosphere of grasses in South Africa at least before 2008 (NRRL B-50754; WO 2014/029697; e. g. BAC-UP or FUSION-P from BASF Agricultural Specialities (Pty) Ltd., South Africa), *B. pumilus* QST 2808 was isolated from soil collected in Pohnpei, Federated States of Micronesia, in 1998 (NRRL B-30087; e. g. Sonata^{®} or Ballad^{®} Plus from Bayer Crop Science LP, USA), *B. simplex* ABU 288 (NRRL B-50304; US 8,445,255), *B. subtilis* FB17 also called UD 1022 or UD10-22 isolated from red beet roots in North America (ATCC PTA-11857; System. Appl. Microbiol. 27, 372-379, 2004; US 2010/0260735; WO 2011/109395); *B. thuringiensis* ssp. *aizawai* ABTS-1857 isolated from soil taken from a lawn in Ephraim, Wisconsin, U.S.A., in 1987 (also called ABG-6346; ATCC SD-1372; e. g. XenTari^{®} from BioFa AG, Münsingen, Germany), *B. t.* ssp. *kurstaki* ABTS-351 identical to HD-1 isolated in 1967 from diseased Pink Bollworm black larvae in Brownsville, Texas, U.S.A. (ATCC SD-1275; e. g. Dipel^{®} DF from Valent BioSciences, IL, USA), *B. t.* ssp. *kurstaki* SB4 isolated from *E. saccharina* larval cadavers (NRRL B-50753; e. g. Beta Pro^{®} from BASF Agricultural Specialities (Pty) Ltd., South Africa), *B. t.* ssp. *tenebrionis* NB-176-1, a mutant of strain NB-125, a wild type strain isolated in 1982 from a dead pupa of the beetle Tenebrio molitor (DSM 5480; EP 585 215 B1; e. g. Novodor^{®} from Valent BioSciences, Switzerland), *Beauveria bassiana* GHA (ATCC 74250; e. g. BotaniGard^{®} 22WGP from Laverlam Int. Corp., USA), *B*. *bassiana* JW-1 (ATCC 74040; e. g. Naturalis^{®} from CBC (Europe) S.r.l., Italy), *B*. *bassiana* PPRI 5339 isolated from the larva of the tortoise beetle *Conchyloctenia punctata* (NRRL 50757; e. g. BroadBand^{®} from BASF Agricultural Specialities (Pty) Ltd., South Africa), *Bradyrhizobium elkanii* strains SEMIA 5019 (also called 29W) isolated in Rio de Janeiro, Brazil and SEMIA 587 isolated in 1967 in the State of Rio Grande do Sul, from an area previously inoculated with a North American isolate, and used in commercial inoculants since 1968 (Appl. Environ. Microbiol. 73(8), 2635, 2007; e. g. GELFIX 5 from BASF Agricultural Specialties Ltd., Brazil), *B. japonicum* 532c isolated from Wisconsin field in U.S.A. (Nitragin 61A152; Can. J. Plant. Sci. 70, 661-666, 1990; e. g. in Rhizoflo^{®}, Histick^{®}, Hicoat^{®} Super from BASF Agricultural Specialties Ltd., Canada), *B. japonicum* E-109 variant of strain USDA 138 (INTA E109, SEMIA 5085; Eur. J. Soil Biol. 45, 28-35, 2009; Biol. Fertil. Soils 47, 81-89, 2011); *B. japonicum* strains deposited at SEMIA known from Appl. Environ. Microbiol. 73(8), 2635, 2007: SEMIA 5079 isolated from soil in Cerrados region, Brazil by Embrapa-Cerrados used in commercial inoculants since 1992 (CPAC 15; e. g. GELFIX 5 or ADHERE 60 from BASF Agricultural Specialties Ltd., Brazil), *B. japonicum* SEMIA 5080 obtained under lab condtions by Embrapa-Cerrados in Brazil and used in commercial inoculants since 1992, being a natural variant of SEMIA 586 (CB259) originally isolated in U.S.A. (CPAC 7; e. g. GELFIX 5 or ADHERE 60 from BASF Agricultural Specialties Ltd., Brazil); *Burkholderia sp.* A396 isolated from soil in Nikko, Japan, in 2008 (NRRL B-50319; WO 2013/032693; Marrone Bio Innovations, Inc., USA), *Coniothyrium minitans* CON/M/91-08 isolated from oilseed rape (WO 1996/021358; DSM 9660; e. g. Contans^{®} WG, Intercept^{®} WG from Bayer CropScience AG, Germany), harpin (alpha-beta) protein (Science 257, 85-88, 1992; e. g. Messenger^{™} or HARP-N-Tek from Plant Health Care plc, U.K.), *Helicoverpa armigera* nucleopolyhedrovirus (HearNPV) (J. Invertebrate Pathol. 107, 112-126, 2011; e. g. Helicovex^{®} from Adermatt Biocontrol, Switzerland; Diplomata^{®} from Koppert, Brazil; Vivus^{®} Max from AgBiTech Pty Ltd., Queensland, Australia), *Helicoverpa zea* single capsid nucleopolyhedrovirus (HzSNPV) (e. g. Gemstar^{®} from Certis LLC, USA), *Helicoverpa zea* nucleopolyhedrovirus ABA-NPV-U (e. g. Heligen^{®} from AgBiTech Pty Ltd., Queensland, Australia), *Heterorhabditis bacteriophora* (e. g. Nemasys^{®} G from BASF Agricultural Specialities Limited, UK), *Isaria fumosorosea* Apopka-97 isolated from mealy bug on gynura in Apopka, Florida, U.S.A. (ATCC 20874; Biocontrol Science Technol. 22(7), 747-761, 2012; e. g. PFR-97^{™} or PreFeRal^{®} from Certis LLC, USA), *Metarhizium anisopliae* var*. anisopliae* F52 also called 275 or V275 isolated from codling moth in Austria (DSM 3884, ATCC 90448; e. g. Met52^{®} Novozymes Biologicals BioAg Group, Canada), *Metschnikowia fructicola* 277 isolated from grapes in the central part of Israel (US 6,994,849; NRRL Y-30752; e. g. formerly Shemer^{®} from Agrogreen, Israel), *Paecilomyces ilacinus* 251 isolated from infected nematode eggs in the Philippines (AGAL 89/030550; WO1991/02051; Crop Protection 27, 352-361, 2008; e. g. BioAct^{®}from Bayer CropScience AG, Germany and MeloCon^{®} from Certis, USA), *Paenibacillus alvei* NAS6G6 isolated from the rhizosphere of grasses in South Africa at least before 2008 (WO 2014/029697; NRRL B-50755; e.g. BAC-UP from BASF Agricultural Specialities (Pty) Ltd., South Africa), *Paenibacillus* strains isolated from soil samples from a variety of European locations including Germany: *P*. *epiphyticus* Lu17015 (WO 2016/020371; DSM 26971), *P. polymyxa* ssp. *plantarum* Lu16774 (WO 2016/020371; DSM 26969), *P. p.* ssp. *plantarum* strain Lu17007 (WO 2016/020371; DSM 26970); *Pasteuria nishizawae* Pn1 isolated from a soybean field in the mid-2000s in Illinois, U.S.A. (ATCC SD-5833; Federal Register 76(22), 5808, February 2, 2011; e.g. Clariva^{™} PN from Syngenta Crop Protection, LLC, USA), *Penicillium bilaiae* (also called *P*. *bilaii*) strains ATCC 18309 (= ATCC 74319), ATCC 20851 and/or ATCC 22348 (= ATCC 74318) originally isolated from soil in Alberta, Canada (Fertilizer Res. 39, 97-103, 1994; Can. J. Plant Sci. 78(1), 91-102, 1998; US 5,026,417,

WO 1995/017806; e. g. Jump Start^{®}, Provide^{®} from Novozymes Biologicals BioAg Group, Canada), *Reynoutria sachalinensis* extract (EP 0307510 B1; e. g. Regalia^{®} SC from Marrone Biolnnovations, Davis, CA, USA or Milsana^{®} from BioFa AG, Germany), *Steinemema carpocapsae* (e. g. Millenium^{®} from BASF Agricultural Specialities Limited, UK), *S. feltiae* (e. g. Nemashield^{®} from BioWorks, Inc., USA; Nemasys^{®} from BASF Agricultural Specialities Limited, UK), *Streptomyces microflavus* NRRL B-50550 (WO 2014/124369; Bayer CropScience, Germany), *Trichoderma asperelloides* JM41R isolated in South Africa (NRRL 50759; also referred to as *T. fertile;* e. g. Trichoplus^{®} from BASF Agricultural Specialities (Pty) Ltd., South Africa), *T. harzianum T-*22 also called KRL-AG2 (ATCC 20847; BioControl 57, 687-696, 2012; e. g. Plantshield^{®} from BioWorks Inc., USA or SabrEx^{™} from Advanced Biological Marketing Inc., Van Wert, OH, USA).

According to another embodiment of the mixtures, the at least one pesticide II is selected from the groups L1) to L5):
L1) Microbial pesticides with fungicidal, bactericidal, viricidal and/or plant defense activator activity: *Aureobasidium pullulans* DSM 14940 and DSM 14941 (L1.1), *Bacillus amyloliquefaciens* AP-188 (L.1.2), *B. amyloliquefaciens* ssp. *plantarum* D747 (L.1.3), *B. amyloliquefaciens* ssp. *plantarum* FZB24 (L.1.4), *B. amyloliquefaciens* ssp. *plantarum* FZB42 (L.1.5), *B*. *amyloliquefaciens* ssp. *plantarum* MBI600 (L.1.6), *B. amyloliquefaciens* ssp. *plantarum* QST-713 (L.1.7), *B. amyloliquefaciens* ssp. *plantarum* TJ1000 (L.1.8), *B. pumilus* GB34 (L.1.9), *B. pumilus* GHA 25 (L.1.10), *B. pumilus* INR-7 (L.1.11), *B. pumilus* KFP9F (L.1.12), *B. pumilus* QST 2808 (L.1.13), *B. simplex* ABU 288 (L.1.14), *B. subtilis* FB17 (L.1.15), *Coniothyrium minitans* CON/M/91-08 (L.1.16), *Metschnikowia fructicola* NRRL Y-30752 (L.1.17), *Paenibacillus alvei* NAS6G6 (L.1.18), *P. epiphyticus* Lu17015 (L.1.25), *P. polymyxa* ssp. *plantarum* Lu16774 (L.1.26), *P. p.* ssp. *plantarum* strain Lu17007 (L.1.27), *Penicillium bilaiae* ATCC 22348 (L.1.19), *P. bilaiae* ATCC 20851 (L.1.20), *Penicillium bilaiae* ATCC 18309 (L.1.21), *Streptomyces microflavus* NRRL B-50550 (L.1.22), *Trichoderma asperelloides* JM41 R (L.1.23), *T. harzianum* T-22 (L.1.24);
L2) Biochemical pesticides with fungicidal, bactericidal, viricidal and/or plant defense activator activity: harpin protein (L.2.1), *Reynoutria sachalinensis* extract (L.2.2);
L3) Microbial pesticides with insecticidal, acaricidal, molluscidal and/or nematicidal activity: Ba*cillus firmus* I-1582 (L.3.1); *B. thuringiensis* ssp. *aizawai* ABTS-1857 (L.3.2), *B. t.* ssp. *kurstaki* ABTS-351 (L.3.3), *B. t.* ssp. *kurstaki* SB4 (L.3.4), *B*. *t.* ssp. *tenebrionis* NB-176-1 (L.3.5), *Beauveria bassiana* GHA (L.3.6), *B*. *bassiana* JW-1 (L.3.7), *B*. *bassiana* PPRI 5339 (L.3.8), *Burkholderia* sp. A396 (L.3.9), *Helicoverpa armigera* nucleopolyhedrovirus (HearNPV) (L.3.10), *Helicoverpa zea* nucleopolyhedrovirus (HzNPV) ABA-NPV-U (L.3.11), *Helicoverpa zea* single capsid nucleopolyhedrovirus (HzSNPV) (L.3.12), *Heterohabditis bacteriophora* (L.3.13), *Isaria fumosorosea* Apopka-97 (L.3.14), *Metarhizium anisopliae* var. *anisopliae* F52 (L.3.15), *Paecilomyces lilacinus* 251 (L.3.16), *Pasteuria nishizawae* Pn1 (L.3.17), *Steinernema carpocapsae* (L.3.18), *S. feltiae* (L.3.19);
L4) Biochemical pesticides with insecticidal, acaricidal, molluscidal, pheromone and/or nematicidal activity: cis-jasmone (L.4.1), methyl jasmonate (L.4.2), Quillay extract (L.4.3);
L5) Microbial pesticides with plant stress reducing, plant growth regulator, plant growth promoting and/or yield enhancing activity: *Azospirillum brasilense* Ab-V5 and Ab-V6 (L.5.1), *A. brasilense* Sp245 (L.5.2), *Bradyrhizobium elkanii* SEMIA 587 (L.5.3), *B*. *elkanii* SEMIA 5019 (L.5.4), *B. japonicum* 532c (L.5.5), *B. japonicum* E-109 (L.5.6), *B. japonicum* SEMIA 5079 (L.5.7), *B. japonicum* SEMIA 5080 (L.5.8).

The present invention furthermore relates to agrochemical compositions comprising a mixture of at least one compound I (component 1) and at least one biopesticide selected from the group L) (component 2), in particular at least one biopesticide selected from the groups L1) and L2), as described above, and if desired at least one suitable auxiliary.

The present invention furthermore relates to agrochemical compositions comprising a mixture of of at least one compound I (component 1) and at least one biopesticide selected from the group L) (component 2), in particular at least one biopesticide selected from the groups L3) and L4), as described above, and if desired at least one suitable auxiliary.

Preference is also given to mixtures comprising as pesticide II (component 2) a biopesticide selected from the groups L1), L3) and L5), preferably selected from strains denoted above as (L.1.2), (L.1.3), (L.1.4), (L.1.5), (L.1.6), (L.1.7), (L.1.8), (L.1.10), (L.1.11), (L.1.12), (L.1.13), (L.1.14), (L.1.15), (L.1.17), (L.1.18), (L.1.19), (L.1.20), (L.1.21), (L.1.25), (L.1.26), (L.1.27), (L.3.1); (L.3.9), (L.3.16), (L.3.17), (L.5.1), (L.5.2), (L.5.3), (L.5.4), (L.5.5), (L.5.6), (L.5.7), (L.5.8); (L.4.2), and (L.4.1); even more preferably selected from (L.1.2), (L.1.6), (L.1.7), (L.1.8), (L.1.11), (L.1.12), (L.1.13), (L.1.14), (L.1.15), (L.1.18), (L.1.19), (L.1.20), (L.1.21), (L.3.1); (L.3.9), (L.3.16), (L.3.17), (L.5.1), (L.5.2), (L.5.5), (L.5.6); (L.4.2), and (L.4.1). These mixtures are particularly suitable for treatment of propagation materials, i. e. seed treatment purposes and likewise for soil treatment. These seed treatment mixtures are particularly suitable for crops such as cereals, corn and leguminous plants such as soybean.

Preference is also given to mixtures comprising as pesticide II (component 2) a biopesticide selected from the groups L1), L3) and L5), preferably selected from strains denoted above as (L1.1), (L.1.2), (L.1.3), (L.1.6), (L.1.7), (L.1.9), (L.1.11), (L.1.12), (L.1.13), (L.1.14), (L.1.15), (L.1.17), (L.1.18), (L.1.22), (L.1.23), (L.1.24), (L.1.25), (L.1.26), (L.1.27), (L.2.2); (L.3.2), (L.3.3), (L.3.4), (L.3.5), (L.3.6), (L.3.7), (L.3.8), (L.3.10), (L.3.11), (L.3.12), (L.3.13), (L.3.14), (L.3.15), (L.3.18), (L.3.19); (L.4.2), even more preferably selected from (L.1.2), (L.1.7), (L.1.11), (L.1.13), (L.1.14), (L.1.15), (L.1.18), (L.1.23), (L.3.3), (L.3.4), (L.3.6), (L.3.7), (L.3.8), (L.3.10), (L.3.11), (L.3.12), (L.3.15), and (L.4.2). These mixtures are particularly suitable for foliar treatment of cultivated plants, preferably of vegetables, fruits, vines, cereals, corn, and leguminous crops such as soybeans.

The compositions comprising mixtures of active ingredients can be prepared by usual means, e. g. by the means given for the compositions of compounds I.

When living microorganisms, such as pesticides II from groups L1), L3) and L5), form part of the compositions, such compositions can be prepared by usual means (e. g. H.D. Burges: Formulation of Microbial Biopesticides, Springer, 1998; WO 2008/002371, US 6,955,912, US 5,422,107).

### I. Synthesis examples

### Step 1 - Preparation of 2,2,3-trimethylchroman-4-one oxime

Hydroxylamine hydrochloride (72.87 g, 3eq) was added to a solution of 2,2,3-trimethylchroman-4-one (1 eq, 66.5g) in pyridine (423 ml, 15 eq) and the reaction mixture was stirred for 18h at 85°C. The reaction solution was poured into water (1000 ml), and the solution was extracted with heptane, washed successively with water and brine, and dried over anhydrous magnesium sulfate. Removal of solvent in vacuo afforded the titled compound (66.6) as a brown powder. The title compound was used directly without further purification.

¹H NMR (400 MHz, CDCl3): µ [ppm]: 9.15 (s, 1H), 7.75 (dd, J = 7.9, 1.7 Hz, 1H), 7.26 (dd, J = 7.1, 1.5 Hz, 1H), 6.91 (ddd, J = 8.2, 7.2, 1.2 Hz, 1H), 6.86 (dd, J = 8.3, 1.2 Hz, 1H), 3.39 (q, J = 7.0 Hz, 1H), 1.45 (s, 3H), 1.27 (s, 3H), 1.13 (d, J = 7.0 Hz, 3H).

### Step 2 - Preparation of 2,2,3-trimethyl-3,4-dihydro-1,4-benzoxazepin-5-one

2,2,3-trimethylchroman-4-one oxime (66 g, 1 eq) was added to thionyl chloride (80, 3.5 eq) at a temperature below 30°C, and the reaction mixture was stirred at 50°C for 17 hours. After removal of thionyl chloride in vacuo the residue was poured into 1,4-dioxan (500 ml) and water (200 ml), and stirred for 1 h at 80°C. After removal of 1,4-dioxan in vacuo the resultant residue was extracted with ethyl acetate, washed successively with water and brine, and dried over anhydrous magnesium sulfate. Removal of solvent in vacuo the crude product was purified by flash chromatography on silica gel using heptane/MTBE as eluent to give the titled compound (25.4 g) as a with powder.

¹H NMR (400 MHz, CDCl3): µ [ppm]: 7.74 (dd, J = 7.7, 1.8 Hz, 1H), 7.44 (td, J = 7.7, 1.8 Hz, 1H), 7.20 (td, J = 7.5, 1.1 Hz, 1H), 6.98 (dd, J = 8.1, 1.1 Hz, 1H), 6.44 (s, 1H), 3.37 (qd, J = 6.9, 5.2 Hz, 1H), 1.39 (s, 3H), 1.29 (s, 3H), 1.20 (d, J = 6.9 Hz, 3H).

### Step 3 - Preparation of 5-chloro-2,2,3-trimethyl-3H-1,4-benzoxazepine

A mixture of 2,2,3-trimethyl-3,4-dihydro-1,4-benzoxazepin-5-one (10g,1 eq) with phosphoryl chloride (100 ml) and 11.67g phosphorus(V) chloride (1.15eq) was stirred Heat for 2h at 110°C. After cooling, the reaction solution concentrated in vacuo, diluted with dichloromethane, washed twice with saturated sodium carbonate solution, and dried over anhydrous magnesium sulfate. Removal of solvent in vacuo afforded the crude product (10.8 g). The title compound was used directly without further purification.

### Step 4 - Preparation of 5-[6-(difluoromethoxy)-5-methyl-3-pyridyl]-2,2,3-trimethyl-3H-1,4-benzoxazepine

[6-(difluoromethyl)-5-methyl-3-pyridyl]boronic acid (3.11 g, 1.2 eq), potassium carbonate (3.83 g, 2 eq), silver oxide (1.61, 0.5 eq) and dichlorobis(triphenylphosphine)palladium(ll) (490 mg, 0.05 eq) were added to a solution of 5-chloro-2,2,3-trimethyl-3H-1,4-benzoxazepine (3.1 g, 1 eq) in dry tetrahydrofuran (57 mL), and the mixture was stirred under argon atmosphere at 80 °C for 18 hours. After cooling, the reaction solution was diluted with ethyl acetate, and the solution was washed successively with water and brine, dried over anhydrous magnesium sulfate, and concentrated in vacuo. The crude product was purified by High Performance Liquid Chromatography on silica RP-18 using acetonitrile / water as eluent to give the titled compound (1.5 g) as a white powder.

¹H NMR (400 MHz, CDCl3): µ [ppm]: 8.58 (d, J = 1.9 Hz, 1H), 7.92 (s, 1H), 7.46 (t, J = 7.7 Hz, 1H), 7.18 (td, J = 7.5, 1.2 Hz, 1H), 7.09 (td, J = 7.7, 1.4 Hz, 2H), 6.73 (t, J = 54.5 Hz, 1H), 3.28 (d, J = 6.8 Hz, 1H), 2.54 (d, J = 2.2 Hz, 3H), 1.59 (s, 3H), 1.45 (s, 3H), 1.39 (s, 3H).

**Table I: Compounds Ex-1 to Ex-101 of formula I, wherein the meaning of R², R³, R⁵, R⁶, R⁷, R⁸ and Xn are as defined in each line.**

| * HPLC: High Performance Liquid Chromatography; HPLC-column Kinetex XB C18 1,7µ (50 x 2,1 mm); eluent: acetonitrile / water + 0.1% trifluoroacetic acid (gradient from 5:95 to 100 : 0 in 1.5 min at 60°C, flow gradient from 0.8 to 1.0 ml/min in 1.5 min). Rt: retention time in minutes. | | | | | | | | |
|---|---|---|---|---|---|---|---|---|
| * Chiral HPLC: High Performance Liquid Chromatography on Shimadzu Nexera LC-30 LCMS-2020; flow: 0.6ml/min; HPLC-column: LUX i-Amylose-3 5 µm 150x4.6mm; Eluent A: water + 0.1% HCOOH; eluent B: acetonitrile; flow gradient from 50% B to 100% B in 10min at 40°C. | | | | | | | | |
| 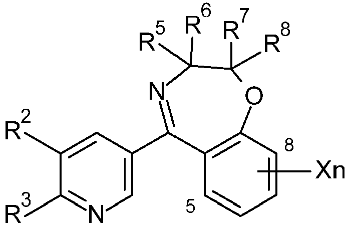 | | | | | | | | |
| **Ex-No** | **R²** | **R³** | **R⁵** | **R⁶** | **R⁷** | **R⁸** | **Xn** | **HPLC Rₜ (min)*** |
| Ex-1 | CH₃ | CH₃ | H | C(CH₃)₃ | H | H | H | 0,968 |
| Ex-2 | CH₃ | CH₃ | H | H | CH₃ | CH₃ | H | 0,75 |
| Ex-3 | CH₃ | CH₃ | CH₃ | CH₃ | CH₃ | CH₃ | H | 0,789 |
| Ex-4 | CH₃ | CHF₂ | CH₃ | CH₃ | CH₃ | CH₃ | H | 0,887 |
| Ex-5 | CH₃ | CH₃ | CH₃ | CH₃ | H | H | H | 0,684 |
| Ex-6 | CH₃ | CHF₂ | CH₃ | CH₃ | H | H | H | 0,794 |
| Ex-7 | CH₃ | CHF₂ | H | H | CH₃ | CH₂-O-CH₃ | H | 0.883 |
| Ex-8 | CH₃ | CHF₂ | H | H | CH₃ | CH₃ | H | 0.86 |
| Ex-9 | CH₃ | CH₃ | CH₃ | CH₃ | CH₃ | CH₂-O-CH₃ | H | 0.796 |
| Ex-10 | CH₃ | CHF₂ | CH₃ | CH₃ | CH₃ | CH₂-O-CH₃ | H | 0.893 |
| Ex-11 | CH₃ | CH₃ | H | CH₃ | CH₃ | CH₃ | H | 0,798 |
| Ex-12 | CH₃ | CHF₂ | H | CH₃ | CH₃ | CH₃ | H | 0,906 |
| Ex-13 | CH₃ | CH₃ | H | CH₃ | CH₃ | CH₂-O-CH₃ | H | 0.797 |
| Ex-14 | CH₃ | CHF₂ | H | CH₃ | CH₃ | CH₂-O-CH₃ | H | 0.927 |
| Ex-15 | CH₃ | CHF₂ | H | C(CH₃)₃ | CH₃ | H | H | 1.132 |
| Ex-16 | CH₃ | CH₃ | C=O | | CH₃ | CH₃ | H | 0,84 |
| Ex-17 | CH₃ | CHF₂ | C=O | | CH₃ | CH₃ | H | 1,224 |
| Ex-18 | CH₃ | CHF₂ | H | H | -(CH₂)₄ | | H | 0.962 |
| Ex-19 | CH₃ | CH₃ | H | H | -(CH₂)₄ | | H | 0.841 |
| Ex-20 | CH₃ | CHF₂ | CH₃ | CH₃ | -(CH₂)₃ | | H | 0.932 |
| Ex-21 | CH₃ | CHF₂ | -(CH₂)₂ | | H | H | H | 0.95 |
| Ex-22 | CH₃ | CHF₂ | H | H | -(CH₂)₃ | | H | 0.939 |
| Ex-23 | CH₃ | CHF₂ | H | C(CH₃)₃ | H | H | H | 1.364 |
| Ex-24 | CH₃ | CH₃ | H | C(CH₃)₃ | CH₃ | H | H | 0.954 |
| Ex-25 | CH₃ | CHF₂ | H | H | CH₃ | CH₃ | 8-F | 1.334 |
| Ex-26 | CH₃ | CHF₂ | CH₃ | H | CH₃ | CH₃ | 8-F | 1.058 |
| Ex-27 | CH₃ | CH₃ | H | H | CH₃ | CH₃ | 8-F | 0.791 |
| Ex-28 | CH₃ | CHF₂ | -(CH₂)₃ | | H | H | H | 0.917 |
| Ex-29 | CH₃ | CHF₂ | CH₃ | H | CH₃ | CH₃ | 8-Cl | 1.116 |
| Ex-30 | CH₃ | CHF₂ | H | H | CH₃ | CH₃ | 7-CH₃ | 0.897 |
| Ex-31 | CH₃ | CHF₂ | H | H | H | H | 7-CH₃ | 0.814 |
| Ex-32 | CH₃ | CHF₂ | H | H | -(CH₂)₂ | | H | 0.855 |
| Ex-33 | CH₃ | CHF₂ | CH₃ | H | CH₃ | CH₃ | 8-CH₃ | 0.945 |
| Ex-34 | CH₃ | CH₃ | CH₃ | H | CH₃ | CH₃ | 8-F | 0.823 |
| Ex-35 | CH₃ | CH₃ | CH₃ | H | CH₃ | CH₃ | 8-CH₃ | 0.837 |
| Ex-36 | CH₃ | CHF₂ | H | c-propyl | H | H | H | 0.952 |
| Ex-37 | CH₃ | CHF₂ | C=O | | CH₃ | CH₃ | 8-Br | 1.33 |
| Ex-38 | CH₃ | CHF₂ | C=O | | CH₃ | CH₃ | 2-F | 1.226 |
| Ex-39 | CH₃ | CHF₂ | C=O | | CH₃ | CH₃ | 8-CH₃ | 1.273 |
| Ex-40 | CH₃ | CHF₂ | C=O | | CH₃ | CH₃ | 8-F-7-OCH₃ | 1.222 |
| Ex-41 | CH₃ | CHF₂ | C=O | | -(CH₂)₂ | | 8-F | 1.209 |
| Ex-42 | CH₃ | CHF₂ | C=O | | -(CH₂)₃ | | 8-F | 1.296 |
| Ex-43 | CH₃ | CHF₂ | C=O | | CH₃ | CH₃ | 7,8-F₂ | 1.287 |
| Ex-44 | CH₃ | CHF₂ | CH₂CH 3 | CH₃ | H | H | H | 0.850 |
| Ex-45 | CH₃ | CHF₂ | H | CH₃ | H | CH₃ | H | 0.864 |
| Ex-46 | CH₃ | CHF₂ | C=O | | -(CH₂)₄ | | 8-F | 1.342 |
| Ex-47 | CH₃ | CHF₂ | (CH₂)₂ | | H | H | 8-F | 1.113 |
| Ex-48 | CH₃ | CH₃ | (CH₂)₂ | | H | H | 8-F | 0.746 |
| Ex-49 | CH₃ | CH₃ | CH₃ | H | CH₃ | CH₃ | 8-Cl | 0.873 |
| Ex-50 | CH₃ | CH₃ | CH₃ | H | CH₃ | CH₃ | 8-CF₃ | 0.979 |
| Ex-51 | CH₃ | CHF₂ | CH₃ | H | CH₃ | CH₃ | 8-CF₃ | 1.22 |
| Ex-52 | CH₃ | CHF₂ | H | H | CH₃ | (S)CH₂-O-CH₃ | H | 6.33 |
| Ex-53 | CH₃ | CHF₂ | H | H | CH₃ | (R)CH₂-O-CH₃ | H | 6.49 |
| Ex-54 | CH₃ | CH₃ | H | (S)CH₃ | CH₃ | (S)CH₂-O-CH₃ | H | 4.24 |
| Ex-55 | CH₃ | CH₃ | H | (S)CH₃ | CH₃ | (R)CH₂-O-CH₃ | H | 4.07 |
| Ex-56 | CH₃ | CH₃ | H | (R)CH₃ | CH₃ | (S)CH₂-O-CH₃ | H | |
| Ex-57 | CH₃ | CH₃ | H | (R)CH₃ | CH₃ | (R)CH₂-O-CH₃ | H | |
| Ex-58 | CH₃ | CHF₂ | H | (S)C(CH₃)₃ | H | (S)CH₃ | H | 10.70 |
| Ex-59 | CH₃ | CHF₂ | H | (S)C(CH₃)₃ | H | (R)CH₃ | H | |
| Ex-60 | CH₃ | CHF₂ | H | (R)C(CH₃)₃ | H | (S)CH₃ | H | |
| Ex-61 | CH₃ | CHF₂ | H | (R)C(CH₃)_{3 3} | H | (R)CH₃ | H | 9.59 |
| Ex-62 | CH₃ | CH₃ | H | (S)C(CH₃)₃ | H | (S)CH₃ | H | 7.34 |
| Ex-63 | CH₃ | CH₃ | H | (S)C(CH₃)₃ | H | (R)CH₃ | H | |
| Ex-64 | CH₃ | CH₃ | H | (R)C(CH₃)₃ | H | (S)CH₃ | H | |
| Ex-65 | CH₃ | CH₃ | H | (R)C(CH₃)₃ | H | (R)CH₃ | H | 7.12 |
| Ex-66 | CH₃ | CHF₂ | H | (S)CH₃ | CH₃ | CH₃ | 8-F | 7.34 |
| Ex-67 | CH₃ | CHF₂ | H | (R)CH₃ | CH₃ | CH₃ | 8-F | 7.12 |
| Ex-68 | CH₃ | CHF₂ | H | (R)CH₃ | CH₃ | CH₃ | H | 4.02 |
| Ex-69 | CH₃ | CHF₂ | H | (S)CH₃ | CH₃ | CH₃ | H | 4.291 |
| Ex-70 | CH₃ | CHF₂ | H | (S)CH₃ | CH₃ | CH₃ | 8-Cl | 9.22 |
| Ex-71 | CH₃ | CHF₂ | H | (R)CH₃ | CH₃ | CH₃ | 8-Cl | 9.09 |
| Ex-72 | CH₃ | CHF₂ | H | (S)CH₃ | CH₃ | CH₃ | 8-CH₃ | |
| Ex-73 | CH₃ | CHF₂ | H | (R)CH₃ | CH₃ | CH₃ | 8-CH₃ | |
| Ex-74 | CH₃ | CH₃ | H | (S)CH₃ | CH₃ | CH₃ | 8-F | |
| Ex-75 | CH₃ | CH₃ | H | (R)CH₃ | CH₃ | CH₃ | 8-F | |
| Ex-76 | CH₃ | CH₃ | H | (S)CH₃ | CH₃ | CH₃ | 8-CH₃ | |
| Ex-77 | CH₃ | CH₃ | H | (R)CH₃ | CH₃ | CH₃ | 8-CH₃ | |
| Ex-78 | CH₃ | CHF₂ | H | (S)CH₃ | H | (R)CH₃ | H | 5.998 |
| Ex-79 | CH₃ | CHF₂ | H | (S)CH₃ | H | (S)CH₃ | H | 7.793 |
| Ex-80 | CH₃ | CHF₂ | H | (R)CH₃ | H | (S)CH₃ | H | 8.259 |
| Ex-81 | CH₃ | CHF₂ | H | (S)c-propyl | H | H | H | |
| Ex-82 | CH₃ | CHF₂ | H | (R)c-propyl | H | H | H | |
| Ex-83 | CH₃ | CH₃ | H | (S)CH₃ | H | (S)CH₃ | H | 4.64 |
| Ex-84 | CH₃ | CH₃ | H | (S)CH₃ | H | (R)CH₃ | H | 4.107 |
| Ex-85 | CH₃ | CH₃ | H | (R)CH₃ | H | (S)CH₃ | H | 4.82 |
| Ex-86 | CH₃ | CHF₂ | (S)CH₃ | (S)CH₂CH₃ | H | H | H | 5.42 |
| Ex-87 | CH₃ | CHF₂ | (R)CH₃ | (R)CH₂CH₃ | H | H | H | 5.37 |
| Ex-88 | CH₃ | CHF₂ | H | (S)CH₃ | H | (S)CH₃ | H | |
| Ex-89 | CH₃ | CHF₂ | H | (S)CH₃ | H | (R)CH₃ | H | |
| Ex-90 | CH₃ | CHF₂ | H | (R)CH₃ | H | (S)CH₃ | H | |
| Ex-91 | CH₃ | CHF₂ | H | (R)CH₃ | H | (R)CH₃ | H | |
| Ex-92 | CH₃ | CHF₂ | H | (R)CH₃ | CH₃ | CH₃ | 8-F | 5.521 |
| Ex-93 | CH₃ | CHF₂ | H | (S)CH₃ | CH₃ | CH₃ | 8-F | 6.011 |
| Ex-94 | CH₃ | CH₃ | H | (S)CH₃ | CH₃ | CH₃ | 8-Cl | 5.68 |
| Ex-95 | CH₃ | CH₃ | H | (R)CH₃ | CH₃ | CH₃ | 8-Cl | 5.54 |
| Ex-96 | CH₃ | CH₃ | H | (S)CH₃ | CH₃ | CH₃ | 8-CF₃ | |
| Ex-97 | CH₃ | CH₃ | H | (R)CH₃ | CH₃ | CH₃ | 8-CF₃ | |
| Ex-98 | CH₃ | CHF₂ | H | (S)CH₃ | CH₃ | CH₃ | 8-CF₃ | |
| Ex-99 | CH₃ | CHF₂ | H | (R)CH₃ | CH₃ | CH₃ | 8-CF₃ | |
| Ex-100 | CH₃ | CHF₂ | -(CH₂)₂ | | H | (R)CH₃ | H | |
| Ex-101 | CH₃ | CHF₂ | -(CH₂)₂ | | H | (S)CH₃ | H | |

### Microtest

The active compounds were formulated separately as a stock solution having a concentration of 10000 ppm in dimethyl sulfoxide.

### Example 1 - Activity against the grey mold Botrytis cinerea in the microtiterplate test

The stock solutions were mixed according to the ratio, pipetted onto a micro titer plate (MTP) and diluted with water to the stated concentrations. A spore suspension of *Botrci cinerea* in an aqueous biomalt or yeast-bactopeptone-sodiumacetate solution was then added. The plates were placed in a water vapor-saturated chamber at a temperature of 18°C. Using an absorption photometer, the MTPs were measured at 405 nm 7 days after the inoculation.

In this test, the samples which had been treated with 31 ppm of the active substance from examples Ex-1, Ex-2, Ex-3, Ex-4, Ex-8, Ex-9, Ex-10 and Ex-11 respectively, showed 0 % growth of the pathogen.

### Example 2 - Activity against Fusarium culmorum in the microtiterplate test

The stock solutions were mixed according to the ratio, pipetted onto a micro titer plate (MTP) and diluted with water to the stated concentrations. A spore suspension of *Fusarium culmorum* in an aqueous biomalt or yeast-bactopeptone-glycerine or DOB solution was then added. The plates were placed in a water vapor-saturated chamber at a temperature of 18°C. Using an absorption photometer, the MTPs were measured at 405 nm 7 days after the inoculation.

In this test, the samples which had been treated with 31 ppm of the active substance from examples Ex-2 and Ex-3 respectively, showed 1 % growth of the pathogen.

### Example 3 - Activity against Venturia inaequalis in the microtiterplate test

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of *Botrci cinerea* in an aqueous biomalt or yeast-bactopeptone-sodiumacetate solution was then added.

In this test, the samples which had been treated with 31 ppm of the active substance from examples Ex-1, Ex-4, Ex-12 (at 50 ppm), Ex-18, Ex-21, Ex-22, Ex-24, Ex-28, Ex-29, Ex-31, Ex-36, Ex-44, Ex-45, Ex-47 respectively, showed unto 20 % growth of the pathogen.

### Example 4 - Activity against the grey mold Botrytis cinerea in the microtiterplate test

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of *Botrci cinerea* in an aqueous biomalt or yeast-bactopeptone-sodiumacetate solution was then added.

In this test, the samples which had been treated with 31 ppm of the active substance from examples Ex-1, Ex-3, Ex-4, Ex-6, Ex-7, Ex-8, Ex-9, Ex-11, Ex-12, Ex-13, Ex-15, Ex-16, Ex-17, Ex-19, Ex-20, Ex-21, Ex-22, Ex-25, Ex-26, Ex-27, Ex-28, Ex-29, Ex-30, Ex-31, Ex-32, Ex-33, Ex-34, Ex-36, Ex-38, Ex-39, Ex-43, Ex-45, Ex-46, Ex-47, Ex-48, Ex-49, Ex-50, Ex-51, Ex-52 + Ex-52 as racemat, Ex-68, Ex-69 respectively, showed unto 1 % growth of the pathogen.

### Example 5 - Activity against Fusarium culmorum in the microtiterplate test

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of *Fusarium culmorum* in an aqueous biomalt yeast-bactopeptone-glycerine or DOB solution was then added.

In this test, the samples which had been treated with 31 ppm of the active substance from examples Ex-3, Ex-11, Ex-26, Ex-27, Ex-30, Ex-34, Ex-46, Ex-48, Ex-49, Ex-52 and Ex-53 as racemat, respectively, showed unto 9 % growth of the pathogen.

### Example 6 - Activity against the leaf blotch on wheat caused by Septoria tritici in the microtiterplate test

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of *Septorion tritici* in an aqueous biomalt or yeast-bactopeptone-glycerine or DOB solution was then added.

In this test, the samples which had been treated with 31 ppm of the active substance from examples Ex-21, Ex-28, Ex-33, Ex-47, Ex-48 respectively, showed unto 18 % growth of the pathogen.

### Example 7 - Activity against Microdochium nivale in the microtiterplate test

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of the *Microdochium nivale* isolates in a DOB media (ph 7) was then added.

In this test, the samples which had been treated with 31 ppm of the active substance from examples Ex-1, Ex-2, Ex-3, Ex-4, Ex-6, Ex-7, Ex-9, Ex-11, Ex-12, Ex-13, Ex-15, Ex-16, Ex-19, Ex-20, Ex-21, Ex-22, Ex-23, Ex-24, Ex-25, Ex-26, Ex-27,Ex-28, Ex-29, Ex-30, Ex-31, Ex-32, Ex-33, Ex-34, Ex-36, Ex-37, Ex-38, Ex-39, Ex-41, Ex-44, Ex-45, Ex-46, Ex-47, Ex-52 and Ex-53 as racemat, Ex-68, Ex-69 respectively, showed unto 19 % growth of the pathogen.

### Example 8 - Activity against Colletotrichum orbiculare in the microtiterplate test

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of the *Colletotrichum orbiculare* isolates in a DOB media (ph 7) was then added.

In this test, the samples which had been treated with 31 ppm of the active substance from examples Ex-1, Ex-2, Ex-3, Ex-4, Ex-7, Ex-9, Ex-11, Ex-12, Ex-13, Ex-16, Ex-19, Ex-22, Ex-24, Ex-25, Ex-26, Ex-27,Ex-28, Ex-29, Ex-32, Ex-33, Ex-34, Ex-36, Ex-41, Ex-45, Ex-52 and Ex-53 as racemat, Ex-69 respectively, showed unto 15 % growth of the pathogen.

### Example 9 - Activity against Leptosphaeria nodorum in the microtiterplate test

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of the *Leptosphaeria nodorum* isolates in a DOB media (ph 7) was then added.

In this test, the samples which had been treated with 31 ppm of the active substance from examples Ex-1, Ex-2, Ex-3, Ex-4, Ex-6, Ex-7, Ex-9, Ex-11, Ex-12, Ex-13, Ex-15, Ex-16, Ex-17, Ex-18, Ex-19, Ex-20, Ex-21, Ex-22, Ex-23, Ex-24, Ex-25, Ex-26, Ex-27,Ex-28, Ex-29, Ex-30, Ex-31, Ex-32, Ex-33, Ex-34, Ex-36, Ex-37, Ex-38, Ex-39, Ex-40, Ex-42, Ex-43, Ex-44, Ex-45, Ex-46, Ex-47, Ex-52 and Ex-53 as racemat, Ex-68, Ex-69 respectively, showed unto 12 % growth of the pathogen.

### Example 10 - Activity against Fusarium gramminearis in the microtiterplate test

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of the *Fusarium gramminearis* isolates in a DOB media (ph 7) was then added.

In this test, the samples which had been treated with 31 ppm of the active substance from examples Ex-2, Ex-27, Ex-30, Ex-34 respectively, showed unto 16 % growth of the pathogen.

### Example 11 - Activity against Monilinia laxa in the microtiterplate test

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of the *Monilinia laxa* isolates in a DOB media (ph 7) was then added.

In this test, the samples which had been treated with 31 ppm of the active substance from examples Ex-1, Ex-2, Ex-3, Ex-4, Ex-6, Ex-7, Ex-9, Ex-10, Ex-11, Ex-12, Ex-13, Ex-15, Ex-16, Ex-17, Ex-18, Ex-20, Ex-21, Ex-22, Ex-23, Ex-24, Ex-25, Ex-26, Ex-27, Ex-28, Ex-29, Ex-30, Ex-31, Ex-32, Ex-33, Ex-34, Ex-36, Ex-40, Ex-44, Ex-45, Ex-47, Ex-52 and Ex-53 as racemat, Ex-68, Ex-69 respectively, showed unto 20 % growth of the pathogen.

### Example 12 - Activity against Ustilago maydis in the microtiterplate test

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of the *Ustilago maydis* isolates in a DOB media (ph 7) was then added.

In this test, the samples which had been treated with 31 ppm of the active substance from examples Ex-1, Ex-24, Ex-41 respectively, showed 0 % growth of the pathogen.

### Example 13 - Activity against Pyrenophora teres Qoi (FL129) resistant isolate in the microtiterplate test

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of the *Pyrenophora teres* Qoi (FL129) resistant isolates in a DOB media (ph 7) was then added.

In this test, the samples which had been treated with 31 ppm of the active substance from examples Ex-1, Ex-2, Ex-3, Ex-4, Ex-6, Ex-7, Ex-9, Ex-10, Ex-11, Ex-12, Ex-13, Ex-15, Ex-16, Ex-19, Ex-20, Ex-21, Ex-22, Ex-23, Ex-24, Ex-25, Ex-26, Ex-27, Ex-28, Ex-29, Ex-30, Ex-32, Ex-33, Ex-34, Ex-36, Ex-37, Ex-38, Ex-39, Ex-41, Ex-42, Ex-43, Ex-44, Ex-45, Ex-47, Ex-52 and Ex-53 as racemat, Ex-68, Ex-69 respectively, showed unto 19 % growth of the pathogen.

### Example 14 - Activity against Leptosphaeria maculans in the microtiterplate test

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of the *Leptosphaeria maculans* isolates in a DOB media (ph 7) was then added.

In this test, the samples which had been treated with 31 ppm of the active substance from examples Ex-1, Ex-2, Ex-3, Ex-4, Ex-6, Ex-11, Ex-12, Ex-15, Ex-16, Ex-17, Ex-18, Ex-19, Ex-20, Ex-21, Ex-22, Ex-23, Ex-25, Ex-26, Ex-27, Ex-28, Ex-29, Ex-30, Ex-31, Ex-32, Ex-33, Ex-34, Ex-36, Ex-37, Ex-38, Ex-39, Ex-41, Ex-42, Ex-43, Ex-44, Ex-45, Ex-46, Ex-68, Ex-69 respectively, showed unto 19 % growth of the pathogen.

### Example 15 - Activity against Phytophthora infestans in the microtiterplate test

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of the *Pyrenophora infenstans* in a DOB media (ph 7) was then added.

In this test, the samples which had been treated with 31 ppm of the active substance from examples Ex-1, Ex-2, Ex-8, Ex-9, Ex-10, Ex-11, Ex-12, Ex-13, Ex-15, Ex-20, Ex-23, Ex-24, Ex-31 respectively, showed unto 19 % growth of the pathogen.

### Example 16 - Activity against Mycosphorella fijiensis in the microtiterplate test

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of the *Mycosphorella fijiensis* isolates in a DOB media (ph 7) was then added.

In this test, the samples which had been treated with 31 ppm of the active substance from examples Ex-1, Ex-2, Ex-3, Ex-4, Ex-6, Ex-15, Ex-20, Ex-21, Ex-22, Ex-23, Ex-24, Ex-25, Ex-26, Ex-27, Ex-28, Ex-29, Ex-30, Ex-31, Ex-32, Ex-33, Ex-34, Ex-36, Ex-37, Ex-38, Ex-39, Ex-44, Ex-45 respectively, showed unto 20 % growth of the pathogen.

### Example 17 - Activity against Corynespora cassiicola in the microtiterplate test

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of the *Corynespora cassiicola* isolates in a DOB media (ph 7) was then added.

In this test, the samples which had been treated with 31 ppm of the active substance from examples Ex-24, Ex-25, Ex-26, Ex-27, Ex-28, Ex-29, Ex-30, Ex-31, Ex-32, Ex-33, Ex-34, Ex-36, Ex-44, Ex-45, Ex-47, Ex-69 respectively, showed unto 16 % growth of the pathogen.

### Example 18 - Activity against Corynespora cassiicola (CORYCA-G) G413A mutant in the microtiterplate test

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of the *Corynespora cassiicola* (CORYCA-G) G413A mutant isolates in a DOB media (ph 7) was then added.

In this test, the samples which had been treated with 31 ppm of the active substance from examples Ex-24, Ex-25, Ex-26, Ex-27, Ex-28, Ex-29, Ex-30, Ex-31, Ex-32, Ex-33, Ex-34, Ex-36, Ex-38, Ex-44, Ex-45, Ex-47, Ex-69 respectively, showed unto 18 % growth of the pathogen.

The measured parameters were compared to the growth of the active compound-free control variant (100%) and the fungus-free blank value to determine the relative growth in % of the pathogens in the respective active compounds.

### Green House

The compound was dissolved in a mixture of acetone and/or dimethylsulfoxide and the wetting agent/emulsifier Wettol, which is based on ethoxylated alkylphenoles, in a ratio (volume) solvent-emulsifier of 99 to 1 to give a total volume of 5 ml. Subsequently, water was added to total volume of 100 ml.

This stock solution was then diluted with the described solvent-emulsifier-water mixture to the final concentration given in the table below.

### Example 19 - Preventative fungicidal control of Botrytis cinerea on leaves of green pepper

Young seedlings of green pepper were grown in pots to the 4 to 5 leaf stage. These plants were sprayed to run-off with previously described spray solution, containing the concentration of active ingredient or mixture mentioned in the table below. The next day the plants were inoculated with an aqueous biomalt or DOB solution containing the spore suspension of *Botrytis cinerea.* Then the plants were immediately transferred to a humid chamber. After 5 days at 22 to 24°C and a saturated relative humidity, the extent of fungal attack on the leaves was visually assessed as % diseased leaf area.

In this test, the samples which had been treated with 250 ppm of the active substance from examples from examples Ex-1, Ex-2, Ex-3, Ex-4, Ex-8, Ex-9, Ex-12, Ex-13, Ex-16 and Ex-18 respectively, showed up to at most 15 % growth of the pathogen whereas the untreated plants were 90% infected.

### Example 20 - Preventative fungicidal control of white mold on oilseed rape caused by Sclerotinia sclerotiorum

Oilseed rapes were grown in pots to the 13 to 14 leaf stage. These plants were sprayed to run-off with previously described spray solution, containing the concentration of active ingredient or their mixture mentioned in the table below. The plants could air-dry. The next day the applicated rape petals were fixed wit 25µl of 2.5% methylcellulose on leaf 1 and 2. 25 µl of a spore suspension of *Sclerotinia sclerotiorum* was pipetted on each fixed rape petal. After 14 days at 20°C and a relative humidity of 60 % the extent of fungal attack on the leaves was visually assessed as % diseased leaf area.

In this test, the samples which had been treated with 100 g/ha of the active substance from examples from Ex-1, Ex-2, Ex-3, Ex-4, Ex-7, Ex-8, Ex-11, Ex-12, Ex-15, Ex-17, Ex-20, Ex-21, Ex-22, Ex-25, Ex-26, Ex-27, Ex-28, Ex-30, Ex-31, Ex-32, Ex-33, Ex-34, Ex-40, Ex-45, Ex-47 respectively, showed up to at most 15 % growth of the pathogen whereas the untreated plants were 100% infected.

### Example 21 - Preventative fungicidal control of Botrytis cinerea on leaves of green pepper

Young seedlings of green pepper were grown in pots to the 4 to 5 leaf stage. These plants were sprayed to run-off with previously described spray solution, containing the concentration of active ingredient or mixture mentioned in the table below. The next day the plants were inoculated with an aqueous biomalt or DOB solution containing the spore suspension of *Botrytis cinerea.* Then the plants were immediately transferred to a humid chamber. After 5 days at 22 to 24°C and a saturated relative humidity, the extent of fungal attack on the leaves was visually assessed as % diseased leaf area.

In this test, the samples which had been treated with 100 g/ha of the active substance from examples from Ex-8, Ex-12, Ex-25, Ex-26, Ex-27 respectively, showed up to at most 18 % growth of the pathogen whereas the untreated plants were 100% infected.

### Example 22 - Long lasting control of Botrytis cinerea on leaves of green pepper

Young seedlings of green pepper were grown in pots to the 4 to 5 leaf stage. These plants were sprayed to run-off with previously described spray solution, containing the concentration of active ingredient or mixture mentioned in the table below. The plants were then cultivated in the greenhouse for 7 days and then inoculated with an aqueous biomalt or DOB solution containing the spore suspension of *Botrytis cinerea.* Then the plants were immediately transferred to a humid chamber. After 5 days at 22 to 24°C and a saturated relative humidity, the extent of fungal attack on the leaves was visually assessed as % diseased leaf area.

In this test, the samples which had been treated with 100 g/ha of the active substance from examples from Ex-21, Ex-25, Ex-26, Ex-27, Ex-28, Ex-29, Ex-45, Ex-47, Ex-69 respectively, showed up to at most 14 % growth of the pathogen whereas the untreated plants were 90% infected.

### Comparative examples

### Example 1 - Activity against leaf blotch on wheat caused by Septoria tritici

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of *Septoria tritici* in an aqueous biomalt or yeast-bactopeptone-glycerine or DOB solution was then added. The plates were placed in a water vapor-saturated chamber at a temperature of 18°C. Using an absorption photometer, the MTPs were measured at 405 nm 7 days after the inoculation.

| **Compound** | **Structure** | **Growth (%) at 125pm SEPTTR** |
|---|---|---|
| D1 = WO 2010/125782 | 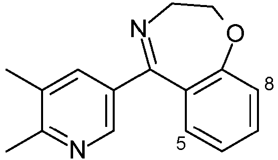 | 98 |
| Ex-2 acc. to the pending application | 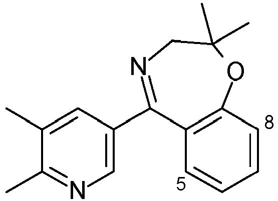 | 51 |
| Ex-11 acc. to the pending application | 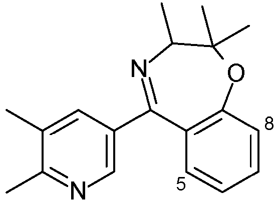 | 52 |
| Ex-3 acc. to the pending application | 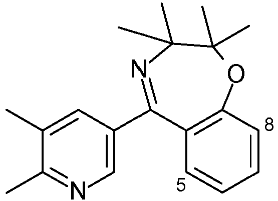 | 64 |

### Example 2 - Activity against wheat leaf spots caused by Leptosphaeria nodorum

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations. A spore suspension of *Leptosphaeria nodorum* in an aqueous biomalt or yeast-bactopeptone-glycerine or DOB solution was then added. The plates were placed in a water vapor-saturated chamber at a temperature of 18°C. Using an absorption photometer, the MTPs were measured at 405 nm 7 days after the inoculation.

| **Compound** | **Structure** | **Growth (%) at 8pm LEPTNO** |
|---|---|---|
| D1 = WO 2010/125782 | 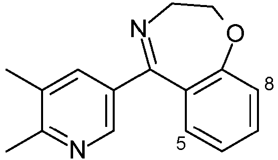 | 45 |
| Ex-2 acc. to the pending application | 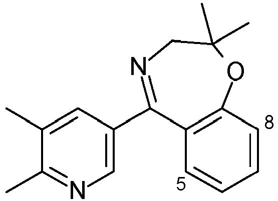 | 4 |
| Ex-11 acc. to the pending application | 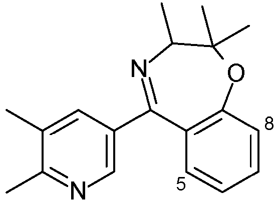 | 6 |
| Ex-3 acc. to the pending application | 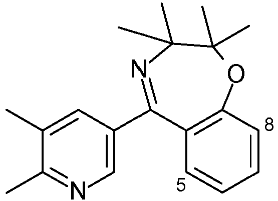 | 30 |

## Claims

1. Compounds of formula I 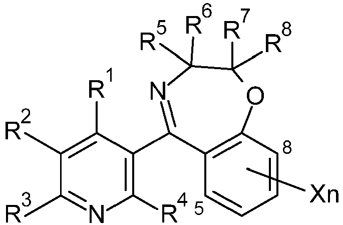 wherein
R¹ is H;
R² is in each case independently selected from halogen, CN, C₁-C₆-alkyl, C₁-C₆-halogenalkyl, C₂-C₆-alkenyl, C₂-C₆-halogenalkenyl, C₂-C₆-alkynyl, O-C₁-C₆-alkyl, C₃-C₆-cycloalkyl;
R³ is in each case independently selected from C₁-C₆-alkyl, C₁-C₆-halogenalkyl, C₂-C₆-alkenyl, C₂-C₆-halogenalkenyl, C₂-C₆-alkynyl, Q-C₁-C₆-alkyl, C₃-C₆-cycloalkyl;
R⁴ is H;
R⁵ are in each case independently selected from H, F, CN, C₁-C₆-alkyl, C₁-C₆-halogenalkyl, C₂-C₆-alkenyl, phenyl, benzyl,
wherein the moieties are unsubstituted or substituted by one to three groups R^{5a}, which independently of one another are selected from:
halogen, CN, C₁-C₆-alkyl, C₁-C₆-halogenalkyl, O-C₁-C₆-alkyl;
R⁶ are in each case independently selected from H, F, CN, C₁-C₆-alkyl, C₁-C₆-halogenalkyl, C₂-C₆-alkenyl, phenyl, benzyl,
wherein the moieties of R⁶ are unsubstituted or substituted by one to three groups R^{6a}, which independently of one another are selected from:
halogen, CN, C₁-C₆-alkyl, C₁-C₆-halogenalkyl, O-C₁-C₆-alkyl;
or
R⁵ and R⁶ form together with the C atoms to which they are bound =O;
or
R⁵ and R⁶ form together with the C atoms to which they are bound a C₃-C₆-cycloalkyl or a a 3- to 6-membered saturated heterocycle which contains 1, 2 or 3 heteroatoms from the group consisting of O and S; wherein the cycloalkyl or heterocycle can be unsubsituted or substitued by halogene, C₁-C₆-alkyl, C₁-C₆-halogenalkyl;
R⁷ are in each case independently selected from H, F, CN, C₁-C₆-alkyl, C₁-C₆-halogenalkyl, C₂-C₆-alkenyl, phenyl, benzyl,
wherein the moieties are unsubstituted or substituted by one to three groups R^{7a}, which independently of one another are selected from:
halogen, CN, C₁-C₆-alkyl, C₁-C₆-halogenalkyl, O-C₁-C₆-alkyl;
R⁸ are in each case independently selected from H, F, CN, C₁-C₆-alkyl, C₁-C₆-halogenalkyl, C₂-C₆-alkenyl, phenyl, benzyl,
wherein the moieties are unsubstituted or substituted by one to three groups R^{8a}, which independently of one another are selected from:
halogen, CN, C₁-C₆-alkyl, C₁-C₆-halogenalkyl, O-C₁-C₆-alkyl;
or
R⁷ and R⁸ form together with the C atoms to which they are bound a C₃-C₆-cycloalkyl or a a 3- to 6-membered saturated heterocycle which contains 1, 2 or 3 heteroatoms from the group consisting of O and S;
X is in each case independently selected from halogen, CN, C₁-C₆-alkyl, C₁-C₆-halogenalkyl, O-C₁-C₆-alkyl, O-C₁-C₆-halogenalkyl, C₃-C₆-cycloalkyl, C₂-C₆-alkenyl, C₂-C₆-alkynyl;
n is 0, 1, 2 or 3,
with the proviso that
R⁵, R⁶, R⁷, R⁸ can not be all H;
and the N-oxides and the agriculturally acceptable salts thereof as fungicides.

2. Compound of claim 1, wherein R² is C₁-C₆-alkyl or O-C₁-C₆-alkyl.

3. Compound of any one of claims 1 to 2, wherein R² is CH₃.

4. Compound of any one of claims 1 to 3, wherein R³ is selected from C₁-C₆-alkyl or C₁-C₆-halogenalkyl.

5. Compound of any one of claims 1 to 4, wherein R³ is CH₃ or CHF₂.

6. Compound of any one of claims 1 to 5, wherein R⁵ is H or C₁-C₆-alkyl.

7. Compound of any one of claims 1 to 6, wherein R⁶ is selected from the H or C₁-C₆-alkyl.

8. Compound of any one of claims 1 to 7, wherein R⁵ and R⁶ form together with the C atoms to which they are bound =O or a C₃-C₆-cycloalkyl.

9. Compound of any one of claims 1 to 8, wherein R⁷ is H or C₁-C₆-alkyl.

10. Compound of any one of claims 1 to 9, wherein R³ is selected from the C₁-C₆-alkyl, phenyl, benzyl, wherein the moieties are unsubstituted or substituted by one to three groups R^{5a}, which independently of one another are selected from:
O-C₁-C₆-alkyl.

11. Compound of any one of claims 1 to 10, wherein X is selected from halogen, C₁-C₆-alkyl, O-C₁-C₆-alkyl, O-C₁-C₆-halogenalkyl.

12. Compound of any one of claims 1 to 11, wherein X is selected from F, CH₃, C₂H₅, OCH₃, OCHF₂, OCF₃.

13. A composition, comprising one compound of formula I, as defined in any of the claims 1 to 12, an N-oxide or an agriculturally acceptable salt thereof.

14. A process for preparing compounds of formula I, comprising a reaction of the compound of the formula Y: 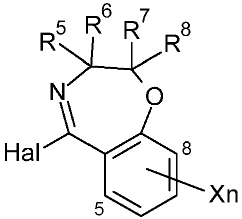 wherein R⁵, R⁶, R⁷, R⁸ and Xn are as defined in claims 1 to 12 and Hal is halogen.

15. A compound of the formula Y 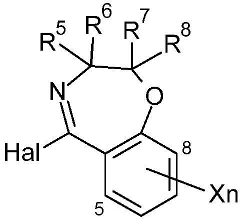 wherein
R⁵ is selected from the group consisting of H, C₁-C₆-alkyl,
R⁶ is selected from the group consisting of H, C₁-C₆-alkyl, or
R⁵ and R⁶ form together with the C atoms to which they are bound a C₃-C₆-cycloalkyl;
R⁷ is selected from the group consisting of H, C₁-C₆-alkyl,
R⁸ is selected from the group consisting of H, C₁-C₆-alkyl, or
R⁷ and R⁸ form together with the C atoms to which they are bound a C₃-C₆-cycloalkyl;
X is halogene, C₁-C₆-alkyl,
N is 0, 1 or 2.

## Patentansprüche

1. Verbindungen der Formel I 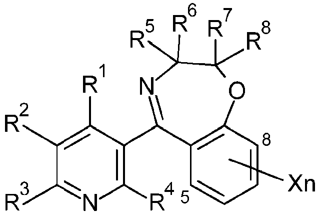 wobei
R¹ für H steht;
R² jeweils unabhängig aus Halogen, CN, C₁-C₆-Alkyl, C₁-C₆-Halogenalkyl, C₂-C₆-Alkenyl, C₂-C₆-Halogenalkenyl, C₂-C₆-Alkinyl, O-C₁-C₆-Alkyl, C₃-C₆-Cycloalkyl ausgewählt ist;
R³ jeweils unabhängig aus C₁-C₆-Alkyl, C₁-C₆-Halogenalkyl, C₂-C₆-Alkenyl, C₂-C₆-Halogenalkenyl, C₂-C₆-Alkinyl, O-C₁-C₆-Alkyl, C₃-C₆-Cycloalkyl ausgewählt ist;
R⁴ für H steht;
R⁵ jeweils unabhängig aus H, F, CN, C₁-C₆-Alkyl, C₁-C₆-Halogenalkyl, C₂-C₆-Alkenyl, Phenyl, Benzyl ausgewählt ist,
wobei die Gruppierungen unsubstituiert sind oder durch eine bis drei Gruppen R^{5a} substituiert sind, die unabhängig voneinander ausgewählt sind aus:
Halogen, CN, C₁-C₆-Alkyl, C₁-C₆-Halogenalkyl, O-C₁-C₆-Alkyl;
R⁶ jeweils unabhängig aus H, F, CN, C₁-C₆-Alkyl, C₁-C₆-Halogenalkyl, C₂-C₆-Alkenyl, Phenyl, Benzyl ausgewählt ist,
wobei die Gruppierungen von R⁶ unsubstituiert sind oder durch eine bis drei Gruppen R^{6a} substituiert sind, die unabhängig voneinander ausgewählt sind aus:
Halogen, CN, C₁-C₆-Alkyl, C₁-C₆-Halogenalkyl, O-C₁-C₆-Alkyl;
oder
R⁵ und R⁶ zusammen mit den C-Atomen, an die sie gebunden sind, =O bilden;
oder
R⁵ und R⁶ zusammen mit den C-Atomen, an die sie gebunden sind, ein C₃-C₆-Cycloalkyl oder einen 3- bis 6-gliedrigen gesättigten Heterocyclus, der 1, 2 oder 3 Heteroatome aus der Gruppe bestehend aus O und S enthält, bilden; wobei das Cycloalkyl oder der Heterocyclus unsubstituiert oder durch Halogen, C₁-C₆-Alkyl, C₁-C₆-Halogenalkyl substituiert sein kann;
R⁷ jeweils unabhängig aus H, F, CN, C₁-C₆-Alkyl, C₁-C₆-Halogenalkyl, C₂-C₆-Alkenyl, Phenyl, Benzyl ausgewählt ist,
wobei die Gruppierungen unsubstituiert sind oder durch eine bis drei Gruppen R^{7a} substituiert sind, die unabhängig voneinander ausgewählt sind aus:
Halogen, CN, C₁-C₆-Alkyl, C₁-C₆-Halogenalkyl, O-C₁-C₆-Alkyl;
R⁸ jeweils unabhängig aus H, F, CN, C₁-C₆-Alkyl, C₁-C₆-Halogenalkyl, C₂-C₆-Alkenyl, Phenyl, Benzyl ausgewählt ist,
wobei die Gruppierungen unsubstituiert sind oder durch eine bis drei Gruppen R^{8a} substituiert sind, die unabhängig voneinander ausgewählt sind aus:
Halogen, CN, C₁-C₆-Alkyl, C₁-C₆-Halogenalkyl, O-C₁-C₆-Alkyl;
oder
R⁷ und R⁸ zusammen mit den C-Atomen, an die sie gebunden sind, ein C₃-C₆-Cycloalkyl oder einen 3- bis 6-gliedrigen gesättigten Heterocyclus, der 1, 2 oder 3 Heteroatome aus der Gruppe bestehend aus O und S enthält, bilden;
X jeweils unabhängig aus Halogen, CN, C₁-C₆-Alkyl, C₁-C₆-Halogenalkyl, O-C₁-C₆-Alkyl, O-C₁-C₆-Halogenalkyl, C₃-C₆-Cycloalkyl, C₂-C₆-Alkenyl, C₂-C₆-Alkinyl ausgewählt ist; n für 0, 1, 2 oder 3 steht,
mit der Maßgabe, dass
R⁵, R⁶, R⁷, R⁸ nicht alle H sein können;
und die N-Oxide und die landwirtschaftlich unbedenklichen Salze davon als Fungizide.

2. Verbindung nach Anspruch 1, wobei R² für C₁-C₆-Alkyl oder O-C₁-C₆-Alkyl steht.

3. Verbindung nach einem der Ansprüche 1 bis 2, wobei R² für CH₃ steht.

4. Verbindung nach einem der Ansprüche 1 bis 3, wobei R³ aus C₁-C₆-Alkyl oder C₁-C₆-Halogenalkyl ausgewählt ist.

5. Verbindung nach einem der Ansprüche 1 bis 4, wobei R³ für CH₃ oder CHF₂ steht.

6. Verbindung nach einem der Ansprüche 1 bis 5, wobei R⁵ für H oder C₁-C₆-Alkyl steht.

7. Verbindung nach einem der Ansprüche 1 bis 6, wobei R⁶ aus H oder C₁-C₆-Alkyl ausgewählt ist.

8. Verbindung nach einem der Ansprüche 1 bis 7, wobei R⁵ und R⁶ zusammen mit den C-Atomen, an die sie gebunden sind, =O oder ein C₃-C₆-Cycloalkyl bilden.

9. Verbindung nach einem der Ansprüche 1 bis 8, wobei R⁷ für H oder C₁-C₆-Alkyl steht.

10. Verbindung nach einem der Ansprüche 1 bis 9, wobei R⁸ aus C₁-C₆-Alkyl, Phenyl, Benzyl ausgewählt ist, wobei die Gruppierungen unsubstituiert sind oder durch eine bis drei Gruppen R^{5a} substituiert sind, die unabhängig voneinander ausgewählt sind aus:
O-C₁-C₆-Alkyl.

11. Verbindung nach einem der Ansprüche 1 bis 10, wobei X aus Halogen, C₁-C₆-Alkyl, O-C₁-C₆-Alkyl, O-C₁-C₆-Halogenalkyl ausgewählt ist.

12. Verbindung nach einem der Ansprüche 1 bis 11, wobei X aus F, CH₃, C₂H₅, OCH₃, OCHF₂, OCF₃ ausgewählt ist.

13. Zusammensetzung, umfassend eine Verbindung der Formel I, wie in einem der Ansprüche 1 bis 12 definiert, ein N-Oxid oder ein landwirtschaftlich unbedenkliches Salz davon.

14. Verfahren zur Herstellung von Verbindungen der Formel I umfassend eine Reaktion der Verbindung der Formel Y: 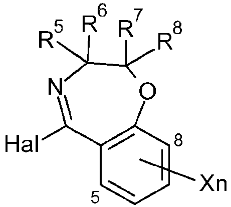 wobei R⁵, R⁶, R⁷, R⁸ und Xn wie in den Ansprüchen 1 bis 12 definiert sind und Hal für Halogen steht.

15. Verbindung der Formel Y 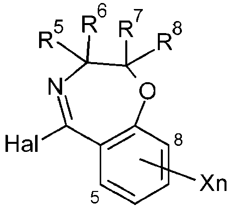 wobei
R⁵ aus der Gruppe bestehend aus H, C₁-C₆-Alkyl ausgewählt ist,
R⁶ aus der Gruppe bestehend aus H, C₁-C₆-Alkyl ausgewählt ist oder
R⁵ und R⁶ zusammen mit den C-Atomen, an die sie gebunden sind, ein C₃-C₆-Cycloalkyl bilden;
R⁷ aus der Gruppe bestehend aus H, C₁-C₆-Alkyl ausgewählt ist,
R⁸ aus der Gruppe bestehend aus H, C₁-C₆-Alkyl ausgewählt ist, oder
R⁷ und R⁸ zusammen mit den C-Atomen, an die sie gebunden sind, ein C₃-C₆-Cycloalkyl bilden;
X für Halogen, C₁-C₆-Alkyl steht,
N für 0, 1 oder 2 steht.

## Revendications

1. Composés de formule I 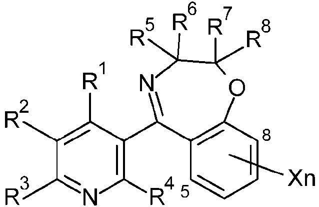 dans laquelle
R¹ est H ;
R² est, dans chaque cas, choisi indépendamment parmi halogène, CN, C₁-C₆-alkyle, C₁-C₆-halogénoalkyle, C₂-C₆-alcényle, C₂-C₆-halogénoalcényle, C₂-C₆-alcynyle, O-C₁-C₆-alkyle, C₃-C₆-cycloalkyle ;
R³ est, dans chaque cas, choisi indépendamment parmi C₁-C₆-alkyle, C₁-C₆-halogénoalkyle, C₂-C₆-alcényle, C₂-C₆-halogénoalcényle, C₂-C₆-alcynyle, O-C₁-C₆-alkyle, C₃-C₆-cycloalkyle ;
R⁴ est H ;
R⁵ sont, dans chaque cas, indépendamment choisis parmi H, F, CN, C₁-C₆-alkyle, C₁-C₆-halogénoalkyle, C₂-C₆-alcényle, phényle, benzyle,
dans laquelle les groupements sont non substitués ou substitués par un à trois groupes R^{5a}, lesquels indépendamment les uns des autres, sont choisis parmi :
halogène, CN, C₁-C₆-alkyle, C₁-C₆-halogénoalkyle, O-C₁-C₆-alkyle ;
R⁶ sont, dans chaque cas, indépendamment choisis parmi H, F, CN, C₁-C₆-alkyle, C₁-C₆-halogénoalkyle, C₂-C₆-alcényle, phényle, benzyle,
dans laquelle les groupements R⁶ sont non substitués ou substitués par un à trois groupes R^{6a}, lesquels indépendamment les uns des autres, sont choisis parmi :
halogène, CN, C₁-C₆-alkyle, C₁-C₆-halogénoalkyle, O-C₁-C₆-alkyle ;
ou
R⁵ et R⁶ forment, conjointement avec les atomes de C auxquels ils sont liés, =O ;
ou
R⁵ et R⁶ forment, conjointement avec les atomes de C auxquels ils sont liés, un C₃-C₆-cycloalkyle ou un hétérocycle saturé à 3 à 6 chaînons qui contient 1, 2 ou 3 hétéroatomes du groupe constitué par O et S ; le cycloalkyle ou l'hétérocycle pouvant être non substitué ou substitué par halogène, C₁-C₆-alkyle, C₁-C₆-halogénoalkyle ;
R⁷ sont, dans chaque cas, indépendamment choisis parmi H, F, CN, C₁-C₆-alkyle, C₁-C₆-halogénoalkyle, C₂-C₆-alcényle, phényle, benzyle,
dans laquelle les groupements sont non substitués ou substitués par un à trois groupes R^{7a}, lesquels indépendamment les uns des autres, sont choisis parmi :
halogène, CN, C₁-C₆-alkyle, C₁-C₆-halogénoalkyle, O-C₁-C₆-alkyle ;
R⁸ sont, dans chaque cas, indépendamment choisis parmi H, F, CN, C₁-C₆-alkyle, C₁-C₆-halogénoalkyle, C₂-C₆-alcényle, phényle, benzyle,
dans laquelle les groupements sont non substitués ou substitués par un à trois groupes R^{8a}, lesquels indépendamment les uns des autres, sont choisis parmi :
halogène, CN, C₁-C₆-alkyle, C₁-C₆-halogénoalkyle, O-C₁-C₆-alkyle ;
ou
R⁷ et R⁸ forment, conjointement avec les atomes de C auxquels ils sont liés, un C₃-C₆-cycloalkyle ou un hétérocycle saturé à 3 à 6 chaînons qui contient 1, 2 ou 3 hétéroatomes du groupe constitué par O et S ;
X est dans chaque cas choisi indépendamment parmi halogène, CN, C₁-C₆-alkyle, C₁-C₆-halogénoalkyle, O-C₁-C₆-alkyle, O-C₁-C₆-halogénoalkyle, C₃-C₆-cycloalkyle, C₂-C₆-alkényle, C₂-C₆-alcynyle ;
n est 0, 1, 2 ou 3,
à condition que
R⁵, R⁶, R⁷, R⁸ ne peuvent pas être tous H ;
et sels acceptables sur le plan agricole et N-oxydes correspondants comme fongicides.

2. Composé selon la revendication 1, dans lequel R² est C₁-C₆-alkyle ou O-C₁-C₆-alkyle.

3. Composé selon l'une quelconque des revendications 1 à 2, dans lequel R² est CH₃.

4. Composé selon l'une quelconque des revendications 1 à 3, dans lequel R³ est choisi parmi C₁-C₆-alkyle ou C₁-C₆-halogénoalkyle.

5. Composé selon l'une quelconque des revendications 1 à 4, dans lequel R³ est CH₃ ou CHF₂.

6. Composé selon l'une quelconque des revendications 1 à 5, dans lequel R⁵ est H ou C₁-C₆-alkyle.

7. Composé selon l'une quelconque des revendications 1 à 6, dans lequel R⁶ est choisi parmi H ou C₁-C₆-alkyle.

8. Composé selon l'une quelconque des revendications 1 à 7, dans lequel R⁵ et R⁶ forment conjointement avec les atomes de C auxquels ils sont liés =O ou un C₃-C₆-cycloalkyle.

9. Composé selon l'une quelconque des revendications 1 à 8, dans lequel R⁷ est H ou C₁-C₆-alkyle.

10. Composé selon l'une quelconque des revendications 1 à 9, dans lequel R⁸ est choisi parmi C₁-C₆-alkyle, phényle, benzyle, les groupements étant non substitués ou substitués par un à trois groupes R^{5a}, lesquels indépendamment les uns des autres, sont choisis parmi :
O-C₁-C₆-alkyle .

11. Composé selon l'une quelconque des revendications 1 à 10, dans lequel X est choisi parmi halogène, C₁-C₆-alkyle , O-C₁-C₆-alkyle, O-C₁-C₆-halogénoalkyle.

12. Composé selon l'une quelconque des revendications 1 à 11, dans lequel X est choisi parmi F, CH₃, C₂H₅, OCH₃, OCHF₂, OCF₃.

13. Composition, comprenant un composé de formule I, tel que défini selon l'une quelconque des revendications 1 à 12, un sel acceptable sur le plan agricole ou un N-oxyde de celui-ci.

14. Procédé de préparation d'un composé de formule I, comprenant une réaction du composé de formule Y : 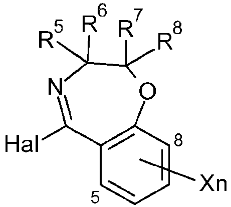 dans laquelle R⁵, R⁶, R⁷, R⁸ et Xn sont tels que définis dans les revendications 1 à 12 et Hal est halogène.

15. Composé de formule Y 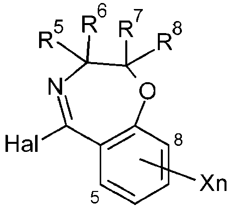 dans laquelle
R⁵ est choisi dans le groupe constitué par H, C₁-C₆-alkyle,
R⁶ est choisi dans le groupe constitué par H, C₁-C₆-alkyle ou
R⁵ et R⁶ forment conjointement avec les atomes de C auxquels ils sont liés un C₃-C₆-cycloalkyle ;
R⁷ est choisi dans le groupe constitué par H, C₁-C₆-alkyle,
R⁸ est choisi dans le groupe constitué par H, C₁-C₆-alkyle ou
R⁷ et R⁸ forment conjointement avec les atomes de C auxquels ils sont liés un C₃-C₆-cycloalkyle ;
X est halogène, C₁-C₆-alkyle,
N est 0, 1 ou 2.
